(19) Europäisches Patentamt
European Patent Office
Office européen des brevets

(11) EP 4 428 128 A1

(12) EUROPEAN PATENT APPLICATION
published in accordance with Art. 153(4) EPC

(43) Date of publication:
**11.09.2024 Bulletin 2024/37**

(21) Application number: **22889413.5**

(22) Date of filing: **04.11.2022**

(51) International Patent Classification (IPC):
***C07D 265/30*** *(2006.01)* ***A61K 31/5377*** *(2006.01)*
***A61P 25/00*** *(2006.01)*

(52) Cooperative Patent Classification (CPC):
**A61K 31/5377; A61P 25/00; C07D 265/30**

(86) International application number:
**PCT/CN2022/129881**

(87) International publication number:
**WO 2023/078392 (11.05.2023 Gazette 2023/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2021 CN 202111301874**
**20.01.2022 CN 202210066119**
**20.01.2022 CN 202210065986**
**15.04.2022 CN 202210398665**
**09.09.2022 CN 202211104724**
**09.09.2022 CN 202211105290**

(71) Applicants:
- **Shanghai Hansoh Biomedical Co., Ltd.**
**Shanghai 201203 (CN)**
- **Jiangsu Hansoh Pharmaceutical Group Co., Ltd.**
**Lianyungang, Jiangsu 222047 (CN)**

(72) Inventors:
- **SU, Yidong**
**Shanghai 201203 (CN)**
- **LI, Kailong**
**Shanghai 201203 (CN)**
- **HUANG, Zhiqiang**
**Shanghai 201203 (CN)**
- **DENG, Haining**
**Shanghai 201203 (CN)**
- **ZHOU, Xiaohan**
**Shanghai 201203 (CN)**
- **YU, Wensheng**
**Shanghai 201203 (CN)**

(74) Representative: **Cabinet Beau de Loménie**
**158, rue de l'Université**
**75340 Paris Cedex 07 (FR)**

# (54) 2-(ARYL-2-YL) MORPHOLINE AND DEUTERATED DERIVATIVE THEREOF, PREPARATION METHOD THEREFOR AND APPLICATION THEREOF

(57) Provided are a 2-(aryl-2-yl) morpholine and a deuterated derivative thereof, a preparation method therefor and an application thereof. In particular, involved are the use of a compound shown by general formula (V) in the treatment of central nervous system diseases, a deuterated compound thereof, a preparation method of deuterated compound, a pharmaceutical composition containing the deuterated compound, and the use thereof as a TAAR1 agonist in the treatment of central nervous system diseases.

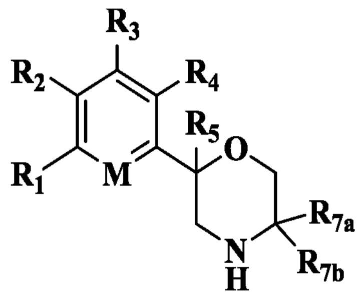

(V)

EP 4 428 128 A1

## Description

### FIELD OF THE INVENTION

The present disclosure belongs to the field of biomedicine, and specifically relates to use of 2-(aryl-2-yl) morpholine in the treatment of schizophrenia and deuterated derivatives thereof, preparation method therefor and application thereof.

### BACKGROUND OF THE INVENTION

Schizophrenia is the most prevalent mental illness, with a slow course and a tendency to recur, aggravation or worsen, causing serious disease burden and adverse consequences for patients and their families. Psychopath have positive symptoms such as delusions, hallucinations, disorders of thought, language and behavior, and negative symptoms such as no emotions and expressions, poor speech, lack of pleasure, and other symptoms such as cognitive disorder. Although there have been significant development in the reasearch and clinical application of antipsychotic drugs in the past few decades, traditional antipsychotics (first-generation) (such as haloperidol, droperidol, methylpyridazine, etc) and atypical antipsychotics (second-generation) (such as clozapine, risperidone, olanzapine, aripiprazole, etc) all target D2 and 5-$HT_{2A}$ and have good treatment effect on positive symptoms, but have no ideal improvement effect on negative symptoms and cognitive disorder, and have side effects such as extrapyramidal tract and weight gain. There are also many studies on non-D2/5-$HT_{2A}$ receptors for antipsychotic drugs, including GlyT1 D1, D4, D3, NMDA, mGluR2/3, AMPA 5-$HT_{2C}$, nicotinic a7, M1/M4 of muscarine, H3, NK-3 etc. Although these targets have shown promising efficacy in preclinical models, most novel non-D2/5-$HT_{2A}$ drugs have shown limited or no success in clinical trials. Therefore, there is an urgent need to develop new target drugs against schizophrenia that can improve both positive symptoms and negative symptoms and cognitive disorder without extrapyramidal side effects.

Trace amines (TAs) have low concentrations in the mammalian brain, and their levels change in many psychiatric disorder (schizophrenia, depression, anxiety, Parkinson's disease/attention deficit hyperactivity disorder). TAAR1 receptor is a subtype of TA1 receptor, belonging to the G-protein-coupled receptor, which is mainly expressed in the peripheral organs and cells (such as stomach, small intestine, duodenum, and hemameba), central nervous system (CNS) astrocytes and presynaptic membrane of monoamine neurons, and has the function of regulating DA, 5-HT and glutaminergic activity.

5-$HT_{1A}$ receptor is a member of the G protein-coupled receptor family and a subtype of the 5-HT receptor, which is distributed both in the central and peripheral regions and is closely related to mental, emotion, learning and memory, etc. The density of 5-$HT_{1A}$ receptor in the frontal and temporal cortex of schizophrenic is increased, and the activation of 5-$HT_{1A}$ receptor can improve the negative symptoms of schizophrenia. And the research found that it is also related to improving positive symptoms.

The international publication application WO-2011069063A2 discloses SEP-363856 of TAAR1 receptor agonist and 5-$HT_{1A}$ receptor partial agonist, which is used for the treatment of schizophrenia. The result of clinical phase II trial is good. It has good efficacy for both positive and negative symptoms and safety. It has currently entered the clinical phase III study. That is an urgent need to develop novel targets drugs with good safety and efficacy for both positive and negative symptoms in the treatment of schizophrenia in order to meet the huge market demand.

### SUMMARY OF THE INVENTION

On the one side, the object of the present invention is to provide the use of a compound as shown below, a stereoisomer thereof or a pharmaceutically acceptable salt thereof in the preparation of medicaments for the treatment of diseases related to TAAR1.

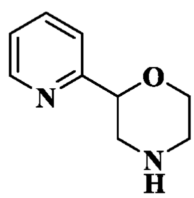 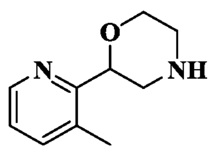 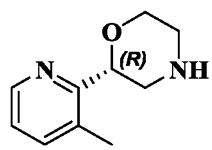 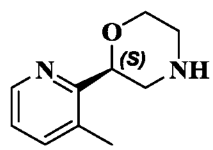

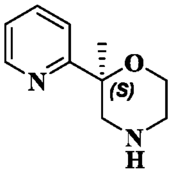 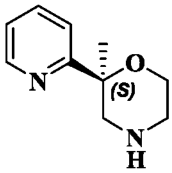 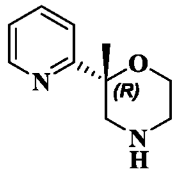 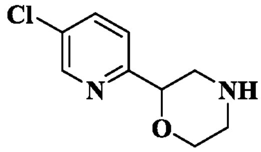

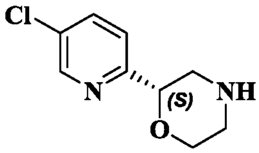 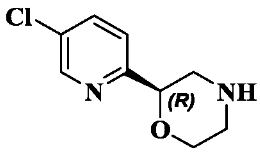 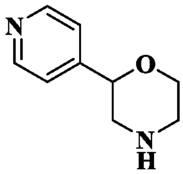 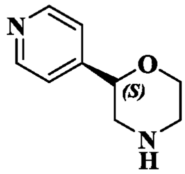

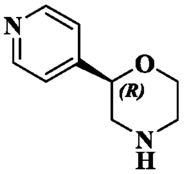 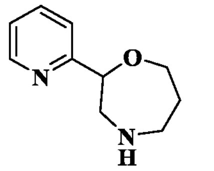 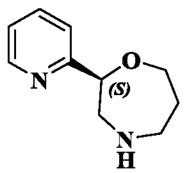 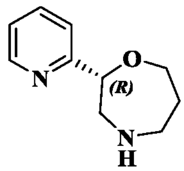

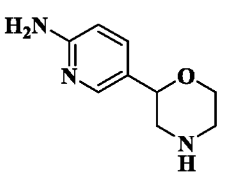 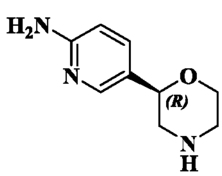 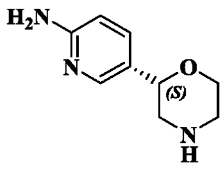 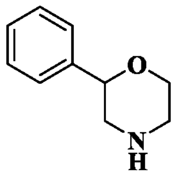

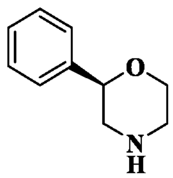 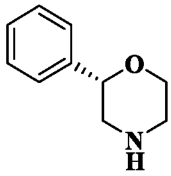 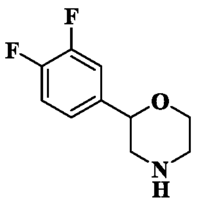 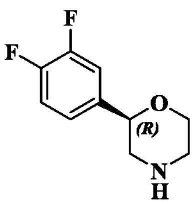 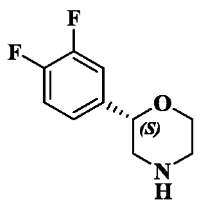

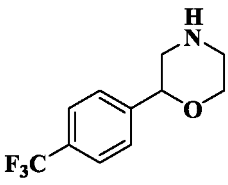 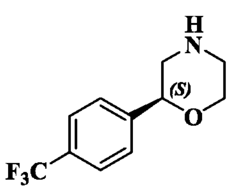 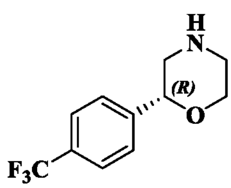 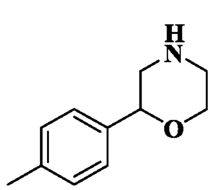

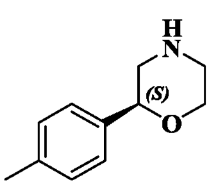 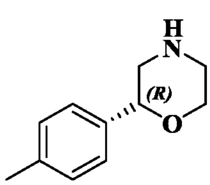 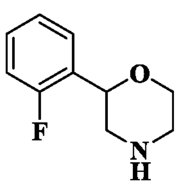 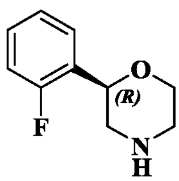 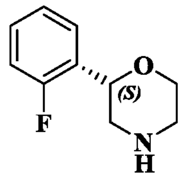

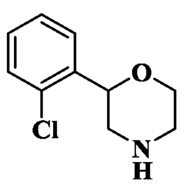 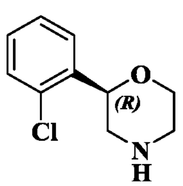 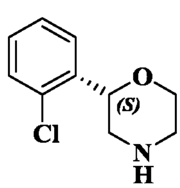 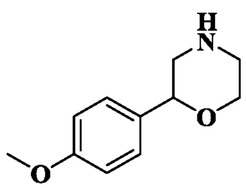

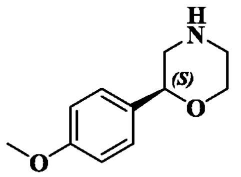 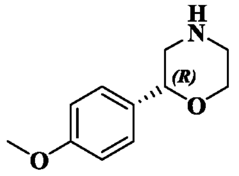 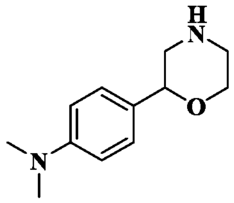 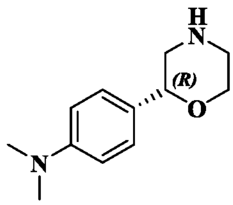

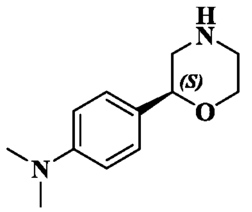 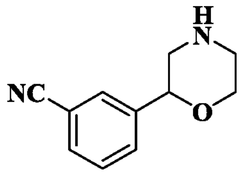 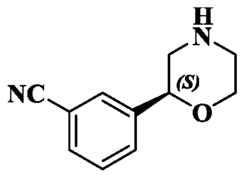 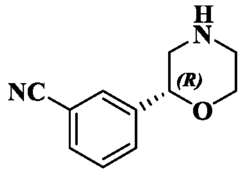

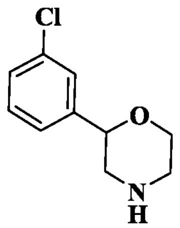 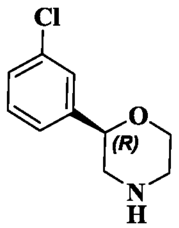 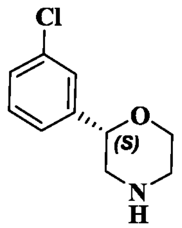 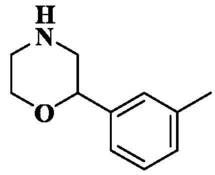 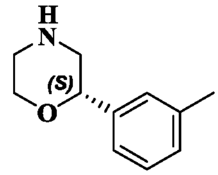

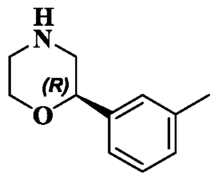 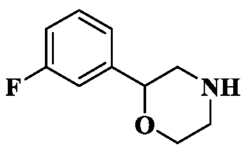 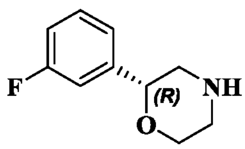 or 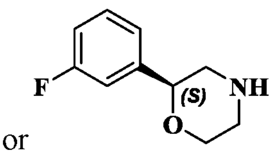 .

On the other side, the object of the present invention is to provide a compound represented by general formula (I), a stereoisomer or a pharmaceutically acceptable salt thereof, wherein the compound of general formula (I) has a structure as follows,

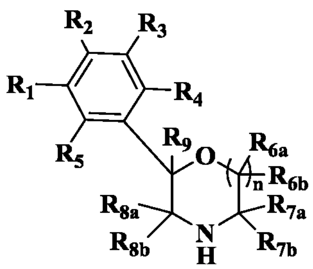

(I)

$R_1$, $R_2$, $R_3$, $R_4$, $R_5$, and $R_9$ are each independently selected from the group consisting of hydrogen, deuterium, halogen, amino, hydroxyl, cyano, $C_{1-6}$ alkyl, $C_{1-6}$ alkoxy, $C_{1-6}$ deuterated alkyl, $C_{1-6}$ deuterated alkoxy, $C_{1-6}$ haloalkyl and $C_{3-12}$ cycloalkyl, which can be each optionally further substituted;

$R_{6a}$, $R_{6b}$, $R_{7a}$, $R_{7b}$, $R_{8a}$ and $R_{8b}$ are each independently selected from the group consisting of hydrogen and deuterium;

n is 0, 1, 2, or 3;

with the proviso that, at least one of $R_1$, $R_2$, $R_3$, $R_4$, $R_5$, $R_9$, $R_{6a}$, $R_{6b}$, $R_{7a}$, $R_{7b}$, $R_{8a}$ and $R_{8b}$ contains D.

In a further preferred embodiment of the present invention, the compound, the stereoisomer thereof or the pharmaceutically acceptable salt thereof is further shown as general formula (I-1) or (I-2),

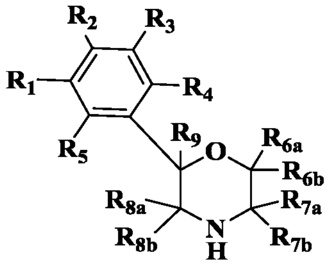 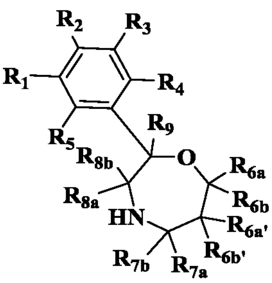

( I-1 ) ( I-2 )

In some embodiments of the present invention, $R_1$, $R_2$, $R_3$, $R_4$, $R_5$ and $R_9$ are each independently selected from the group consisting of hydrogen, deuterium, halogen, amino, hydroxyl, cyano, $C_{1-6}$ alkyl, $C_{1-6}$ alkoxy, $C_{1-6}$ deuterated alkyl, $C_{1-6}$ deuterated alkoxy, $C_{1-6}$ haloalkyl and $C_{3-12}$ cycloalkyl, which are optionally further substituted by substituents selected from the group consisting of deuterium, halogen, $C_{1-6}$ alkyl, $C_{1-6}$ alkoxy, $C_{1-6}$ deuterated alkyl, $C_{1-6}$ deuterated alkoxy and $C_{1-6}$ haloalkyl; preferably, $R_1$, $R_2$, $R_3$, $R_4$, $R_5$ and $R_9$ are each independently selected from the group consisting of hydrogen, deuterium, fluorine, chlorine, methyl, deuterated methyl, deuterated methoxy and cyclopropyl; further preferably, $R_1$, $R_2$, $R_3$, $R_4$, $R_5$ and $R_9$ are each independently selected from the group consisting of hydrogen, deuterium, methyl, fluorine, $-OCD_3$ and $-N(CH_3)_2$;

$R_{6a}$, $R_{6b}$, $R_{6a'}$, $R_{6b'}$, $R_{7a}$, $R_{7b}$, $R_{8a}$ and $R_{8b}$ are each independently selected from the group consisting of hydrogen and deuterium;

with the proviso that, at least one of $R_1$, $R_2$, $R_3$, $R_4$, $R_5$, $R_9$, $R_{6a}$, $R_{6b}$, $R_{6a'}$, $R_{6b'}$, $R_{7a}$, $R_{7b}$, $R_{8a}$ and $R_{8b}$ contains D.

In a further preferred embodiment of the present invention, the compound, the stereoisomer thereof or pharmaceutically acceptable salt thereof is further shown as general formula (I-3) or (I-4),

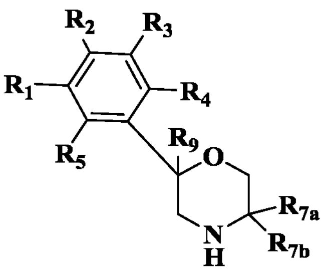

( I-3)

or

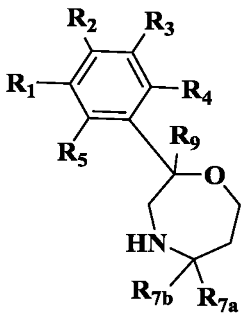

( I-4 )

$R_1$, $R_2$, $R_3$, $R_4$, $R_5$, $R_9$, $R_{7a}$, $R_{7b}$ and $R_9$ are as defined in the general formula (I-1) or (I-2).

In a further preferred embodiment of the present invention, the compound, the stereoisomer thereof or pharmaceutically acceptable salt thereof is further shown as general formula (II),

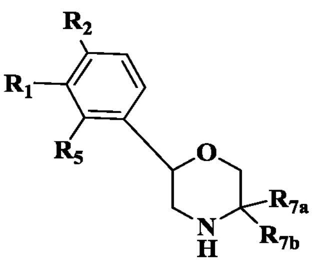

( II )

$R_1$, $R_2$ and $R_5$ are each independently selected from the group consisting of hydrogen, deuterium, halogen, amino, hydroxyl, cyano, $C_{1-3}$ alkyl, $C_{1-3}$ alkoxy, $C_{1-3}$ deuterated alkyl, $C_{1-3}$ deuterated alkoxy, $C_{1-3}$ haloalkyl and $C_{1-3}$

alkylamine;
$R_{7a}$ and $R_{7b}$ are each independently selected from the group consisting of hydrogen and deuterium;
at least one of $R_1$, $R_2$, $R_5$, $R_{7a}$ and $R_{7b}$ contains D.

In a further preferred embodiment of the present invention, the compound, the stereoisomer thereof or pharmaceutically acceptable salt thereof is further shown as general formula (II-1),

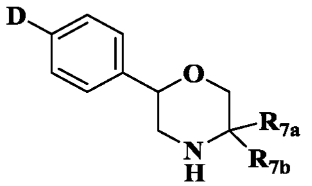

( II-1 )

$R_{7a}$ and $R_{7b}$ are each independently selected from the group consisting of hydrogen and deuterium.

In a further preferred embodiment of the present invention, in the compound, the stereoisomer thereof or the pharmaceutically acceptable salt thereof, the general formula (II-1) is further shown as general formula (II-1-a) or (II-1-b):

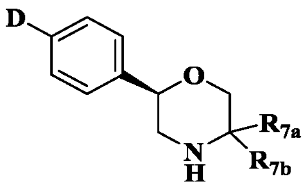

( II-1-a )

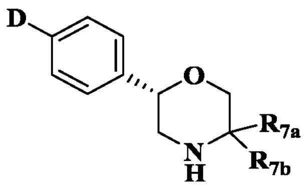

( II-1-b )

In a further preferred embodiment of the present invention, the compound, the stereoisomer thereof or the pharmaceutically acceptable salt thereof is further shown as general formula (II-2),

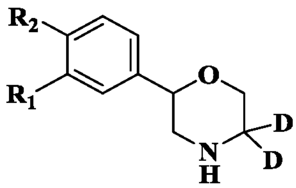

( II-2 )

$R_1$ and $R_2$ are each independently selected from the group consisting of hydrogen, deuterium, halogen, amino, hydroxyl, $C_{1-3}$ alkyl, $C_{1-3}$ alkoxy, $C_{1-3}$ deuterated alkyl, $C_{1-3}$ deuterated alkoxy, $C_{1-3}$ haloalkyl and $C_{1-3}$ alkylamine;
Preferably, $R_1$ and $R_2$ are each independently selected from the group consisting of hydrogen, deuterium, fluorine, chlorine, dimethylamino, methyl, methoxyl, deuterated methyl and deuterated methoxyl.

In a further preferred embodiment of the present invention, in the compound, the stereoisomer thereof or the pharmaceutically acceptable salt thereof, the general formula (II-2) is further shown as general formula (II-2-a) or (II-2-b):

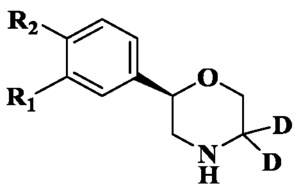 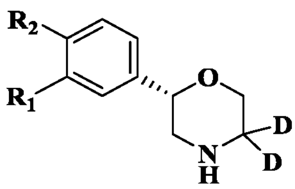

( II-2-a ) ( II-2-b ) .

Another object of the present invention is to provide a compound represented by general formula (I'), a stereoisomer or a pharmaceutically acceptable salt thereof, wherein the compound represented by general formula (I') has a structure as follows,

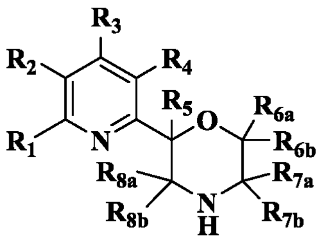

( I' )

In some embodiments of the present invention, $R_1$, $R_2$, $R_3$, $R_4$, $R_5$, $R_{6a}$, $R_{6b}$, $R_{7a}$, $R_{7b}$, $R_{8a}$ and $R_{8b}$ are each independently selected from the group consisting of hydrogen and deuterium, with the proviso that at least one of $R_1$, $R_2$, $R_3$, $R_4$, $R_5$, $R_{6a}$, $R_{6b}$, $R_{7a}$, $R_{7b}$, $R_{8a}$ and $R_{8b}$ is D.

In some embodiments of the present invention, $R_1$, $R_2$, $R_3$ and $R_4$ are each independently selected from the group consisting of hydrogen, deuterium, halogen, amino, hydroxyl, cyano, $C_{1-6}$ alkyl, $C_{1-6}$ deuterated alkyl, $C_{1-6}$ haloalkyl and $C_{3-12}$ cycloalkyl;

$R_5$,$R_{6a}$,$R_{6b}$,$R_{7a}$,$R_{7b}$,$R_{8a}$ and $R_{8b}$ are each independently selected from the group consisting of hydrogen and deuterium;

with the proviso that,

$R_1$, $R_2$, $R_3$, $R_4$, $R_5$, $R_{6a}$, $R_{6b}$, $R_{7a}$, $R_{7b}$, $R_{8a}$ and $R_{8b}$ are not hydrogen at the same time, and

when $R_1$, $R_2$, $R_3$ and $R_4$ are each independently selected from the group consisting of halogen, cyano, hydroxyl, amino, $C_{1-6}$ alkyl and $C_{1-6}$ haloalkyl, at least one of $R_5$, $R_{6a}$, $R_{6b}$, $R_{7a}$, $R_{7b}$, $R_{8a}$ and $R_{8b}$ is D.

In a further preferred embodiment of the present invention, the compound, the stereoisomer thereof or pharmaceutically acceptable salt thereof is further shown as **general formula (II'),**

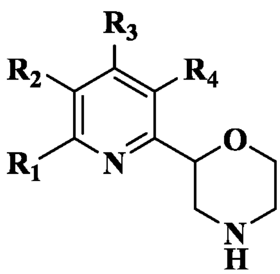

( II' )

wherein,

$R_1$, $R_2$, $R_3$ and $R_4$ are each independently selected from the group consisting of hydrogen, deuterium, halogen, amino, hydroxyl, cyano, $C_{1-6}$ alkyl, $C_{1-6}$ deuterated alkyl, $C_{1-6}$ haloalkyl and $C_{3-12}$ cycloalkyl; and

at least one of $R_1$, $R_2$, $R_3$ and $R_4$ are selected from the group consisting of deuterium, deuterated methyl and cyclopropyl.

In a further preferred embodiment of the present invention, the compound, the stereoisomer thereof or pharmaceutically acceptable salt thereof is further shown as general formula (III),

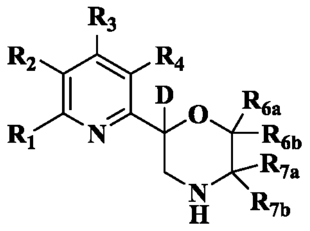

( III )

wherein,

$R_1$, $R_2$, $R_3$ and $R_4$ are each independently selected from the group consisting of hydrogen, deuterium, halogen, amino, hydroxyl, cyano, $C_{1-6}$ alkyl, $C_{1-6}$ deuterated alkyl, $C_{1-6}$ haloalkyl and $C_{3-12}$ cycloalkyl;
$R_{6a}$, $R_{6b}$, $R_{7a}$ and $R_{7b}$ are each independently selected from the group consisting of hydrogen and deuterium.

In a further preferred embodiment of the present invention, the compound, the stereoisomer thereof or pharmaceutically acceptable salt thereof is further shown as general formula (IV),

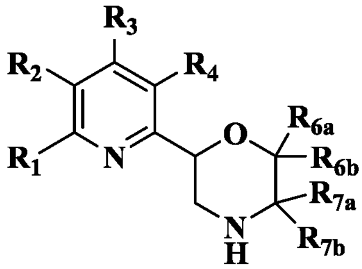

( IV )

wherein,

$R_{6a}$, $R_{6b}$, $R_{7a}$ and $R_{7b}$ are each independently selected from the group consisting of hydrogen and deuterium, and at least two of them are deuterium;
$R_1$, $R_2$, $R_3$ and $R_4$ are each independently selected from the group consisting of hydrogen, deuterium, halogen, amino, hydroxyl, cyano, $C_{1-6}$ alkyl, $C_{1-6}$ deuterated alkyl, $C_{1-6}$ haloalkyl and $C_{3-12}$ cycloalkyl.

In a further preferred embodiment of the present invention, in any of the above formulas, $R_1$, $R_2$, $R_3$ and $R_4$ are each independently selected from the group consisting of hydrogen, deuterium, halogen, $C_{1-3}$ alkyl, $C_{1-3}$ deuterated alkyl and $C_{3-6}$ cycloalkyl; preferably selected from the group consisting of hydrogen, deuterium, fluorine, chlorine, methyl, deuterated methyl and cyclopropyl.

Another object of the present invention is to provide a compound represented by general formula (V), a stereoisomer or a pharmaceutically acceptable salt thereof, wherein the compound represented by general formula (V) has a structure as follows:

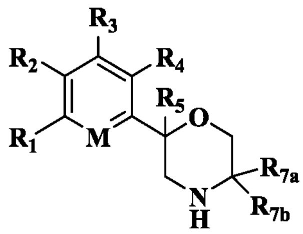

**(V)**

wherein,

M is selected from the group consisting of N and $CR_a$;
$R_a$, $R_1$, $R_2$, $R_3$ and $R_4$ are each independently selected from the group consisting of hydrogen, deuterium, halogen, amino, hydroxyl, cyano, $C_{1-6}$ alkyl, $C_{1-6}$ deuterated alkyl, $C_{1-6}$ haloalkyl and $C_{3-12}$ cycloalkyl;
$R_5$, $R_{7a}$ and $R_{7b}$ are each independently selected from the group consisting of hydrogen and deuterium, and at least one of them is D.

In a further preferred embodiment of the present invention, the general formula (V) is further shown as the compound of general formula (VI), the stereoisomer thereof or the pharmaceutically acceptable salt thereof:

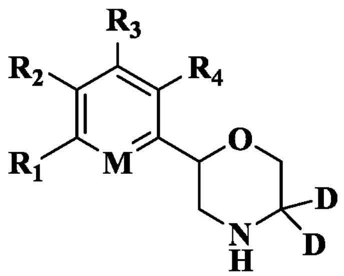

**( VI )** ,

M is selected from the group consisting of N and CH;
$R_1$, $R_2$, $R_3$ and $R_4$ are each independently selected from the group consisting of hydrogen, deuterium, halogen, $C_{1-3}$ alkyl, $C_{1-3}$ deuterated alkyl and $C_{3-6}$ cycloalkyl.

In a further preferred embodiment of the present invention, $R_1$ and $R_2$ are each independently selected from the group consisting of halogen, $C_{1-3}$ alkyl, $C_{1-3}$ deuterated alkyl and $C_{3-6}$ cycloalkyl; $R_3$ and $R_4$ are each hydrogen.
In a further preferred embodiment of the present invention, $R_1$ and $R_2$ are each independently selected from the group consisting of fluorine, $C_{1-3}$ alkyl, $C_{1-3}$ deuterated alkyl and $C_{3-6}$ cycloalkyl; $R_3$ and $R_4$ are each hydrogen.
In a further preferred embodiment of the present invention, the general formula (V) is further shown as the compound of general formula (VI-1) or (VI-2), thte stereoisomer or the pharmaceutically acceptable salt thereof:

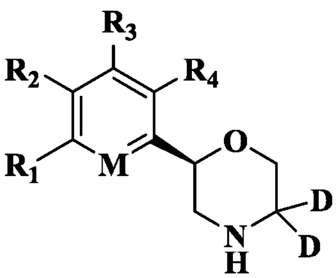

**( VI-1 )** or

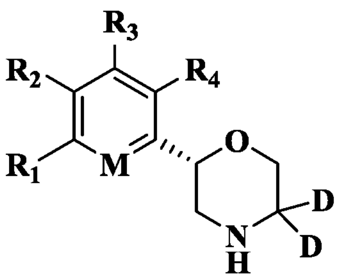

**( VI-2 )** .

In a further preferred embodiment of the present invention, the general formula (I') is further shown as the compound of general formula (V-a), the stereoisomer or the pharmaceutically acceptable salt thereof:

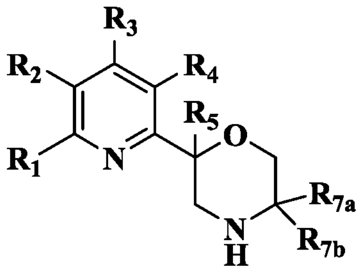

( V-a ) .

In a further preferred embodiment of the present invention, the general formula (I') is further shown as the compound of general formula (VI-a), the stereoisomer or the pharmaceutically acceptable salt thereof:

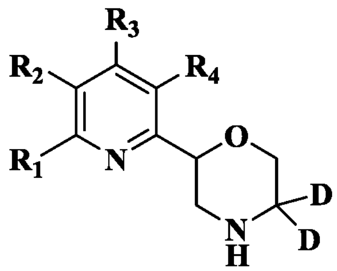

( VI-a ) ,

$R_1$, $R_2$, $R_3$ and $R_4$ are each independently selected from the group consisting of hydrogen, deuterium, halogen, $C_{1-3}$ alkyl, $C_{1-3}$ deuterated alkyl and $C_{3-6}$ cycloalkyl.

In a further preferred embodiment of the present invention, $R_1$ and $R_2$ are each independently selected from the group consisting of halogen, $C_{1-3}$ alkyl, $C_{1-3}$ deuterated alkyl and $C_{3-6}$ cycloalkyl; $R_3$ and $R_4$ are each hydrogen.

In a further preferred embodiment of the present invention, $R_1$ and $R_2$ are each independently selected from the group consisting of fluorine, $C_{1-3}$ alkyl, $C_{1-3}$ deuterated alkyl and $C_{3-6}$ cycloalkyl; $R_3$ and $R_4$ are each hydrogen.

In a further preferred embodiment of the present invention, the general formula (VI-a) is further shown as the compound of general formula (VI-a-1) or (VI-a-2), the stereoisomer or the pharmaceutically acceptable salt thereof:

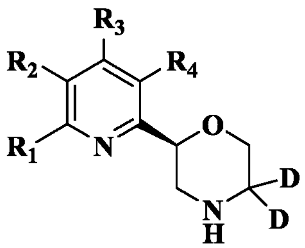

( VI-a-1 ) or

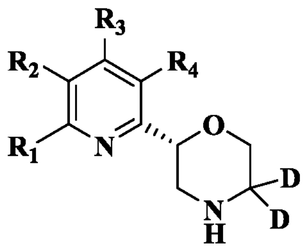

( VI-a-2 ) .

Another object of the present invention is to provide a compound represented by general formula (VII), a stereoisomer or a pharmaceutically acceptable salt thereof, wherein the compound represented by general formula (VII) has a structure as follows:

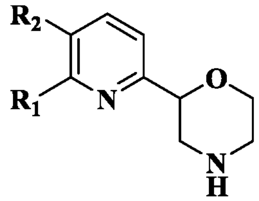

(VII) ,

$R_1$ and $R_2$ are each independently selected from the group consisting of hydrogen, deuterium, halogen, hydroxyl, cyano, $C_{1-6}$ alkyl, $C_{1-6}$ deuterated alkyl, $C_{1-6}$ haloalkyl and $C_{3-12}$ cycloalkyl, preferably halogen or $C_{1-3}$ alkyl.

In a further preferred embodiment of the present invention, the compound of general formula (VII), the stereoisomer or the pharmaceutically acceptable salt thereof is further shown as general formula (VII-1) or (VII-2):

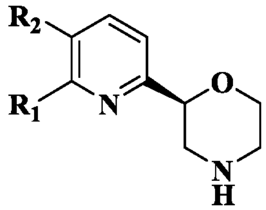

(VII-1) or

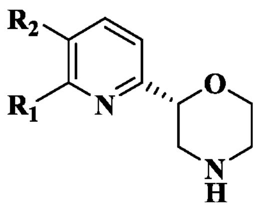

(VII-2) .

In a further preferred embodiments of the present invention, the disease related to TAAR1 is neurological disorder; wherein the neurological disorder is selected from the group consisting of schizophrenia, social dysfunction and psychosis; preferably, the schizophrenia is selected from the group consisting of schizophrenia spectrum disorders, acute schizophrenia, chronic schizophrenia, NOS schizophrenia, paranoid schizophrenia, schizoid personality disorder and schizoid personality disorder; preferably, the psychosis is selected from the group consisting of delusional disorder, brief psychotic disorder, shared psychotic disorder, mental disorders caused by physical diseases, drug-induced psychosis, psychoaffective disorder, aggression, delirium, excitative psychosis, Tourette syndrome, organic or NOS psychosis, seizure, agitation, post-traumatic stress disorder, behavior disorder, neurodegenerative disease, Alzheimer's disease, Parkinson's disease, dyskinesia, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive disorder, vertigo, epilepsy, pain, neuropathic pain, sensitization accompanying neuropathic pain, inflammatory pain, fibromyalgia, migraine, cognitive impairment, movement disorder, restless leg syndrome, multiple sclerosis, sleep disorder, sleep apnea, narcolepsy, excessive daytime sleepiness, jet lag, drowsy side effect of medications, insomnia, substance abuse dependency, addiction, eating disorder, sexual dysfunction, hypertension, emesis, Lesche-Nyhane disease, hepatolenticular degeneration, autism, and premenstrual dysphoria.

In a further preferred embodiment of the present invention, the unit dose of the compound, the stereoisomer thereof or the pharmaceutically acceptable salt thereof (in terms of free base) is 1-1000 mg; preferably 1-500 mg; for example, the dose is selected from the group consisting of 1mg, 2mg, 3mg, 4mg, 5mg, 6mg, 7mg, 8mg, 9mg, 10mg, 11mg, 12mg, 13mg, 14mg, 15mg, 16mg, 17mg, 18mg, 19mg, 20mg, 21mg, 22mg, 23mg, 24mg, 25mg, 26mg, 27mg, 28mg, 29mg, 30mg, 31mg, 32mg, 33mg, 34mg, 35mg, 36mg, 37mg, 38mg, 39mg, 40mg, 41mg, 42mg, 43mg, 44mg, 45mg, 46mg, 47mg, 48mg, 49mg, 50mg, 52.5mg, 55mg, 60mg, 65mg, 70mg, 75mg, 80mg, 85mg, 90mg, 95mg, 100mg, 105mg, 110mg, 120mg, 130mg, 140mg, 150mg, 160mg, 170mg, 180mg, 190mg, 200mg, 210mg, 220mg, 230mg, 240mg, 250mg, 260mg, 270mg, 280mg, 290mg, 300mg, 350mg, 400mg, 450mg, 500mg, 550mg, 600mg, 650mg, 700mg, 750mg, 800mg, 850mg, 900mg, 950mg and 1000mg.

In a further preferred embodiment of the present invention, the administration frequency of the compound, the stereoisomer thereof or the pharmaceutically acceptable salt thereof is once a day, twice a day, three times a day, once a week, twice a week or three times a week.

In a further preferred embodiment of the present invention, the administration of the compound, the stereoisomer thereof or the pharmaceutically acceptable salt thereof is once a day, and the unit dose is 1-500 mg, preferably 1-300mg, more preferably 1-200mg, further preferably 5-100mg, and yet preferably 20-100mg.

The administration of the present invention can be in the form of free base or salt. Unless otherwise specified,

the unit dose is calculated in terms of free base. It is known for the person skilled in the art that the dose of the administration of the present invention may be different depending on the disease being treated, the severity of the disease, the route of administration, the gender, age and general health of the subject, the use of the excipient, the likelihood of co-use with other therapeutic methods (such as the use of other drugs) and the judgment of the treating physician.

In a further preferred embodiment of the present invention, the administration route of the compound, the stereoisomer thereof or the pharmaceutically acceptable salt thereof is selected from the group consisting of intramuscular, intravenous, subcutaneous, transdermal, intradermal, intranasal, oral and sublingual.

Another object of the present invention is to provide a method for preparing the compound of general formula (VI), the stereoisomer thereof or the pharmaceutically acceptable salt thereof, wherein the method comprises the following steps:

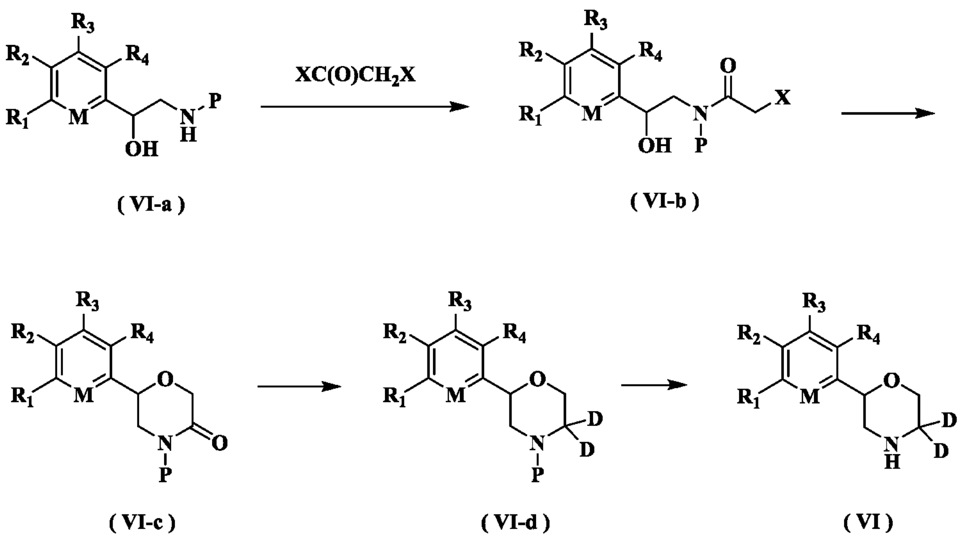


(VI-a) (VI-b) (VI-c) (VI-d) (VI)

a compound of formula (VI-a) is subject to acylation reaction to give a compound of formula (VI-b); the compound of formula (VI-b) is subject to cyclization to give a compound of formula (VI-c) under alkaline conditions; and the compound of formula (VI-c) is subject to deutration to give a compound of formula (VI-d); the compound of formula (VI-d) is subject to deprotection to give a compound of general formula (VI); optionally, a pharmaceutically acceptable salt can be further prepared;

wherein, X is halogen; P is amino protective group; $R_1$, $R_2$, $R_3$, and $R_4$ are as defined in the general formula (VI).

In a further preferred embodiment of the present invention, X is chlorine, P is Boc protective group.

The present invention further relates to a pharmaceutical composition comprising a therapeutically effective dose of any one of the above compound of the general formula, the stereoisomer thereof or the pharmaceutically acceptable salt thereof, and one or more pharmaceutically acceptable carriers, diluents or excipients.

In a further preferred embodiment of the present invention, the pharmaceutical composition is selected from the group consisting of tablet, capsule, liquid and injection.

In a further preferred embodiment of the present invention, the pharmaceutical composition is selected from the group consisting of flash-release preparation and sustained-release preparation.

In a preferred embodiment of the present invention, the pharmaceutical composition comprises filler; optionally, the pharmaceutical composition comprises disintegrant; optionally, the pharmaceutical composition comprises binder; optionally, the pharmaceutical composition comprises surfactant; optionally, the pharmaceutical composition comprises one or more of filler, disintegrant, binder, surfactant, sweetening agent, glidant and lubricant.

In a preferred embodiment of the present invention, in the pharmaceutical composition, the weight pecentage of the compound, the stereoisomer thereof or the pharmaceutically acceptable salt thereof in terms of free base is 0.1-99%, 1-95%, 2-90%, 3-80%, 5-75%, 5-60%, 5-50% and 5-40%.

In some embodiments of the present invention, in the pharmaceutical composition, the weight pecentage of the compound, the stereoisomer thereof or the pharmaceutically acceptable salt thereof in terms of free base is 1-10%, 10-20%, 20-30%, 15-30%, 20-50%, 40-70% and 50-70%.

In some embodiments of the present invention, in the pharmaceutical composition, the unit dose of the compound, the stereoisomer thereof or the pharmaceutically acceptable salt thereof in terms of free base is 1mg, 2mg, 3mg, 4mg, 5mg, 6mg, 7mg, 8mg, 9mg, 10mg, 11mg, 12mg, 13mg, 14mg, 15mg, 16mg, 17mg, 18mg, 19mg, 20mg, 21mg,

22mg, 23mg, 24mg, 25mg, 26mg, 27mg, 28mg, 29mg, 30mg, 31mg, 32mg, 33mg, 34mg, 35mg, 36mg, 37mg, 38mg, 39mg, 40mg, 41mg, 42mg, 43mg, 44mg, 45mg, 46mg, 47mg, 48mg, 49mg, 50mg, 52.5mg, 55mg, 60mg, 65mg, 70mg, 75mg, 80mg, 85mg, 90mg, 95mg, 100mg, 105mg, 110mg, 120mg, 130mg, 140mg, 150mg, 160mg, 170mg, 180mg, 190mg, 200mg, 210mg, 220mg, 230mg, 240mg, 250mg, 260mg, 270mg, 280mg, 290mg, 300mg, 350mg, 400mg, 450mg, 500mg, 550mg, 600mg, 650mg, 700mg, 750mg, 800mg, 850mg, 900mg, 950mg, 1000mg.

In some embodiments of the present invention, the compound, the stereoisomer thereof or the pharmaceutically acceptable salt thereof can be given by any convenient method, such as by oral, parenteral, buccal, sublingual, nasal, rectal, intrathecal, or transdermal administration, and the pharmaceutical composition is adjusted accordingly.

In some embodiments of the present invention, the compound, the stereoisomer thereof or the pharmaceutically acceptable salt thereof can be formulated into liquid or solid preparations, such as syrup, suspension, emulsion, tablet, capsule, powder, granule and lozenge.

Furthermore, in some embodiments of the present invention, the specific structures of the compound are as shown below:

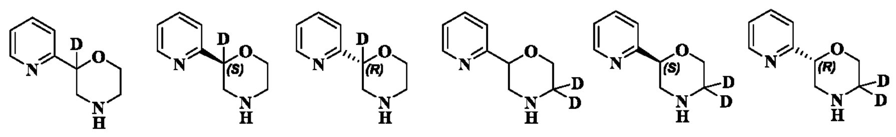

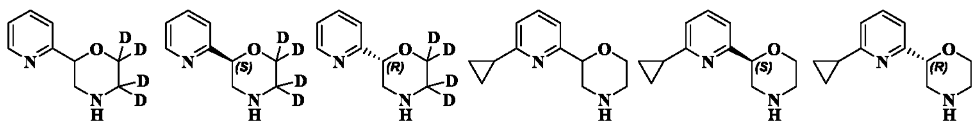

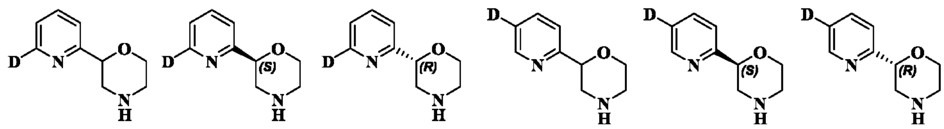

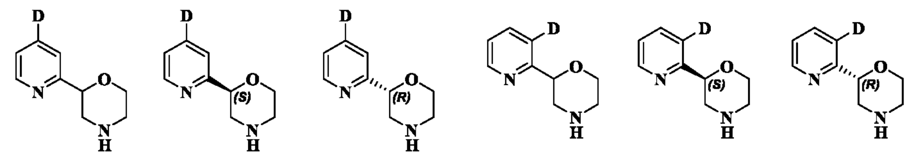

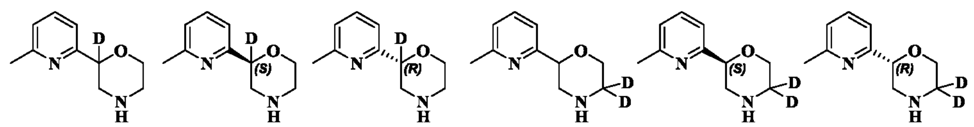

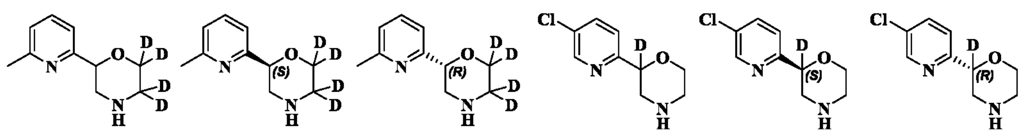

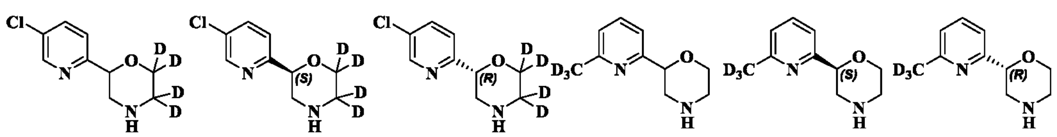

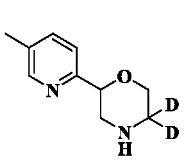 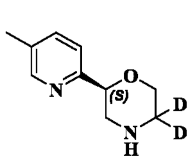 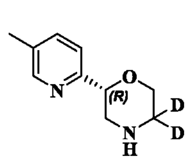 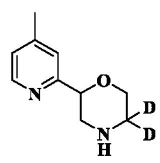 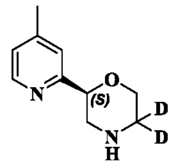

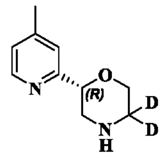 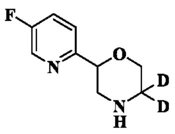 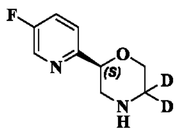 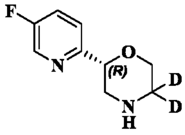 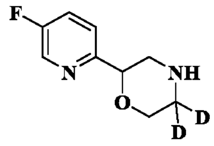

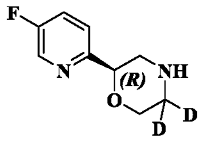 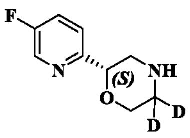 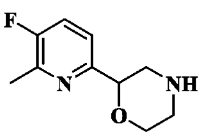 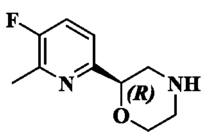 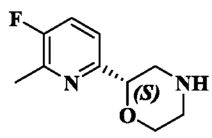

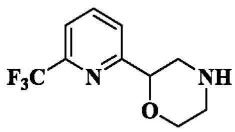 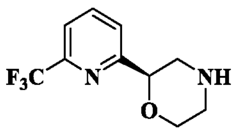 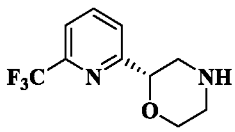 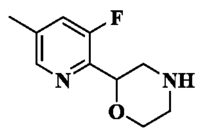

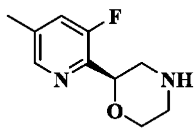 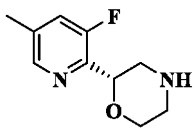 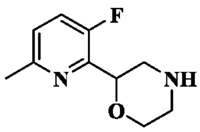 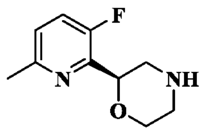 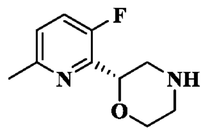

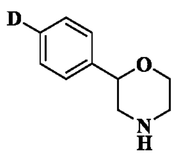 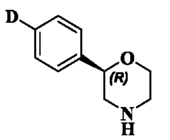 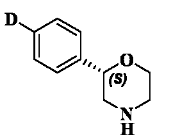 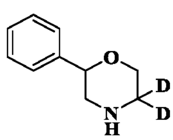 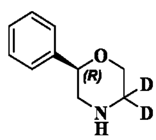 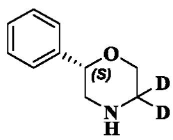

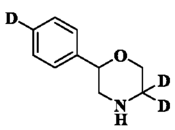 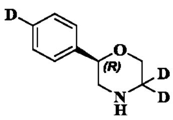 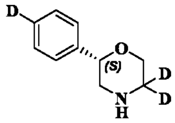 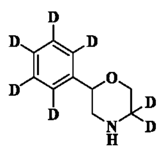 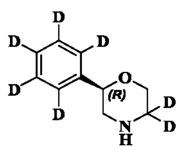 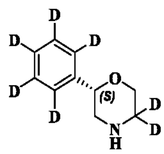

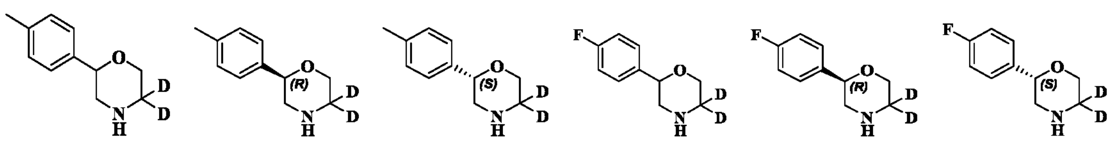

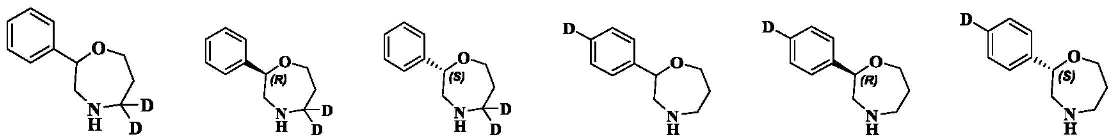

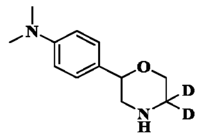 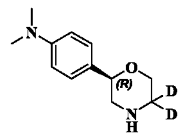 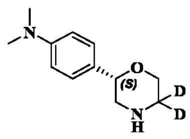 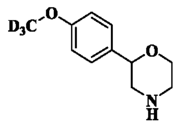 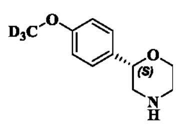

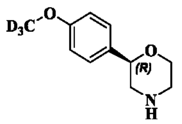 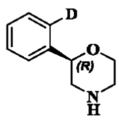 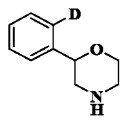 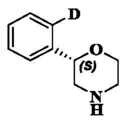 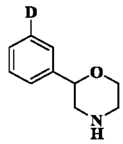 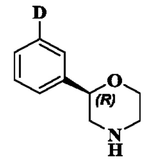 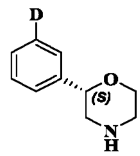

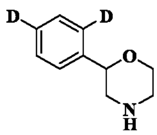 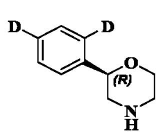 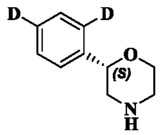 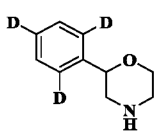 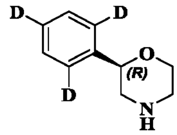 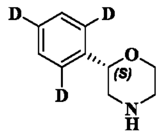

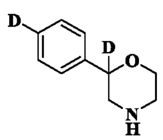 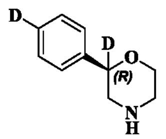 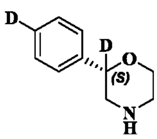 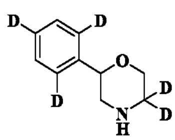 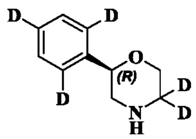 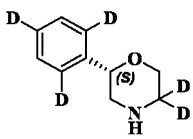

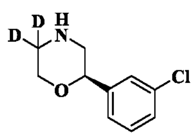 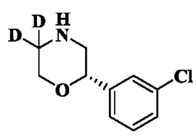 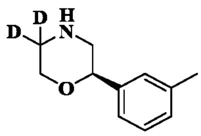 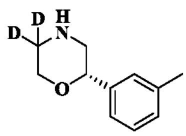 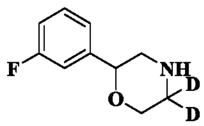

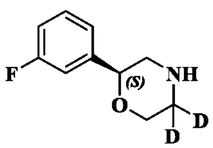 or 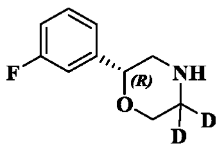 .

The present invention further relates to a use of any one of the above compound of the general formula and the specific compound, the stereoisomer thereof or the pharmaceutically acceptable salt thereof, or the pharmaceutical composition in the preparation of the medicaments for the treatment of diseases associated with TAAR1.

The present invention further relates to a use of any one of the above compound of the general formula and the specific compound, the stereoisomer thereof or the pharmaceutically acceptable salt thereof, or the pharmaceutical composition in the preparation of a TAAR1 agonist medicament.

The present invention further relates to a use of any one of the above compound of the general formula and the specific compound, the stereoisomer thereof or the pharmaceutically acceptable salt thereof, or the pharmaceutical composition in the preparation of medicaments for the treatment, prevention, or management of neurological disorders; wherein the neurological disorder is selected from the group consisting of schizophrenia, social dysfunction and psychosis; preferably, the schizophrenia is selected from the group consisting of schizophrenia spectrum disorders, acute schizophrenia, chronic schizophrenia, NOS schizophrenia, paranoid schizophrenia, schizoid personality disorder and schizoid personality disorder; preferably, the psychosis is selected from the group consisting of delusional disorder, brief psychotic disorder, shared psychotic disorder, mental disorders caused by physical diseases, drug-induced psychosis, psychoaffective disorder, aggression, delirium, excitative psychosis, Tourette syndrome, organic or NOS psychosis, seizure, agitation, post-traumatic stress disorder, behavior disorder, neurodegenerative disease, Alzheimer's disease, Parkinson's disease, dyskinesia, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive disorder, vertigo, epilepsy, pain, neuropathic pain, sensitization accompanying neuropathic pain, inflammatory pain, fibromyalgia, migraine, cognitive impairment, movement disorder, restless leg syndrome, multiple sclerosis, sleep disorder, sleep apnea, narcolepsy, excessive daytime sleepiness, jet lag, drowsy side effect of medications, insomnia, substance abuse dependency, addiction, eating disorder, sexual dysfunction, hypertension, emesis, Lesche-Nyhane disease, hepatolenticular degeneration, autism, and premenstrual dysphoria.

The present invention also relates to a method for preventing and/or treating diseases associated with TAAR1 comprising administering to the patient a therapeutically effective amount of any one of the above compound of the general formula and the specific compound or the pharmaceutically acceptable salt, or the pharmaceutical composition thereof.

The present invention also provides a method for treating a disease condition by using the compound or the pharmaceutical composition according to the present invention, wherein the disease condition includes, but is not limited to a condition related to TAAR1 dysfunction.

The present invention also relates to a method for treating diseases related to TAAR1 in a mammal, comprising administering to the mammal a therapeutically effective amount of the compound or the pharmaceutically acceptable salt, ester, prodrug, solvate, hydrate or derivative thereof according to the present invention. In an embodiment, the mammal is human.

In some embodiments, the method relates to schizophrenia, schizophrenia spectrum disorders, acute schizophrenia, chronic schizophrenia, NOS schizophrenia, paranoid schizophrenia, schizoid personality disorder and schizoid personality disorder, delusional disorder, brief psychotic disorder, shared psychotic disorder, mental disorders caused by physical diseases, drug-induced psychosis, psychoaffective disorder, aggression, delirium, excitative psychosis, Tourette syndrome, organic or NOS psychosis, seizure, agitation, post-traumatic stress disorder, behavior disorder, neurodegenerative disease, Alzheimer's disease, Parkinson's disease, dyskinesia, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive disorder, vertigo, epilepsy, pain, neuropathic pain, sensitization accompanying neuropathic pain, inflammatory pain, fibromyalgia, migraine, cognitive impairment, movement disorder, restless leg syndrome, multiple sclerosis, sleep disorder, sleep apnea, narcolepsy, excessive daytime sleepiness, jet lag, drowsy side effect of medications, insomnia, substance abuse dependency, addiction, eating disorder, sexual dysfunction, hypertension, emesis, Lesche-Nyhane disease, hepatolenticular degeneration, autism, and premenstrual dysphoria, preferably the treatment involving schizophrenia.

## DEFINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, most preferably an alkyl having 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and alkoxycarbonyl. Preferably, the alkyl of the present invention is substituted with methyl, ethyl, isopropyl, tert-butyl, haloalkyl, deuterated alkyl, alkoxy and hydroxy.

The term "alkylene" refers to an alkyl with one hydrogen being further substituted, for example, "methylene" refers to $-CH_2-$, "ethylene" refers to $-(CH_2)_2-$, "propylene" refers to $-(CH_2)_3-$, "butylene" refers to $-(CH_2)_4-$, and the like.

The term "alkenyl" refers to an alkyl group as defined above consisting of at least two carbon atoms and at least one carbon-carbon double bond, such as ethenyl, 1-propenyl, 2-propenyl, 1-, 2- or 3 -butenyl, etc. Alkenyl can be substituted or unsubstituted. When the alkenyl is substituted, the substituent is preferably one or more of groups independently selected from the group consisting of alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkoxy, heterocycloalkoxy, cycloalkylthio and heterocyclylthio.

**The term "cycloalkyl" refers to a saturated or partially unsaturated monocyclic or** polycyclic cyclic hydrocarbon substituent having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes cycloalkyl having a spiro ring, fused ring or bridged ring. The cycloalkyl is preferably cyclopropyl, cyclobutyl, cyclohexyl, cyclopentyl or cycloheptyl.

**The term "alkoxy" refers to an** -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

**The "haloalkyl" refers to an alkyl substituted by one or more halogens**, wherein the alkyl is as defined above.

**The "haloalkoxy" refers to an alkoxy substituted by one or more halogens,** wherein the alkoxy is as defined above.

**The "hydroxyalkyl" refers to an alkyl substituted by hydroxy(s), wherein the** alkyl is as defined above.

**The "hydroxy" refers to an** -OH group.

**The "halogen" refers to fluorine, chlorine, bromine or iodine.**

**The "amino" refers to** $-NH_2$.

**The "cyano" refers to** -CN.

**The "nitro" refers to** $-NO_2$.

**The** "carbonyl" **refers to** -C(O)-.

**The "carboxy" refers to** -C(O)OH.

**The "THF" refers to tetrahydrofuran.**

**Different expressions such as "X is selected from the group consisting of A, B, or C", "X is selected from the group consisting of A, B and C", "X is A, B or C", "X is A, B and C"** have the same meaning, that is, X can be any one or more of A, B and C.

The hydrogen described in the present invention all can be replaced by its isotope deuterium, and any hydrogen in a compound involved in the examples of the present invention can also be substituted by deuterium.

In some embodiments, in the compound of the present invention, each designated deuterium atom has deuterium incorporation of at least 52.5%. In some embodiments, in the compound of the present invention, each designated deuterium atom has deuterium incorporation of at least 60%. In some embodiments, in the compound of the present invention, each designated deuterium atom has deuterium incorporation of at least 67.5%. In some embodiments, in the compound of the present invention, each designated deuterium atom has deuterium incorporation of at least 75%. In some embodiments, in the compound of the present invention, each designated deuterium atom has deuterium incorporation of at least 82.5%. In some embodiments, in the compound of the present invention, each designated deuterium atom has deuterium incorporation of at least 90%. In some embodiments, in the compound of the present invention, each designated deuterium atom has deuterium incorporation of at least 95 %. In some embodiments, in the compound of the present invention, each designated deuterium atom has deuterium incorporation of at least 97%. In some embodiments, in the compound of the present invention, each designated deuterium atom has deuterium incorporation of at least 97.5%. In some embodiments, in the compound of the present invention, each designated deuterium atom has deuterium incorporation of at least 98%. In some embodiments, in the compound of the present invention, each designated deuterium atom has deuterium incorporation of at least 99%. In some embodiments, in the compound of the present invention, each designated deuterium atom has deuterium incorporation of at least 99.5%.

**"Optional" or "optionally" means that the event or circumstance described** subsequently can, but need not, occur, and such a description includes the situation in **which the event or circumstance does or does not occur. For example, "the** heterocyclyl optionally substituted with **an alkyl" means that an alkyl group can be,** but need not be, present, and such a description includes the situation of the heterocyclyl being substituted with an alkyl and the heterocyclyl being not substituted with an alkyl.

**"Substituted" refers to one or more hydrogen atoms in a group, preferably up to 5,** and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) may be unstable.

**A "pharmaceutical composition" refers to a mixture of one or more of the** compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

The pharmaceutical compositions of the invention include those suitable for oral, rectal, nasal, topical (including buccal and sublingual), vaginal or parenteral (including subcutaneous, intramuscular, intravenous and intradermal) administration. In some embodiments, the formula compound herein is administered transdermally (e.g., using a transdermal patch or ionic electroosmotic therapy technique). Other formulations may conveniently be presented in unit dosage form, e.g., tablets and sustained release capsules, and in liposomes, and may be prepared by any methods well known in the art of pharmacy.

**"Pharmaceutically acceptable salts" refer to salts of the compounds of the present** invention, which are safe and effective when used in mammals, and have appropriate biological activity.

## DETAILED DESCRIPTION OF THE INVENTION

The present invention will be further described according to the following examples. However, these examples do not limit the scope of the present invention.

### Examples

The structures of the compounds of the present invention were determined by nuclear magnetic resonance (NMR) or/and liquid chromatography-mass spectrometry (LC-MS). NMR chemical shift ($\delta$) was given in units of parts per million (ppm). NMR was determined using a Bruker AVANCE-400 NMR instrument with deuterated dimethyl sulfoxide (DMSO-$d_6$), deuterated methanol ($CD_3OD$) and deuterated chloroform ($CDCl_3$) as solvents and tetramethylsilane (TMS) as internal standard.

Liquid chromatography-mass spectrometry LC-MS was determined with an Agilent 1200 Infinity Series mass spectrometer. HPLC determinations were performed using an Agilent 1200DAD high pressure liquid chromatograph (Sunfire C18 150 × 4.6 mm column) and a Waters 2695-2996 high pressure liquid chromatograph (Gimini C18 150 × 4.6 mm column).

Yantai Huanghai HSGF254 or Qingdao GF254 silica gel plate was used as thin layer chromatography silica gel plate, the specification of TLC was 0.15 mm-0.20 mm, and the specification of thin layer chromatography separation and purification was 0.4 mm-0.5 mm. Generally, Yantai Huanghai silica gel 200-300 mesh silica gel was used as carrier for column chromatography.

The starting materials in the examples of the present invention are known and commercially available, or can be synthesized according to the methods known in the art.

Unless otherwise specified, all reactions of the present invention were carried out under continuous magnetic stirring under dry nitrogen or argon atmosphere, the solvent is a dry solvent, and the unit of the reaction temperature was degrees Celsius.

**Compound A**

**2-(Pyridin-2-yl)morpholine**

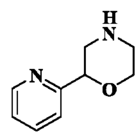

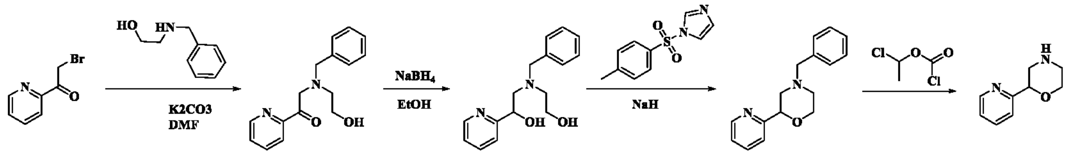


Step 1: 2-(Benzyl(2-hydroxyethyl)amino)-1-(pyridin-2-yl)ethane-1-one

2-Bromo-1-(2-pyridyl)ethanone hydrobromide (30g, 0.11mol) was dissolved in 150 mL N,N-dimethylformamide.

Potassium carbonate (36.8g, 0.27mol) and 2-(benzylamino) ethanol (17.8g, 0.12mol) were added. The reaction solution was stirred at room temperature for 18 hours. 200 mL of water was added to the reaction solution, which was then extracted with ethyl acetate (150 mL×3). The organic phase was combined, dried over anhydrous sodium sulfate, then filtered and concentrated under reduced pressure. The crude product was purified by column chromatography to obtain the title product 2-(benzyl(2-hydroxyethyl)amino)-1-(pyridine-2-yl)ethane-1-one (17 g, brown liquid), yield: 58.9%.

MS m/z (ESI):271.1 [M+1].

Step 2: 2-(Benzyl(2-hydroxyethyl)amino)-1-(pyridin-2-yl)ethane-1-ol

2-(Benzyl(2-hydroxyethyl)amino)-1-(pyridine-2-yl)ethane-1-one (15 g, 55.5 mmol) was dissolved in 200 mL anhydrous ethanol. Sodium borohydride (2.7g, 72 mmol) was added in batches. The reaction solution was stirred at room temperature for 2 hours. 200 mL of ammonium chloride was added to quench the reaction. Ethanol was removed under reduced pressure to obtain aqueous phase which was extracted with ethyl acetate (200 mL×3). The organic phase was combined, dried over anhydrous sodium sulfate, then filtered and concentrated under reduced pressure to obtain the title product 2-(benzyl(2-hydroxyethyl)amino)-1-(pyridine-2-yl)ethane-1-ol (10 g, brown liquid), yield: 66.2%.

MS m/z (ESI):273.1 [M+1].

Step 3: 4-Benzyl-2 -(pyridin-2-yl)morpholine

2-(Benzyl(2-hydroxyethyl)amino)-1-(pyridin-2-yl)ethane-1-ol (1.1g, 4mmol) was dissolved in 20mL anhydrous tetrahydrofuran, and sodium hydride was added in batches (0.2g, 8mmol). The reaction was stirred at room temperature for 1 hour. 1-Toluenesulfonyl-1H-imidazole was added in batches (1.0g, 4.5mmol) under ice bath, and stirred at room temperature for 18 h. 50 mL of water was added to the reaction solution which was extracted with ethyl acetate (50mL×3). The organic phase was combined, dried over anhydrous sodium sulfate, then filtered and concentrated under reduced pressure. The crude product was purified by column chromatography to obtain the title product 4-benzyl-2-(pyridin-2-yl)morpholine (0.4g, colorless gum), yield: 38.9%.

Step 4: 2-(Pyridin-2-yl(morpholine

4-Benzyl-2-(pyridin-2-yl)morpholine (0.4g, 1.6mmol) was dissolved in 10 mL anhydrous dichloromethane, and 1-chloroethyl chlorate (0.34g, 2.4mmol) was added. The reaction solution was stirred at room temperature for 4 hours. The solvent was dried under reduced pressure. 5mL methanol was added. The reaction solution was stirred under reflux for 1 hour, and cooled to room temperature. The solvent was dried under reduced pressure. 10mL saturated sodium carbonate solution was added to the reaction. The aqueous phase was extracted with ethyl acetate (20 mL×3). The organic phase was combined, dried over anhydrous sodium sulfate, then filtered and concentrate under reduced pressure. The crude product was purified by column chromatography to obtain the title product 2-(pyridin-2-yl)morpholine (0.18g, colorless gum), yield: 69.7%.

MS m/z (ESI):165.1 [M+1].

**Example 1**

**2-(Pyridin-2-yl)morpholine-2-d**

Step 1: 2-(Benzyl(2-hydroxyethyl)amino)-1-(pyridin-2-yl)ethane-1-one

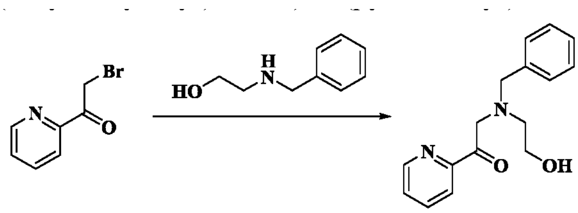

2-Bromo-1-(2-pyridyl) ethanone (30g, 106.8mmol, HBr) and 2-(benzylamino)ethanol (17.876g, 117.5mmol) were dissolved in N,N-dimethylformamide (150mL), and potassium carbonate (36.8g, 267.0mmol) was added. The reaction was stirred at room temperature for 18 hours. Ammonium chloride solution (1000mL) was added. The aqueous

phase was extracted with EA (500 mL × 2). The organic phase was dried over anhydrous sodium sulfate, then filtered and concentrated under reduced pressure. The crude product was purified by column chromatography to obtain 2-(benzyl(2-hydroxyethyl) amino)-1-(pyridine-2-yl)ethane-1-one (17g). Yield: 59%.

MS m/z (ESI): 271.14[M+1].

Step 2: 2-(Benzyl(2-hydroxyethyl)amino)-1-(pyridine-2-yl) ethane-1-d-1-ol-d

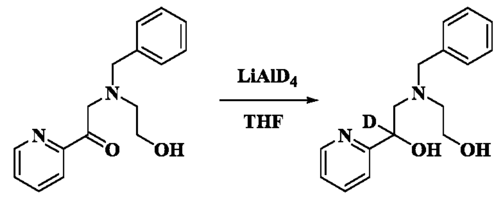


2-(Benzyl(2-hydroxyethyl)amino)-1-(pyridin-2-yl)ethane-1-one (12g, 44.4mmol) was dissolved in tetrahydrofuran (150mL), and deuterinated aluminum lithium hydrogen (3.7g, 88.8mmol) was added at 0°C. The reaction was stirred at room temperature for 18 h. Sodium sulfate decahydrate was added to quench the reaction. Ethyl acetate was added for dilution, which was then filtered, and the filter cake was washed with ethyl acetate. The organic phase was dried over anhydrous sodium sulfate, then filtered and concentrated under reduced pressure. The crude product was purified by column chromatography to obtain 2-(benzyl(2-hydroxyethyl)amino)-1-(pyridin-2-yl)ethane-1-d-1-ol-d (3.98g). Yield: 32.8%.

MS m/z (ESI):274.16[M+1].

Step 3: 4-Benzyl-2-(pyridin-2-yl)morpholine-2-d

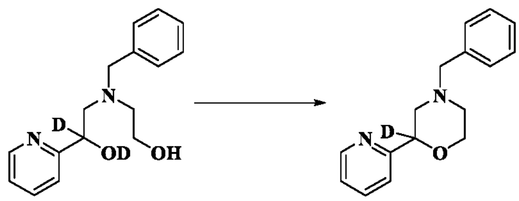

2-(Benzyl(2-hydroxyethyl)amino)-1-(pyridin-2-yl)ethane-1-d-1-ol-d (4g, 14.6 mmol) was dissolved in tetrahydrofuran (40mL) and replaced with nitrogen. Sodium hydride (1.2g, 30.6mmol, 60% purity) was added. The reaction was stirred at room temperature for one hour, cooled to 0°C, and 1-(p-toluenesulfonyl)imidazole (3.9g, 17.5mmol) was added in batches. The reaction was warmed slowly to room temperature and then stirred for 18 h. A saturated ammonium chloride aqueous solution (50mL) was added. The aqueous phase was extracted with ethyl acetate (50mL × 2). The organic phase was dried over anhydrous sodium sulfate, then filtered and concentrated under reduced pressure. The crude product was purified by column chromatography to obtain 4-benzyl-2-(pyridin-2-yl)morpholin-2-d (1.4g), yield: 37.6%.

MS m/z (ESI):256.15 [M+1].

Step 4: 2-(Pyridin-2-yl)morpholine-2-d

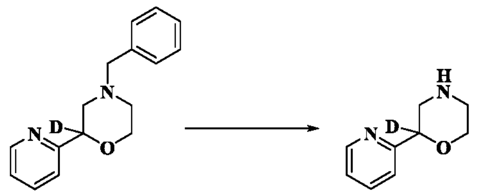

4-Benzyl-2-(pyridin-2-yl) morpholine-2-d (1.4g, 5.5mmol) was dissolved in anhydrous dichloromethane (30mL) and 1-chloroethyl chlorate (2.4g, 16.5mmol) was added. The reaction was stirred at room temperature for 18h. The solvent was concentrated to obtain 2-(pyridin-2-yl) morpholine-2-d (0.9g), yield: 99.4%, which was directly used in the next step.

$^1$H NMR (400 MHz, DMSO-$d_6$): δ 8.50-8.48 (m, 1H), 7.80-7.76 (m, 1H), 7.43-7.41 (m, 1H), 7.29-7.26 (m, 1H), 3.91-3.88 (m, 1H), 3.66-3.59 (m, 1H), 3.12-3.09 (m, 1H), 2.76-2.71 (m, 2H), 2.52-2.49 (m, 2H).

MS m/z (ESI):166.10[M+1].

Step 5: (*S*) -2-(Pyridin-2-yl)morpholine-2-d and (*R*)-2-(pyridin-2-yl) morpholine-2-d

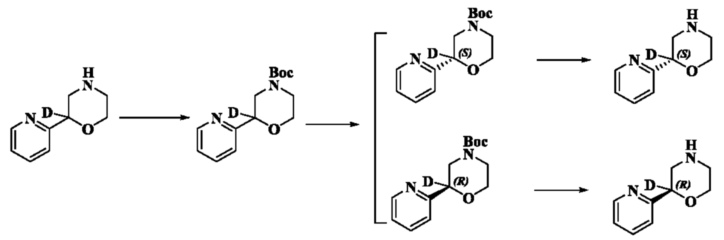

1-P1/1-P2 1-1/1-2

2-(Pyridin-2-yl)morinoline-2-d (0.9g, 5.5mmol) was dissolved in dichloromethane (30 mL), then triethylamine (2.8g, 27.2mmol) and di-tert-butyl dicarbonate (2.4g, 10.9mmol) were added. The reaction was stirred at room temperature for 18 hours. Water (30mL) was added. The aqueous phase was extracted with dichloromethane (30mL × 2). The organic phase was dried over anhydrous sodium sulfate, then filtered and concentrated under reduced pressure. The crude product was purified by column chromatography to obtain tert-butyl 2-(pyridin-2-yl) morinolin-4-carboxylate-2-d (0.8g), yield:55.4%. The compound was further resolved **by chiral column (CHIRALCEL OD 4.6*150mm, 5μm; Detection wavelength (nm):** 214; Temperature (°C):35; Flow rate (mL/min):1; Mobile phase: Hexane:IPA =70:30; Running time (min):8; Linear mode: isocratic), to obtain compound 1-P1 (300mg, RT(retention time) = 2.487 min) and compound 1-P2 (300 mg, RT(retention time)=3.377 min).

MS m/z (ESI):266.15 [M+1].

Compound 1-P1 or compound 1-P2 (0.3 g, 1.1 mmol) was dissolved in 1,4-dioxane (1 mL), then hydrochloric acid/dioxane (4 M, 5 mL) was added. The reaction was stirred at room temperature for 1 hour. The solvent was concentrated under reduced pressure. Saturated sodium bicarbonate aqueous solution (1mL) was added. The aqueous phase was extracted with methylene chloride (3mL*3). The organic phase was dried by oil pump. Compound 1-1 (200 mg) was obtained from compound 1-P1 and Compound 1-2 (200 mg) was obtained from compound 1-P2.

Compound 1-1 : $^1$H NMR (400 MHz, DMSO-$d_6$): δ **8.50-**8.48 (m, 1H), 7.80-7.76 (m, 1H), 7.43-7.41 (m, 1H), 7.29-7.26 (m, 1H), 3.91-3.88 (m, 1H), 3.66-3.59 (m, 1H), 3.12-3.09 (m, 1H), 2.76-2.71 (m, 2H), 2.52-2.49 (m, 2H).

MS m/z (ESI):166.10[M+1].

Compound 1-2: $^1$H NMR (400 MHz, DMSO-$d_6$): δ **8.50**-8.48 (m, 1H), 7.80-7.76 (m, 1H), 7.43-7.41 (m, 1H), 7.29-7.26 (m, 1H), 3.91-3.88 (m, 1H), 3.66-3.59 (m, 1H), 3.12-3.09 (m, 1H), 2.76-2.71 (m, 2H), 2.52-2.49 (m, 2H).

MS m/z (ESI):166.10[M+1].

**Example 2**

**2-(Pyridin-2-yl)morpholine-5,5-d2**

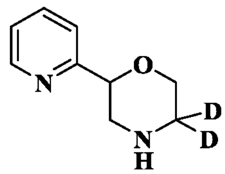

2-(Pyridin-2-yl)morpholine-5,5-d2 was obtained according to Example 1 with 2-(benzylamino)ethane-2,2-d2-1-ol as the starting material.

MS: 167.10[M+1].

**Example 3**

**2- (Pyridin-2-yl)morpholine-5,5,6,6-d4**

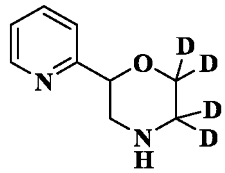

2-(Pyridin-2-yl) morpholine-5,5,6,6-d4 was obtained according to Example 1 with 2-(benzylamino)ethane-1,1,2,2-d4-1-ol as the starting material.

MS: 169.10[M+1].

**Example 4**

**2-(6-Cyclopropylpyridin-2-yl)morpholine**

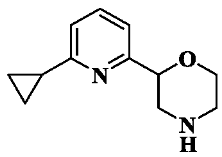

Step 1: 1-(6-Cyclopropylpyridin-2-yl)ethane-1-one

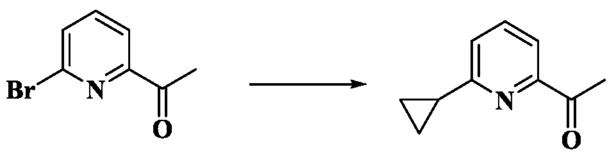

1-(6-Bromo-2-pyridyl)ethanone (6.0g, 30.00mmol), cyclopropyl boronic acid (3.86g, 44.99mmol), $Pd(dppf)Cl_2.CH_2Cl_2$ (1.23g, 1.50mmol) and potassium phosphate (15.92g, 74.99mmol) were dissolved in tetrahydrofuran (60 mL) at room temperature, and then replaced by nitrogen. The reaction was heated to 80°C for 4 hours, and cooled to room temperature. LCMS indicates the end of the reaction. The reaction solution was diluted with ethyl acetate and filtered. The filter residues were washed with ethyl acetate. The organic phase was combined, then washed with saturated NaCl aqueous solution. The organic phase was dried over anhydrous sodium sulfate, then filtered and concentrated under reduced pressure. The crude product was purified by column chromatography (petroleum ether: ethyl acetate: 10: 1) to obtain the product which was colorless oil (3.80g), yield: 78.6%.

MS: 162.08[M+1].

Step 2: 2-Bromo-1-(6-cyclopropylpyridin-2-yl)ethane-1-one

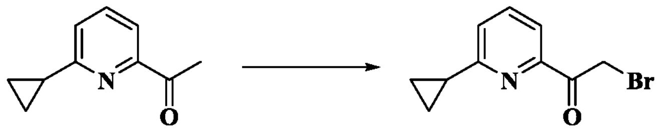

1-(6-Cyclopropylpyridin-2-yl)ethane 1-one (1.0g, 6.20mmol) was dissolved in HBr/HOAc (6.20mmol, 6mL, 33% purity) at room temperature, then liquid bromine (0.99g, 6.20mmol, 0.4mL) was added. The reaction was stirred at room temperature for 10 minutes, and then heated to 40°C for two hours. The reaction was cooled to room temperature. LCMS indicated generation of the target product. Ether was added to the reaction, which was then stirred at room temperature for half an hour. A solid was precipitated, and then filtered. The solid was washed with ether. The filtrate was concentrated under reduced pressure to obtain a yellow solid (1.99g), yield: 100%.

MS: 239.99, 241.99[M+1].

Step 3: 2-(Benzyl(2-hydroxyethyl)amino)-1-(6-cyclopropylpyridin-2-yl)ethane-1-one

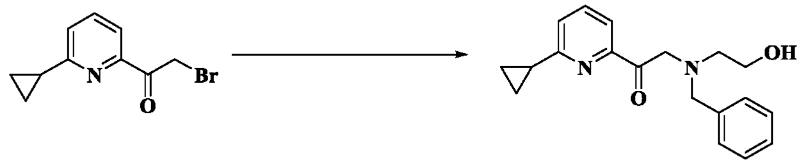

2-Bromo-1-(6-cyclopropylpyridin-2-yl)ethane-1-one (1.99g, 6.20mmol hydrobromide) was dissolved in N,N-dimethylformamide (20mL) at room temperature, then potassium carbonate (3.00g, 21.70mmol) and 2-(benzyl amino)ethanol (1.12 g, 7.44 mmol) were added. The reaction was stirred at room temperature for 12 hours. TLC indicated the end of the reaction. The reaction solution was diluted with ethyl acetate, then washed with saturated sodium chloride solution. The organic phase was dried over anhydrous sodium sulfate, filtered and concentrated under reduced pressure. The crude product was purified by column chromatography (petroleum ether: ethyl acetate = 1:1) to obtain the target product which was yellow oil (0.90g), yield: 46.8%.

MS: 311.17[M+1].

Step 4:2-(Benzyl(2-hydroxyethyl)amino)-1-(6-cyclopropylpyridin-2-yl)ethane-1-ol

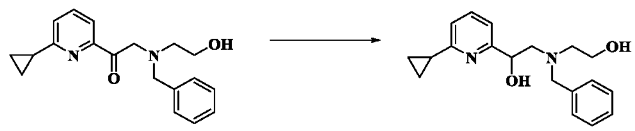

2-(BenzylBenzyl(2-hydroxyethyl)amino)-1-(6-cyclopropylpyridin-2-yl)ethane-1-one (0.9g, 2.90mmol) was dissolved in methanol (10 mL) under ice bath, then sodium borohydride (0.13 g, 3.48mmol) was added. The reaction was stirred at room temperature for 1 hour. LCMS indicated the end of the reaction. The solvent was concentrated under reduced pressure. The residues were dissolved in ethyl acetate, then washed with saturated ammonium chloride solution and saturated sodium chloride solution successively. The organic phase was dried over anhydrous sodium sulfate, then filtered and concentrated under reduced pressure. The residues were purified by flash silica gel chromatography (petroleum ether: ethyl acetate = 70:30 to 30: 70 elution) to obtain the target product which was a yellow oil (0.55g), yield: 60.7%.

MS: 313.17[M+1].

Step 5: 4-Benzyl-2-(6-cyclopropylpyridin-2-yl)morpholine

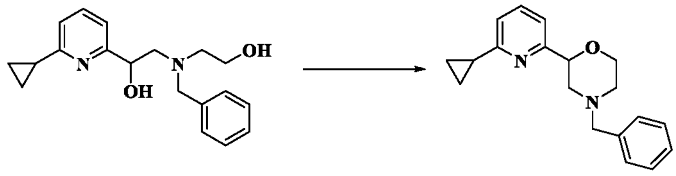

2-(Benzyl(2-hydroxyethyl) amino)-1-(6-cyclopropylpyridin-2-yl)ethane-1-ol (0.55g, 1.76mmol) was dissolved in tetrahydrofuran (5mL) at room temperature, and then replaced by nitrogen. Sodium hydrogen (84.50mg, 3.52mmol, 60%) was added. The reaction was stirred at room temperature for 1 hour, then a solution of 1-(4-toluensulfonyl) imidazole (0.43g, 1.94mmol) in tetrahydrofuran (2 mL) was added dropwise. The reaction was stirred at room temperature for 2 hours. TLC indicated the end of the reaction. The reaction solution was quenched with saturated ammonium chloride solution. The aqueous phase was extracted with ethyl acetate. The organic phase was combined, dried over anhydrous sodium sulfate, filtered and concentrated under reduced pressure. The crude product was purified by thin layer chromatography (PE: EA= 4:1) to obtain the target product which was yellow oil (0.30g), yield: 57.9%.
MS: 295.17[M+1].

Step 6: 2-(6-cyclopropylpyridin-2-yl)morpholine

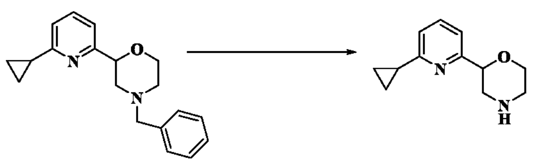

4-Benzyl-2-(6-cyclopropylpyridin-2-yl)**morpholine (132.0mg, 448.38μmol) was** dissolved in dichloromethane (5mL) at room temperature, then 1-chloroethyl chloroformate (192.32 mg, 1.35 mmol) was added dropwise and stirred at room temperature for 2 h. The reaction solution was concentrated under reduced pressure. The residues were dissolved in methanol. The reaction was heated to 70°C for 1 hour and then cooled to room temperature. LCMS indicated the end of the reaction. The reaction solution was concentrated under reduced pressure. The residues were directly used in the next step (107 mg), yield: 99.1%.
MS: 205.17[M+1].

Step 7: Tert-butyl-2-(6-cyclopropylpyridin-2-yl)morpholin-4-carboxylate

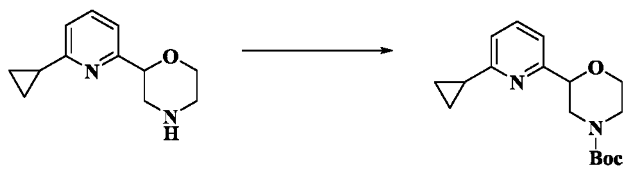

2-(6-Cyclopropylpyridin-2-**yl) morpholine (107mg, 444.48μmol, hydrochloride)** was dissolved in dichloromethane (5mL) at room temperature, then N,N-diethylethylamine (224.89mg, 2.22mmol) and tert-butoxycarbonyltert-butyl carbonate **(179.83mg, 888.97μmol) were added**. The reaction was stirred at room temperature for 12 hours. LCMS indicated the end of the reaction, and the reaction solution was diluted with dichloromethane, then washed with saturated sodium chloride solution. The organic phase was dried over anhydrous sodium sulfate, filtered and concentrated

under reduced pressure. The residues were purified by thin layer chromatography (PE: EA= 10:1) to obtain the target product which was colorless oil (118 mg), yield: 87.2%.

MS: 249.39[M-55].

Step 8: (*R*)-2-(6-cyclopropylpyridin-2-yl)morpholine and (5)-2-(6-cyclopropylpyri din-2-yl)morpholine

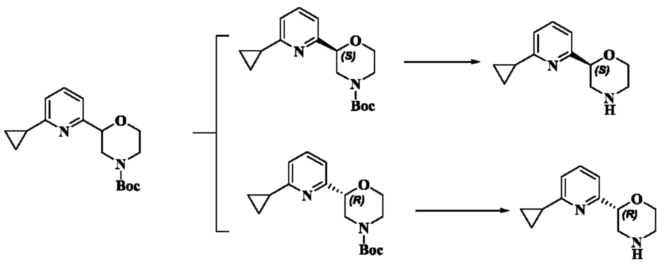

4-P1/4-P2

4-1/4-2

The compound tert-butyl 2-(6-cyclopropylpyridin-2-yl) morpholin-4-carboxylate was resolved by chiral column **(CHIRALCEL OD 4.6*150mm, 5μm; Detection** wavelength (nm): 214; Temperature (°C): 35; Flow rate(mL/min): 1; Mobile phase: Hexane : IPA = 90:10; Running time (min): 10; Linear mode: isocratic). Compound 4-P1 (0.05g, RT(retention time)= 2.690 min) and compound 4-P2 (0.04 g, RT(retention time)= 3.410 min) were obtained.

The compound 4-P1 was dissolved in 1,4-dioxane, then hydrochloric acid/dioxane (4 M) was added. The reaction was stirred at room temperature for 1 hour. The solvent was concentrated under reduced pressure. Saturated sodium bicarbonate aqueous solution was added. The aqueous phase was extracted with dichloromethane. The organic phase was dried by oil pump to obtain compound 4-1. Compound 4-P2 was subject to Boc deprotection by the same method to obtain compound 4-2.

Compound 4-1, MS: 205.17[M+1].

Compound 4-2, MS: 205.17[M+1].

**Example 5**

**2-(Pyridin-2-yl-6-d)morpholine**

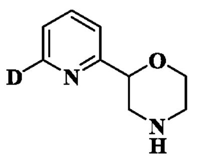

Step 1: Preparation of 2-(6-chloropyridin-2-yl)morpholine

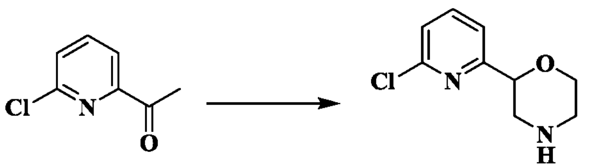

2-(6-Chloropyridin-2-yl)morpholine was prepared by the well-known method" WO2015/165835" with 1-(6-chlorpyridin-2-yl)ethane-1-one as the starting material.

MS: 199.06[M+1].

Step 2: 2-(Pyridin-2-yl-6-d)morpholine

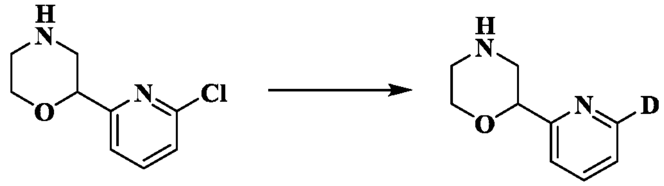

2-(6-Chlorpyridin-2-yl)morpholine (300mg, 1.51mmol) and cesium carbonate (980mg, 3.02mmol) were dissolved in deuterated methanol (7mL) under nitrogen atmosphere, then 1,1'-bis(diphenylphosphino)ferrocene]dichloropalladium (110.38mg, 0.15mmol) was added. The mixture was stirred at 80 °C for 16 hours, and the reaction solution was concentrated under reduced pressure. The crude product was separated by high pressure liquid preparative chromatography to obtain 2-(pyridin-2-yl-6-d)morpholine (35mg, yield 14.0%).
MS: 166.10[M+1].

**Example 6**

**(*R*)-2-(pyridin-2-yl-5-d)morpholine and (*S*)-2-(pyridin-2-yl-5-d) morpholine**

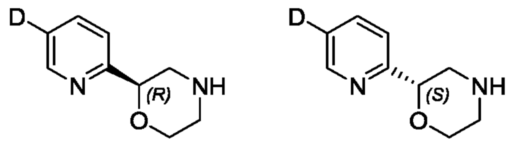

Step 1: The preparation of 2-bromo-1-(5-chloropyridin-2-yl)ethane-1-one

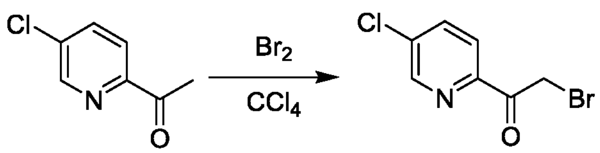


1-(5-Chloropyridin-2-yl)ethane-1-one (10 g, 64.3mmol) was dissolved in carbon tetrachloride (100 mL), and liquid bromine (10.3g, 64.3mmol) was added. The reaction was stirred at 70°C for 18 h. The solvent was concentrated under reduced pressure. Water (30mL) was added. The aqueous phase was extracted with ethyl acetate (30 mL × 2). The organic phase was dried over anhydrous sodium sulfate, then filtered, and the filtrate was concentrated under reduced pressure to obtain the crude product 2-bromo-1-(5-chloropyridin-2-yl)ethane-1-one (15 g, yield 99.5%).

Step 2: Preparation of 2-(benzyl (2-hydroxyethyl) amino)-1-(5-chloropyridin-2-yl)ethane-1-one

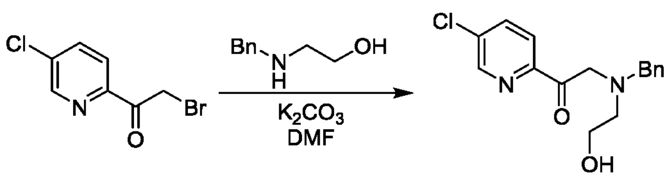

2-Bromo-1-(5-chloropyridin-2-yl)ethane-1-one hydrobromide (15g, 64.0mmol) and 2-(benzylamino) ethanol (10.6g, 70.4mmol) were dissolved in N,N-dimethylformamide (200 mL), and potassium carbonate (22.1g, 160mmol) was added. The reaction was stirred at 25°C for 18 hours. Water (1000 mL) was added. The aqueous phase was extracted with ethyl acetate (500 mL × 2). The organic phase was dried over anhydrous sodium sulfate, then filtered and concentrated under reduced pressure. The crude product was separated by silica gel column chromatography to obtain the title product 2-(benzyl(2-hydroxyethyl)amino)-1-(5-chloropyridin-2-yl)ethane-1-one (11 g, yield 56.4%).
MS m/z (ESI): 305.1 [M+H].

Step 3: Preparation of 2-(benzyl (2-hydroxyethyl) amino)-1-(5-chloropyridin-2-yl)ethane-1-ol

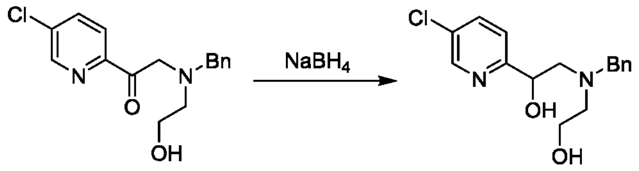


2-(Benzyl(2-hydroxyethyl)amino)-1-(5-chlorpyridin-2-yl)ethane-1-one (11g, 36.1mmol) was dissolved in ethanol (200 mL), and sodium borohydride (1.78 g, 46.9 mmol) was added in batches. The reaction was stirred at 20°C for 2 hours. The solvent was concentrated under reduced pressure. Water (100 mL) was added, and the aqueous phase was extracted with ethyl acetate (100 mL × 3). The organic phased was dried over anhydrous sodium sulfate, then filtered and concentrated under reduced pressure. The crude product was separated by silica gel column chromatography to obtain the title product 2-(benzyl(2-hydroxyethyl)amino)-1-(5-chloropyridin-2-yl) ethane-1-ol (11 g, 99% yield).
MS m/z (ESI): 307.1 [M+H].

Step 4: Preparation of 4-benzyl-2-(5-chloropyridin-2-yl)morpholine

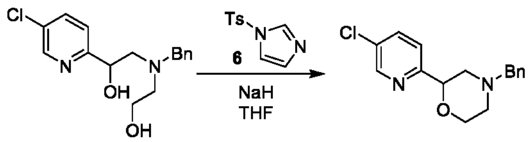


2-(Benzyl(2-hydroxyethyl)amino)-1-(5-chlorpyridin-2-yl)ethane-1-ol (5g, 16.3mmol) was dissolved in tetrahydrofuran (100 mL), and then replaced with nitrogen. Sodium hydrogen (1.37g, 34.2mmol, 60% purity) was added. The reaction was stirred at this temperature for one hour and then cooled to 0°C. 1-(p-Toluenesulfonyl)imidazole was added in batches (4.35g, 19.6mmol), then the reaction was warmed slowly to room temperature and stirred for 18 h. Saturated ammonium chloride (100 mL) was added. The aqueous phase was extracted with ethyl acetate (100 mL × 2). The organic phase was dried over anhydrous sodium sulfate, then filtered and concentrated under reduced pressure. The crude product was separated by silica gel column chromatography to obtain 4-benzyl-2-(5-chlorpyridin-2-yl)morpholine (1.4 g, yield 29.8%).
MS m/z (ESI): 289.1 [M+H].

Step 5: Preparation of 4-benzyl-2-(pyridin-2-yl-5-d)morpholine

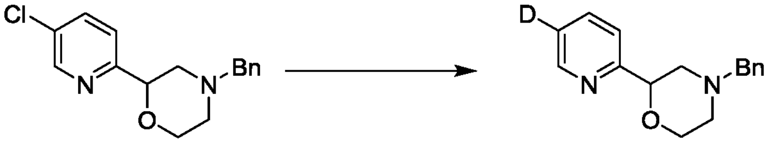

4-Benzyl-2-(5-chloropyridin-2-yl)morpholine (0.8g, 2.8mmol), 1,1'-Bis(diphenylphosphino)ferrocene]dichloropalladium (3.9mg, 5.3umol), and cesium carbonate (2.7g, 8.3mmol) were dissolved in deuterated methanol (10 mL), and then replaced with nitrogen. The reaction was stirred at 75°C for 18 hours. Water (50 mL) was added. The aqueous phase was extracted with ethyl acetate (30 mL × 2). The organic phase was washed with saturated NaCl solution (30 mL), dried over anhydrous sodium sulfate, then filtered and concentrated under reduced pressure. The crude product was separated by silica gel column chromatography to obtain 4-benzyl-2-(pyridin-2-yl-5-d)morpholine (0.6 g, yield 84.8%).

MS m/z (ESI): 256.1 [M+H].

Step 6: Preparation of 2-(pyridin-2-yl-5-d)morpholine

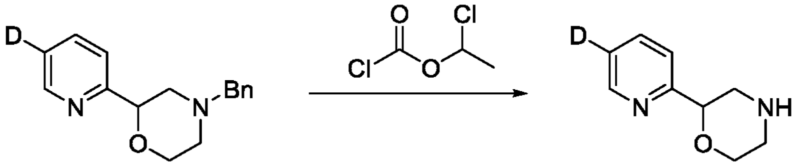

4-Benzyl-2-(pyridin-2-yl-5-d)morpholine (0.6g, 2.4mmol) and 1-chloroethyl chlorate (1.0g, 7. 1mmol) were dissolved in dichloromethane (10 mL), and the reaction was stirred at 15°C for 18 h. The solvent was concentrated under reduced pressure to obtain the crude product 2-(pyridin-2-yl-5-d)morpholine (0.38 g, yield: 97.9%).

MS m/z (ESI): 166.1 [M+H].

Step 7: Preparation of tert-butyl 2-(pyridin-2-yl-5-d)morpholin-4-carboxylate

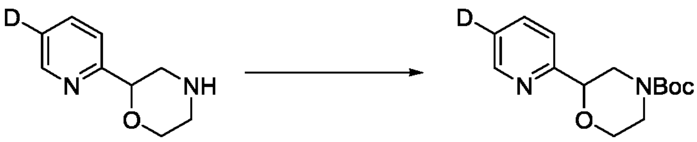

2-(Pyridin-2-yl-5-d)morpholine (0.38 g, 2.30mmol) was dissolved in dichloromethane (10 mL). N,N-diethylethylamine (1.16g, 11.50mmol) and di-tert-butyl dicarbonate (1.5g, 6.9mmol) were added, The reaction was stirred at 10°C for 2 hours. Water (30 mL) was added. The aqueous phase was extracted with ethyl acetate (30 mL × 2). The organic phase was dried over anhydrous sodium sulfate, then filtered and concentrated under reduced pressure. The crude product was separated by silica gel column chromatography to obtain tert-butyl 2 -(pyridin-2-yl-5-d)morpholin-4-carboxylate (0.5 g, yield 81.9%). Then the compounds were resolved by chiral column (CHIRALCEL OD**-H 4.6* 150mm, 5μm; Detection** wavelength (nm): 214; Temperature (°C): 35; Flow rate (mL/min): 1; Mobile phase: Hexane : IPA = 70 : 30; Running time (min): 8; Linear mode: isocratic), to obtain the product P1 (200 mg, RT(retention time)=2.490 min, yield: 32.8%) and product P2 (200 mg, RT(retention time)=3.373 min, yield: 32.8%).

P1, MS m/z (ESI): 266.1 [M+1].

P2, MS m/z (ESI): 266.1 [M+1].

Step 8: Preparation of *(R)* -2-(pyridin-2-yl-5-d)morpholine and (*S*)-2-(pyridin-2-yl-5-d)morpholine

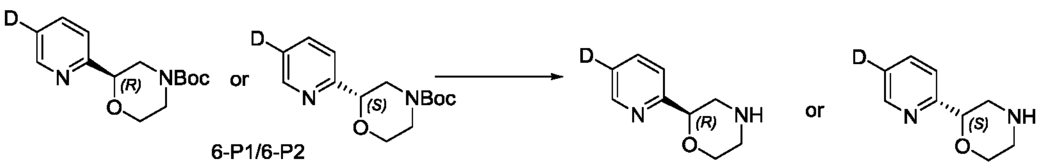
or
6-P1/6-P2
or
6-1/6-2

P1 (0.2g, 0.75mmol) and P2 (0.2g, 0.75mmol) of tert-butyl 2-(pyridin-2-yl-5-d) morpholin-4-carboxylate were dissolved in 1,4-dioxane (1mL), and hydrochloric acid/dioxane (4 M, 4 mL) was added. The reaction was stirred at room

temperature for one hour. The solvent was concentrated under reduced pressure. Water and 4M hydrochloric acid aqueous solution were added, which was then lyophilized. Saturated sodium bicarbonate aqueous solution was added. The aqueous phase was extracted with methylene chloride. The organic phase was dried with oil pump to obtain the title products 6-1 (120 mg, 96% yield) and 6-2 (120 mg, 96% yield).

6-1: MS m/z (ESI): 166.1[M+H]$^{+}$.

6-2: MS m/z (ESI): 166.1[M+H]$^{+}$.

**Example 7**

**(*R*) -2-(pyridin-2-yl-4-d)morpholine and (*S*)-2-(pyridin-2-yl-4-d) morpholine**

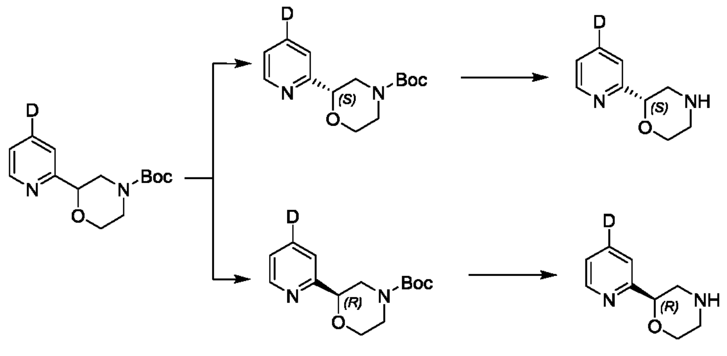

7-P1/7-P2

7-1/7-2

Tert-butyl 2 -(pyridin-2-yl-4-d) morpholin-4-carboxylate was obtained according to Example 6 with 1-(4-chloropyridin-2-yl)ethane-1-one as the starting material. Then the compounds were resolved by chiral column (CHIRALCEL OD-H 4.6*150mm, **5μm; Detection wavelength (nm): 214; Temperature** (°C): 35; Flow rate (mL/min): 1; Mobile phase: Hexane:IPA =70:30; Running time (min):8; Linear mode: isocratic), to obtained the product P1 (RT(retention time)=2.490 min, and product P2 (RT(retention time)=3.373 min).

Products 7-1 and 7-2 were obtained according step 8 Example 6.

Compound 7-1 MS: 166.1[M+1].

Compound 7-2 MS: 166.1[M+1].

**Example 8**

**2-(Pyridin-2-yl -3-d)morpholine**

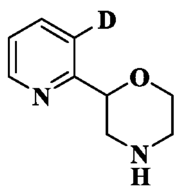

2-(Pyridin-2-yl -3-d)morpholine was obtained according to Example 5 with 1-(3-chloropyridin-2-yl)ethane-1-one as the starting material.

MS: 166.10[M+1].

**Example 9**

**2-(6-Methylpyridin-2-yl)morpholin-2-d**

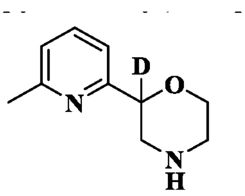

2-(6-Methylpyridin-2-yl) morpholin-2-d was obtained according to the step 2 of Example 4 and Example 1 with 1-(6-methylpyridin-2-yl)ethane-1-one as starting material.

MS: 180.12[M+1].

**Example 10**

**2-(6-Methylpyridin-2-yl)morpholin-5,5-d2**

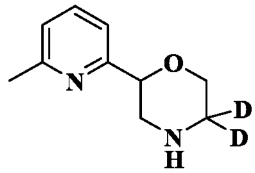

Step 1: 2-Methyl-6-(oxiran-2-yl)pyridine

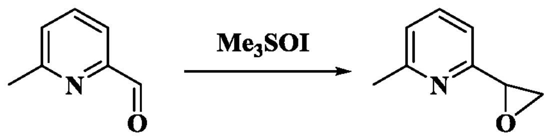


Trimethylsulfoxonium iodide (17.7g, 86.7mmol) was dissolved in dimethyl sulfoxide (100 mL), and potassium tert-butoxide (9.1 g, 80.9mmol) was added. The reaction was stirred at room temperature for 1 hour. A solution of 6-methylpyridin-2-formaldehyde (7g, 57.8mmol) in dimethyl sulfoxide (20 mL) was added and the reaction was stirred at room temperature for 2 hours. Water (300 mL) was added. The aqueous phase was extracted with EA (300 mL × 2). The organic phase was washed with saturated NaCl aqueous solution (300 mL), and dried over anhydrous sodium sulfate, then filtered and concentrated under reduced pressure. The crude product was purified by column chromatography to obtain 2-methyl-6-(oxiran--2-yl) pyridine (6g), yield: 76.8%.

Step 2: 2-(Benzylamino)-1-(6-methyl-2-pyridyl)ethanol

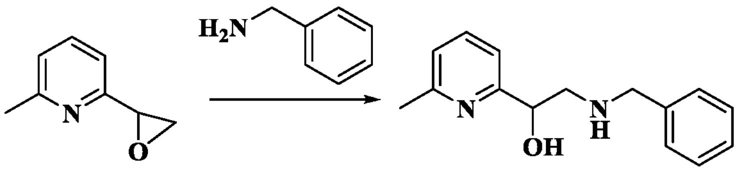

2-Methyl-6-(oxiran-2-yl)pyridine (6g, 44.4mmol) was dissolved in isopropyl alcohol (60 mL), and benzylamine (5.7 g, 53.3mmol) was added. The reaction was stirred at 80 °C for 18 h. The solvent was concentrated under reduced pressure. Water (100 mL) was added. The aqueous phase was extracted with EA (100 mL × 2). The organic phase was washed with saturated NaCl aqueous solution (100 mL), dried over anhydrous sodium sulfate, then filtered and concentrated under reduced pressure. The crude product was purified by column chromatography to obtain 2-(benzylamino)-1-(6-methyl-2-pyridyl)ethanol (3.8 g), yield: 35.3%.

MS m/z (ESI):243.14 [M+1].

Step 3: 4-Benzyl-6 -(6-methyl-2-pyridyl) morpholin-3-one

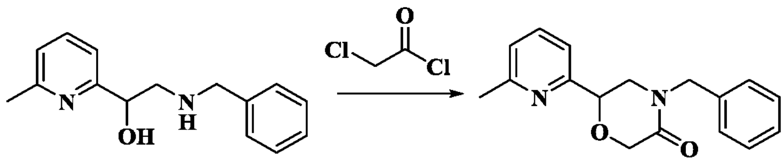

2-(Benzylamino)-1-(6-methyl-2-pyridyl)ethanol (3.8g, 15.7mmol) was dissolved in dichloromethane (50 mL), then triethylamine (4.8g, 47.1mmol) and 3-chloropropionyl chloride (2.4g, 18.8mmol) were added. The reaction was stirred at room temperature for 2 hours. Water (50 mL) was added. The aqueous phase was extracted with dichloromethane (50 mL × 2). The organic phase was dried over anhydrous sodium sulfate, then filtered and concentrated under reduced pressure to obtain the crude intermediate. THF (50 mL) and potassium tert-butoxide (3.4g, 30.1mmol) were added to the crude product, and the reaction was stirred at room temperature overnight. Water (50 mL) was added. The aqueous phase was extracted with ethyl acetate (50 mL × 2). The organic phase was dried over anhydrous sodium sulfate, then filtered and concentrated under reduced pressure. The crude product was purified by column chromatography to obtain 4-benzyl-6-(6-methyl-2-pyridyl) morpholin-3-one (2.2 g). yield: 50.0%.

MS m/z (ESI):283.14 [M+1].

Step 4: 4-Benzyl-2-(6-methylpyridin-2-yl) morpholin-5,5-d2

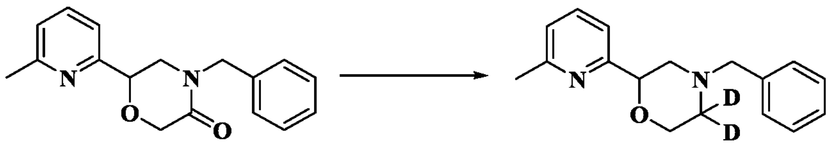

4-Benzyl-6-(6-methyl-2-pyridyl)morpholin-3-one (1.5g, 5.3mmol) was dissolved in tetrahydrofuran (30 mL), and aluminum trichloride (1.1 g, 8.0 mmol) was added, then dissolved by ultrasound. Lithium hydrogen aluminum deuterium (446.3 mg, 10.6mmol) was added and the reaction was stirred at room temperature for 1 hour. Water (0.5mL) and 15% sodium hydroxide (0.5mL) were added, then water (1.5mL) was added. The reaction was stirred for half an hour, then filtered. The filter cake was washed with tetrahydrofuran. The organic phase was dried over anhydrous sodium sulfate, then filtered and concentrated under reduced pressure to obtain 4-benzyl-2 -(6-methylpyridin-2-yl) morpholin-5,5-d2 (1.2g), Yield: 83.5%.

MS m/z (ESI):271.17[M+1].

Step 5: 2-(6-Methylpyridin-2-yl)morpholin-5,5-d2

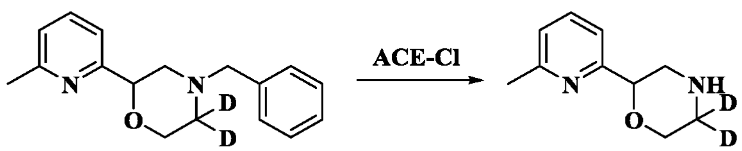


4-Benzyl-2-(6-methylpyridin-2-yl)morpholin-5,5-d2 (1.2g, 4.4mmol) and 1-chloroethyl chlorate (1.9g, 13.3mmol) were dissolved in dichloromethane (10mL), and the reaction was stirred at room temperature overnight. The solvent was concentrated under reduced pressure. The crude product was purified by HPLC to obtain 2-(6-methylpyridin-2-yl)

morpholin-5,5-d2 (0.5g), yield: 62.5%.

$^1$H NMR (400 MHz, DMSO-$d_6$): δ 7.65 (t, 1H), 7.20 (d, 1H), 7.12 (d, 1H), 4.38-4.35 (m, 1H), 3.89-3.87 (m, 1H), 3.60-3.58 (m, 1H), 3.11-3.07 (m, 1H), 2.52-2.46 (m, 1H), 2.43 (s, 3H).

MS: 181.12[M+1].

Step 6: 2-(6-Methylpyridin-2-yl) morpholin-5,5-d2, (*R*)-2-(6-methylpyridin-2-) morpholin-5,5-d2 and (*S*)-2-(6-methylpyridin-2-yl)morpholin-5,5-d2

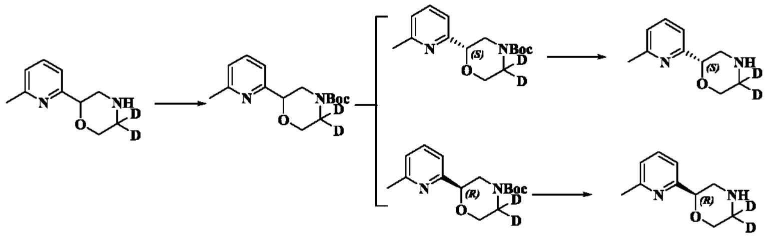

10-P1/10-P2

10-1/10-2

2-(6-Methylpyridin-2-yl)morpholin-5,5-d2 (0.8g, 4.4mmol) was dissolved in dichloromethane (20 mL), then triethylamine (2.3g, 22.3mmol) and di-tert-butyl dicarbonate (1.5g, 6.7mmol) were added. The reaction was stirred at room temperature for 1 hour. Water (30 mL) was added. The aqueous phase was extracted with dichloromethane (30 mL × 2). The organic phase was dried over anhydrous sodium sulfate, then filtered and concentrated under reduced pressure. The crude product was purified by column chromatography to obtain tert-butyl 2-(6-methylpyridin-2-yl)morpholin-4-carboxylate-5,5-d2 (1.1g). The compounds were resolved **by chiral column (CHIRALCEL OD 4.6*150mm, 5μm; Detection** wavelength (nm): 214; Temperature (°C):35; Flow rate (mL/min): 1; Mobile phase: Hexane: IPA =80:20; Running time (min): 8; Linear mode: isocratic). Compound 10-P1 (450 mg, RT (retention time)=2.730 min) and compound 10-P2 (420 mg, RT (retention time)=3.830 min) were obtained.

MS m/z (ESI):281.12 [M+1].

Compound 10-P1 or compound 10-P2 (450mg, 1.6mmol) were dissolved in 1,4-dioxane (1mL), and hydrochloric acid/dioxane (4M, 5mL) was added. The reaction was stirred at room temperature for 1 hour. The solvent was concentrated under reduced pressure. Saturated sodium bicarbonate aqueous solution(1mL) was added. The aqueous phase was extracted with methylene chloride (3mL*3). The organic phase was dried by oil pump. Compound 10-1 was obtained from compound 10-P1, and compound 10-2 (310mg) was obtained from compound 10-P2.

Compound 10-1: $^1$H NMR (400 MHz, DMSO-$d_6$): δ 7.65 (t, 1H), 7.20 (d, 1H), 7.12 (d, 1H), 4.38-4.35 (m, 1H), 3.89-3.87 (m, 1H), 3.60-3.58 (m, 1H), 3.11-3.07 (m, 1H), 2.52-2.46 (m, 1H), 2.43 (s, 3H).

MS: 181.12$[M+H]^+$.

Compound 10-2: 1H NMR (400 MHz, DMSO-d6): δ 7.65 (t, 1H), 7.20 (d, 1H), 7.12 (d, 1H), 4.38-4.35 (m, 1H), 3.89-3.86 (m, 1H), 3.60-3.58 (m, 1H), 3.11-3.07 (m, 1H), 2.52-2.46 (m, 1H), 2.43 (s, 3H).

MS: 181.12$[M+H]^+$.

**Example 11**

**2-(6-Methylpyridin-2-yl)morpholin-5,5,6,6-d4**

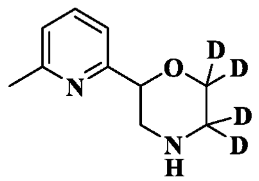

2-(6-Methylpyridin-2-yl) morpholin-5,5,6, 6-d4 was obtained according to the step 2 of Example 4 and Example 1 with 1-(6-methylpyridin-2-yl)ethane-1-one and 2-(benzylamino) ethane-1,1,2,2-d4-1-ol as raw materials.

MS: 183.12[M+1].

**Example12**

**2-(5-Chloropyridin-2-yl)morpholin-2-d**

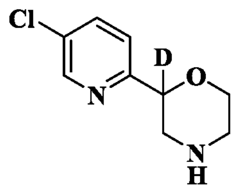

2-(5-Chloropyridin-2-yl)morpholin-2-d was obtained according to the step 2 of Example 4 and Example 1 with 1-(5-chloropyridin-2-yl)ethane-1-one as raw material.

MS: 200.06[M+1].

**Example 13**

**2-(5-Chloropyridin-2-yl)morpholin-5,5,6,6-d4**

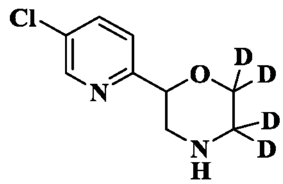

2-(5-Chloropyridin-2-yl) morpholin-5,5,6,6-d4 was obtained according to the step 2 of Example 4 and Example 1 with 1-(5-chloropyridin-2-yl)ethane-1-one and 2-(benzylamino) ethane-1,1,2,2-d4-1-ol as raw materials.

MS: 203.08[M+1].

**Example 14**

**2-(6-(Methyl-d3)pyridin-2-yl)morpholine**

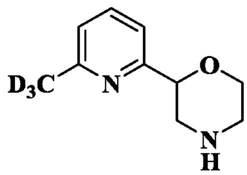

2-(6-(Methyl-d3) pyridin-2-yl)morpholine was obtained according to step 2 of Example 4 and Example 1 with 1-(6-(methyl-d3)pyridin-2-yl)ethane-1-one as raw material.

MS: 182.13[M+1].

**Example 15**

**2-(5-Methylpyridin-2-yl)morpholin-5,5-d2**

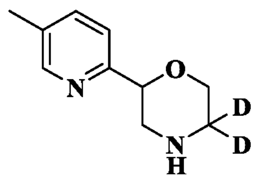

Step 1: 2-(5-Methylpyridin-2-yl)morpholin-4-carboxylate-5,5-d2

2-(5-Methylpyridin-2-yl) morpholin-5,5-d2 was obtained according to Example 10 with 5-methylpyridin-2-formaldehyde as raw material.
MS: 181.12[M+1].

Step 2: (*R*)-2-(5-Methylpyridin-2-yl)morpholin-4-carboxylate-5,5-d2 and (S)-2-(5-methylpyridin-2-yl)morpholin-4-carboxylate-5,5-d2

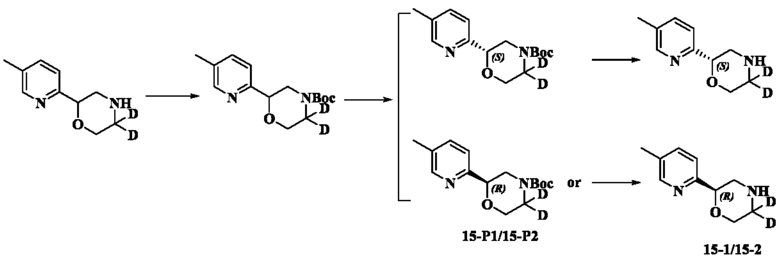

or

15-P1/15-P2

15-1/15-2

Tert-butyl-2 -(5-methylpyridin-2-yl)morpholin-4-carboxylate-5,5-d2 was obtained according to Example 10. The compounds were resolved by chiral column **(CHIRALCEL OD 4.6*150mm, 5μm; Detection wavelength** (nm): 214; Temperature (°C): 35; Flow rate (mL/min): 1; Mobile phase: Hexane:IPA =80:20; Running time (min): 10; Linear mode: isocratic). Compounds 15-P1 (450mg, RT(retention time)=2.730 min) and compounds 15-P2 (420 mg, RT(retention time)= 3.867 min) were obtained.
Boc protection group was removed according to Example 10. compound 15-1 was obtained from compound 15-P1, and compound 15-2 was obtained from compound 15-P2.
Compound 15-1: $^1$H NMR (400 MHz, DMSO-d6) δ 8.32 (s, 1H), 7.58 (dd, 1H), 7.30 (d, 1H), 4.38 (dd, 1H), 3.87 (d, 1H), 3.60 (d, 1H), 3.07 (dd, 1H), 2.53-2.47 (m , 1H), 2.27 (s ,3H).
MS: 181.12[M+1].
Compound 15-2: $^1$H NMR (400 MHz, DMSO-d6) δ 8.32 (s, 1H), 7.58 (dd, 1H), 7.30 (d, 1H), 4.38 (dd, 1H), 3.87 (d, 1H), 3.59 (d, 1H), 3.07 (dd, 1H), 2.52-2.46 (m , 1H), 2.27 (s ,3H).
MS: 181.12[M+1].

**Example 16**

**2-(4-Methylpyridin-2-yl)morpholin-5,5-d2**

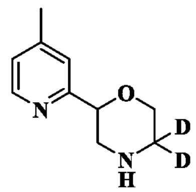

Step 1: Tert-butyl-2-(4-methylpyridin-2-yl)morpholin-4-carboxylate-5,5-d2

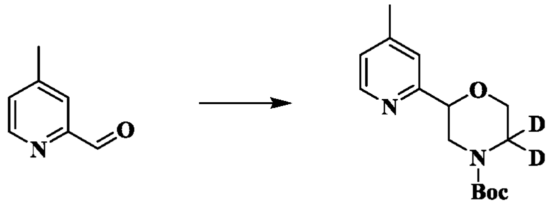

Tert-butyl-2-(4-methylpyridin-2-yl) morpholin-4-carboxylate-5,5-d2 was obtained according to Example 10 with 4-methylpyridin-2-formaldehyde as raw material.

Step 2: (*R*)-2-(4-Methylpyridin-2-yl)morpholin-5,5-d2 and (S) 2-(4-methylpyridin-2-yl) morpholine-5,5-d2

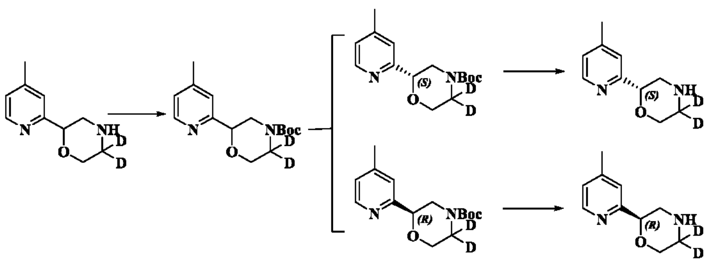

16-P1/16-P2

16-1/16-2

Tert-butyl-2-(4-methylpyridin-2-yl)morpholin-4-carboxylate-5,5-d2 was obtained according to Example 10. The compounds were resolved by chiral column **(CHIRALCEL AD 4.6*150mm, 5μm; Detection wavelength (nm):** 214; Temperature (°C): 35; Flow rate (mL/min): 1; Mobile phase: Hexane: EtOH =80:20; Running time (min): 10; Linear mode: isocratic). Compound 16-P1 (450 mg, RT(retention time)= 4.290 min) and compound 16-P2 (420 mg, RT(retention time)= 4.730 min) were obtained.

Boc protection group was removed according to Example 10. Compound 16-1 was obtained from compound 16-P1, and compound 16-2 was obtained from compound 16-P2.

Compound 16-1: $^1H$ NMR (400 MHz, DMSO-d6) δ 8.32 (d, 1H), 7.25 (s, 1H), 7.09 (d, 1H), 4.38 (dd, 1H), 3.88 (d, 1H), 3.60 (d, 1H), 3.10 (dd, 1H), 2.52-2.46 (m , 1H), 2.31 (s ,3H).

MS: 181.12[M+1].

Compound 16-2: $^1H$ NMR (400 MHz, DMSO-d6) δ 8.32 (d, 1H), 7.25 (s, 1H), 7.09 (d, 1H), 4.38 (dd, 1H), 3.88 (d, 1H), 3.60 (d, 1H), 3.10 (dd, 1H), 2.52-2.46 (m , 1H), 2.31 (s ,3H).

MS: 181.12[M+1].

## Example 17

**2-(5-Fluoropyridin-2-yl)morpholin-5, 5-d2**

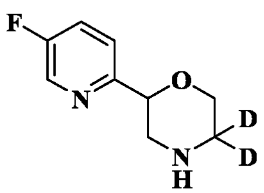

Step 1: Preparation of 5-fluoro-2-(oxiran-2-yl) pyridine

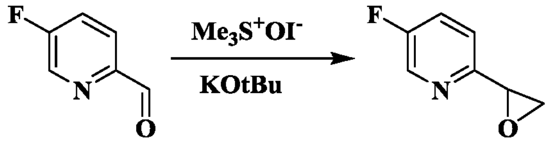


Trimethylsulfoxide (79.16g, 359.71mmol) was dissolved in dimethyl sulfoxide (300 mL), and potassium tert-butanol (37.67g, 335.73mmol) was added under nitrogen atmosphere in an ice water bath. The mixture was stirred at 26°C for 2 hours. 5-Fluoropyridin-2-formaldehyde (30g, 239.81mmol) was added under ice bath and nitrogen atmosphere. The mixture was stirred at 26 °C for 2 hours. The mixture was quenched with saturated NaCl aqueous solution (250mL). The aqueous phase was extracted with ethyl acetate (150mL×3). The organic phase was combined, washed with saturated NaCl aqueous solution (150mL), dried over anhydrous sodium sulfate and concentrated under reduced pressure. The residues were purified by flash silica gel chromatography (petroleum ether: ethyl acetate = 100:0 to 90: 10 elution) to obtain the target product 5-fluoro-2-(oxiran-2-yl) pyridine (15.3 g, yield: 45.86%).

Step 2: Preparation of 2-(benzylamino)-1-(5-fluoro-2-pyridyl)ethanol

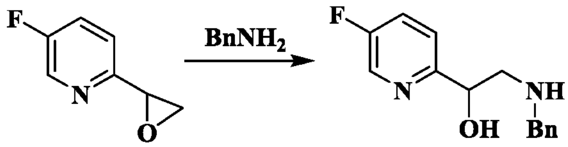


5-Fluoro-2-(oxiran-2-yl)pyridine (15.3g, 109.97mmol) was added to benzylamine (35.35g, 329.91mmol) under nitrogen atmosphere. The mixture was stirred at 85°C for 6 hours and stirred at room temperature for 6 hours. The mixture was concentrated under reduced pressure to remove the benzylamine. The residues were purified by flash silica gel chromatography (petroleum ether: ethyl acetate = 100:0 to 20:80 elution) to obtain the target product 2-(benzylamino-1-(5-fluoro-2-pyridyl)ethanol (22 g, yield: 81.23%).

MS: 247.1[M+1].

Step 3: Preparation of N-benzyl-2-chloro-N-[2-(5-fluoro-2-pyridyl)-2-hydroxyethyl] acetamide

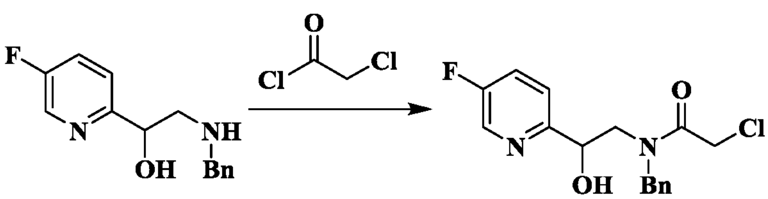

2-(Benzylamino)-1-(5-fluoro-2-pyridyl)ethanol (22g, 89.33mmol) and triethylamine (27.12g, 267.99mmol, 37.38mL) were dissolved in dichloromethane (300 mL) under ice bath and nitrogen atmosphere. Chloroacetyl chloride (15.13g, 133.99mmol, 10.67mL) was added. The mixture was stirred at 0°C for 2 hours. The reaction solution was quenched with saturated NaCl aqueous solution (130mL). The organic phase was separated and washed with saturated NaCl aqueous solution (50mL×2), dried over anhydrous sodium sulfate and concentrated under reduced pressure to obtain the crude product (N-benzyl-2-chloro-N-[2-(5-fluoro-2-pyridyl)-2-hydroxyethyl] acetamide (28 g, yield: 97.11%). The crude product was directly used in the next step.

MS: 323.1[M+1].

Step 4: Preparation of 4-benzyl-6-(5-fluoro-2-pyridyl) morpholin-3-one

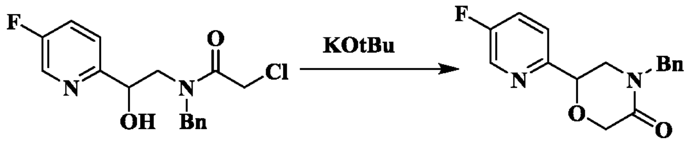


(N-Benzyl-2-chloro-N-[2-(5-fluoro-2-pyridyl)-2-hydroxyethyl]acetamide (28g, 86.75mmol) was dissolved in tetrahydrofuran (1000 mL), and potassium tert-butanol (19.47g, 173.50mmol) was added under nitrogen atmosphere in ice bath. The mixture was stirred at 26°C for 12 hours. Saturated NaCl aqueous solution (250mL) was added to the mixture to quench the reaction. The organic phase was separated, and washed with saturated NaCl aqueous solution (150mL×2), dried over anhydrous sodium sulfate and concentrated under reduced pressure. The residues were purified by flash silica gel chromatography (petroleum ether: ethyl acetate = 100:0 to 70:30 elution) to obtain the target product 4-benzyl-6-(5-fluoro-2-pyridyl)morpholin-3-one (14 g, yield: 56.37%).
MS: 287.1[M+1].

Step 5: Preparation of 4-benzyl-5,5-dideuterium-2-(5-fluoro-2-pyridinyl) morpholine

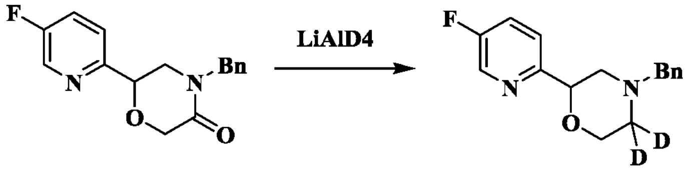


4-Benzyl-6-(5-fluoro-2-pyridyl)morpholin-3-one (6.5g, 22.70mmol) and aluminum trichloride (3.03g, 22.70mmol) were dissolved in tetrahydrofuran (150 mL) in an ice water bath under nitrogen atmosphere, and lithium hydrogen aluminum deuterium was added (953.07mg, 22.70mmol). The mixture was stirred at 26°C for 12 hours. The mixture was quenched with saturated NaCl aqueous solution (20mL) under ice bath and nitrogen protection, and dilute hydrochloric acid (1N, 20mL) was added. The solution was then adjusted PH=8~9 with 15% sodium hydroxide aqueous solution, and filtered. The solid was washed with ethyl acetate (20mL×3). The organic phases was combined, washed with saturated NaCl aqueous solution (100mL×2), dried over anhydrous sodium sulfate and concentrated under reduced pressure to obtain the target product 4-benzyl-5,5-dideuterium-2-(5-fluoro-2-pyridyl)morpholine (5.7 g, yield: 91.52%). The crude product was directly used in the next step.
MS: 275.2[M+1].

Step 6: Preparation of tert-butyl 5,5-dideuterium-2-(5-fluoro-2-pyridyl) morpholin-4-carboxylate

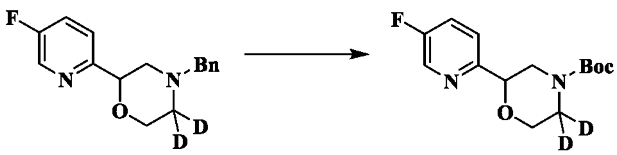

4-Benzyl-5,5-dideuterium-2-(5-fluoro-2-pyridinyl)morpholine (5.7g, 20.78mmol) and di-tert-butyl dicarbonate (9.07g, 41.56mmol) were dissolved in ethanol (200 mL), and 10% palladium carbon (1g, 50%w/w water) under nitrogen atmosphere. The mixture was stirred at 26°C under a hydrogen atmosphere (1atm) for 12 hours. The catalyst was removed by filtration, and the filtrate was concentrated under reduced pressure to obtain a crude product. The residues were purified by flash silica gel chromatography (elution: petroleum ether: ethyl acetate = 100:0 to 80:20) to obtain the target product tert-butyl 5,5-dideuterium-2-(5-fluoro-2-pyridinyl) morpholin-4-carboxylate (3.43 g, yield: 58.06%).

MS: 285.2[M+1].

Step 7: Preparation of (S)-2-(5-fluoropyridin-2-yl)morpholin-5,5-d2 and (R)-2-(5-fluoropyridin-2-yl)morpholin-5,5-d2

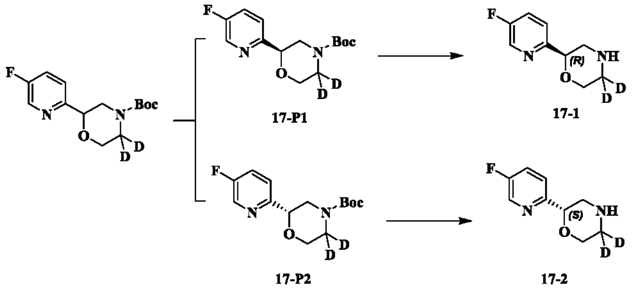

17-P1 17-1 17-P2 17-2

Tert-butyl-2-(5-fluoropyridin-2-yl)morpholin-4-carboxylate-5,5-d2. The compounds were resolved **by chiral column (CHIRALCEL OJ 4.6*150mm,** 5μm; Detection wavelength (nm) : 214; Temperature (°C): 35; Flow rate (mL/min): 1; Mobile phase: Hexane:EtOH = 95:5; Running time (min):10; Linear mode: isocratic). Compound 17-P1 (1.5g, RT(retention time)= 3.340 min), and compound 17-P2 (1.4g, RT(retention time)= 3.773 min) were obtained.
Boc protection group was removed according to Example 10. Compound 17-1 was obtained from compound 17-P1, and compound 17-2 was obtained from compound 17-P2.
Compound 17-1: MS: 185.1[M+1].
Compound 17-2: **1H NMR (400 MHz, DMSO)** δ **8.52** - 8.43 (m, 1H), 7.77 - 7.64 (m, 1H), 7.55 - 7.41 (m, 1H), 4.48 - 4.35 (m, 1H), 3.94 - 3.82 (m, 1H), 3.65 - 3.56 (m, 1H), 3.13 - 3.04 (m, 1H), 2.56 - 2.45 (m, 1H).
MS: 185.1[M+1].

**Example 18**

**5,5-Dimethyl-2-(pyridin-2-yl)morpholine**

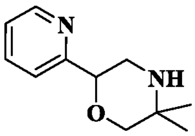

5,5-Dimethyl-2-(pyridin-2-yl) morpholine was obtained according to Example 4 with 2-bromo-1-(pyridin-2-yl) ethane-1-one as raw material.
MS: 193.1[M+1].

**Example 19**

Step 1: Preparation of 3-fluoro-2-methyl-6-vinylpyridine

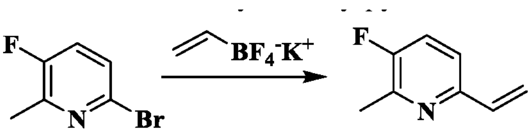

6-Bromo-3-fluoro-2-**methylpyridine (0.1g, 526.28μmol),** potassium **vinyltrifluoroborate (84.59mg, 631.54μmol) and cesium carbonate (514.42 mg,** 1.58mmol) were dissolved in 1,4-dioxane (5mL) and water (2 mL),

and 1,1'-bis(diphenylphosphino)ferrocene **dichloropalladium (19.25 mg, 26.31μmol) was** added under nitrogen atmosphaere. The mixture was stirred at 90°C for 6 hours under nitrogen atmosphaere. Saturated NaCl aqueous solution (50mL) was added to the mixture to quench the reaction, which was then extracted with ethyl acetate (30mL×2). The organic phase was combined, washed with saturated NaCl aqueous solution (50mL×2), dried over anhydrous sodium sulfate and concentrated under reduced pressure. The residues were purified by flash silica gel chromatography (elution: petroleum ether: ethyl acetate = 100:0 to 90:10) to obtain the target product 3-fluoro-2-methyl-6-vinylpyridine (50 mg, yield: 69.27%).

MS m/z (ESI):138.1 [M+1].

Step 2: Preparation of 3-fluoro-2-methyl-6-(oxiran-2-yl) pyridine

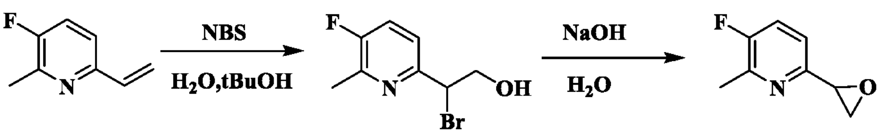


3-Fluoro-2-methyl-6-vinylpyridine (0.6g, 4.37mmol) was dissolved in water (10mL) and tert-butanol (5 mL), and N-bromosuccinimide (856.48mg, 4.81mmol) was added. The mixture was stirred at 25°C for 1 hour. After cooled to 0°C, a solution of sodium hydroxide (524.92mg, 13.12mmol) in water (2ml) was added, and the mixture was stirred at 0°C for 1 hour. LCMS showed that the reaction was completed. The reaction solution was extracted with ethyl acetate (30mL×2). The organic phase was separated, and washed with saturated NaCl aqueous solution (30mL×2), dried over anhydrous sodium sulfate and concentrated under reduced pressure to obtain crude 3-fluoro-2-methyl-6-(oxiran-2-yl)pyridine (0.6 g). The crude product is directly used in the next step.

MS m/z (ESI): 154.1[M+1].

Step 3: Preparation of 2-[benzyl(2-hydroxyethyl)amino]-1-(5-fluoro-6-methyl-2-pyridyl)ethanol

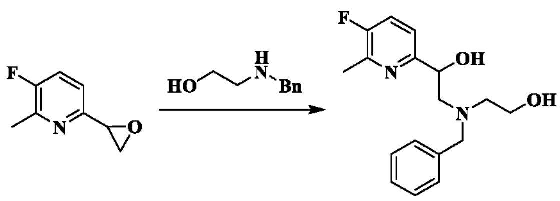

2-(Benzylamino)ethanol (651.60mg, 4.31mmol) was dissolved in isopropyl alcohol (15mL) and a solution of 3-fluoro-2-methyl-6- (oxiran-2-yl)pyridine (0.6g, 3.92mmol) in isopropanol (3mL) was added under nitrogen protection at 90°C. The mixture was stirred at 90°C for 12 hours. The mixture was concentrated under reduced pressure. The residues were purified by flash silica gel chromatography (elution:petroleum ether: ethyl acetate = 100:0 to 20:80) to obtain the target product 2-[benzyl (2-hydroxyethyl)amino]-1-(5-fluoro-6-methyl-2-pyridyl)ethanol (0.77 g, yield: 64.58%).

MS m/z (ESI): 305.2[M+1].

Step 4: Preparation of 4-benzyl-2-(5-fluoro-6-methyl-2-pyridyl)morpholine

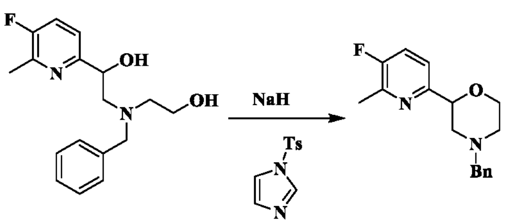

2-[Benzyl(2-hydroxyethyl)amino]-1-(5-fluoro-6-methyl-2-pyridyl)ethanol (0.77g, 2.53mmol) was dissolved in tetrahydrofuran (15 mL), and then sodium hydrogen (202.37mg, 5.06mmol, 60% w/w) was added in an ice water bath under nitrogen atmosphere. The mixture was stirred at 0°C for 1 hour. Then 1-(p-toluenesulfonyl) imidazole (562.31mg, 2.53mmol) was added. The mixture was stirred at 23°C for 12 hours. The mixture was quenched with saturated NaCl aqueous solution (50mL) at 0°C. The reaction solution was extracted with ethyl acetate (30mL×2). The organic phase was combined, washed with saturated NaCl aqueous solution (50mL×2), dried over anhydrous sodium sulfate and concentrated under reduced pressure. The residues were purified by flash silica gel chromatography (elution: petroleum ether: ethyl acetate = 100:0 to 70:30) to obtain the target product 4-benzyl-2-(5-fluoro-6-methyl-2-pyridinyl)morpholine (0.36 g, yield: 49.69%).

MS m/z (ESI): 287.2[M+1].

Step 5: Preparation of 2-(5-fluoro-6-methyl-2-pyridinyl)morpholine

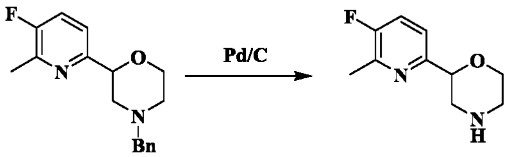


4-Benzyl-2-(5-fluoro-6-methyl-2-**pyridinyl)morpholine (180mg, 628.62μmol)** was dissolved in EtOH (15mL), **and Pd/C (66.90mg, 628.62μmol) was added under** nitrogen atmosphere. The mixture was stirred at 25°C under hydrogen atmosphere (1atm) for 12 hours. The reaction was filtered to remove the catalyst and then concentrated under reduced pressure to obtain the target product 2-(5-fluoro-6-methyl-2-pyridyl)morpholine (70mg, yield: 56.75%).

MS m/z (ESI): 197.1[M+1].

Step 6: Preparation of tert-butyl 2- (5-fluoro-6-methyl-2-pyridyl) morpholin-4-carboxylate

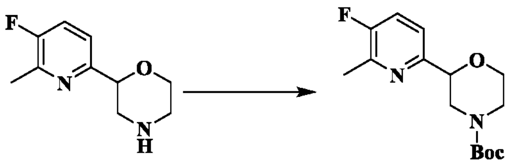

2-(5-Fluoro-6-methyl-2-**pyridinyl)morpholine (150mg, 764.44μmol)** and **triethylamine (232.06mg, 2.29mmol, 319.64μL) were dissolved** in dichloromethane (10 mL), and di-tert-butyl dicarbonate (250.26mg, 1.15mmol) was added under nitrogen atmosphere. The mixture was stirred at 25°C for 1 hour. The mixture was concentrated under reduced pressure. The residues were purified by flash silica gel chromatography (elution: petroleum ether: ethyl acetate = 100:0 to 80:20) to obtain the target product tert-butyl 2-(5-fluoro-6-methyl-2-pyridinyl)morpholin-4-carboxylate (0.2g, yield: 88.29%).

MS m/z (ESI): 297.2[M+1].

Step 7: Preparation of (S)-2-(5-fluoro-6-methyl-2-pyridyl)morpholine and (R)-2-(5-fluoro-6-methyl-2-pyridyl)morpholine

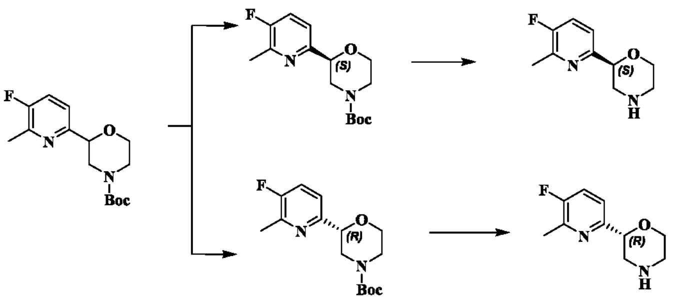

19-P1/19-P2 19-1/19-2

Tert-butyl 2-(5-fluoro-6-methyl-2-pyridyl) morpholin-4-carboxylate was resolved by chiral column (chromatographic column: CHIRALCEL OJ 4.6 * 150mm, **5) μM; Detection wavelength (nm):** 214; Temperature (°C): 35; Flow rate (mL/min): 1; Mobile phase: Hexane: EtOH=70:30; Running time (min): 10; Linear mode: isocratic). Compound 19-P1 (80mg, RT (retention time)=2.213 min) and compound 19-P2 (98mg, RT (retention time)=2.523 min) were obtained.

Boc protection group was removed according to Example 10. Compound 19-1 was obtained from compound 19-P1, and compound 19-2 was obtained from compound 19-P2.

Compound 19-1: MS: 197.1[M+1].

Compound 19-2: MS: 197.1[M+1].

**Example 20**

**2-(6-(Trifluoromethyl)pyridin-2-yl) morpholine**

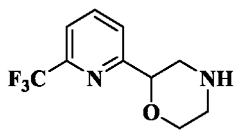

Step 1: 2-Trifluoromethyl-6-( oxiran-2-) pyridine

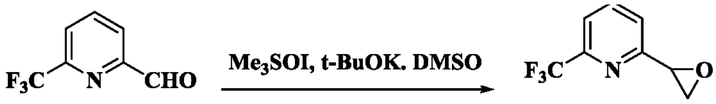


2-Trifluoromethyl-6-( oxiran-2-)pyridine was obtained according to step 1 of Exmaple 10 with 6-trifluoromethylpyridin-2-formaldehyde as raw material.

MS m/z (ESI):243.14 [M+1].

Step 2:2-(Benzyl(2-hydroxyethyl)amino)-1-(6-(trifluoromethyl)pyridine-2-yl)ethane-1-ol

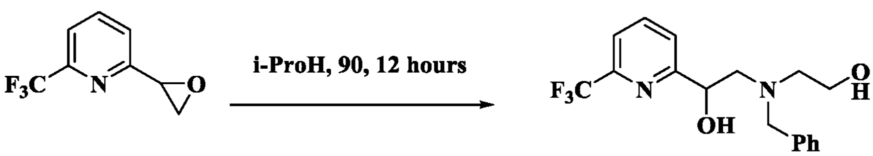


2-(Benzylamino)ethanol (1.10g, 7.30mmol) was dissolved in isopropyl alcohol (40 mL) at room temperature and heated to 90°C. Then a solution of 2-trifluoromethyl-6-(oxiran--2-yl) pyridine (1.15g, 6.08mmol) in isopropyl alcohol

(40 mL) was added, and the reaction was reacted at 90°C for 14 hours. The reaction was cooled to room temperature. LCMS indicated the end of the reaction, and the reaction solution was diluted with ethyl acetate (60 mL), then washed with saturated NaCl aqueous solution. The organic phase was dried over anhydrous sodium sulfate, then filtered and concentrated under reduced pressure. The crude product was purified by flash silica gel chromatography (elution with petroleum ether: ethyl acetate = 50:50 to 30:70) to obtain the target product, which was light yellow oil (1.90g), yield: 91.8%.

MS: 341.14[M+1].

Step 3: 4-Benzyl-2-(6-(trifluoromethyl)pyridin-2-yl)morpholine

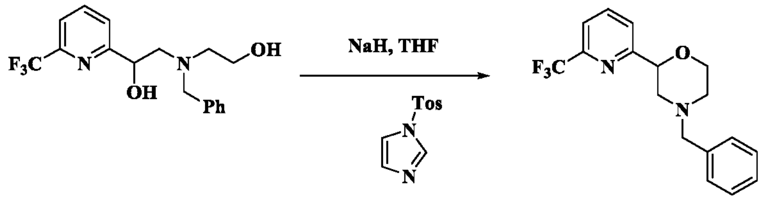


2-(Benzyl(2-hydroxyethyl)amino)-1-(6-(trifluoromethyl)pyridin-2-yl)ethane-1-ol (0.77g, 2.26mmol) was dissolved in tetrahydrofuran (40 mL) in an ice bath and then replaced with nitrogen. Sodium hydrogen (181.00mg, 4.52mmol, 60% purity) was added. The reaction was stirred at room temperature for 1 hour, then a solution of 1-(4-toluenesulfonyl) imidazole (553.14mg, 2.49mmol) in tetrahydrofuran (2mL) was added dropwise. The reaction was stirred at room temperature for 2 hours. TLC indicated the end of the reaction, then the reaction liquid was quenched with saturated ammonium chloride solution, and then extracted with ethyl acetate. The organic phase was combined, dried over anhydrous sodium sulfate, then filtered and concentrated under reduced pressure. The crude product was purified by flash silica gel column (elution with PE: EA = 90:10 to 70: 30) to obtain the target product as yellow oil (0.32g), yield: 43.8%.

MS: 323.14[M+1].

Step 4: 2-(6-(Trifluoromethyl)pyridin-2-yl)morpholine

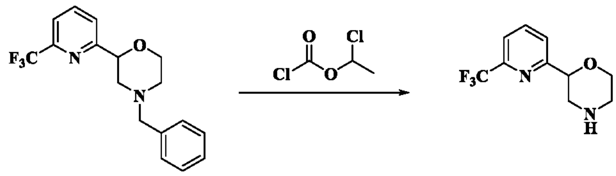

4-Benzyl-2-(6-(trifluoromethyl) pyridin-2-**yl)morpholine (0.30g, 930.74μmol)** was dissolved in dichloromethane (10 mL) at room temperature, then 1-chloroethyl chloroformate (532.27mg, 3.72mmol) was added and the reaction was stirred at room temperature for 2h. The reaction solution was concentrated under reduced pressure. The residues were dissolved in methanol (10 mL), and heated to 70°C for 1 hour. The reaction was then cooled to room temperature. LCMS indicated the end of the reaction. The reaction solution was concentrated under reduced pressure to obtain the residues which were directly used in the next step (0.28 g), yield: 99.0%.

MS: 233.08[M+1].

Step 5: Tert-butyl 2-(6-(trifluoromethyl)pyridin-2-yl)morpholin-4-carboxylate

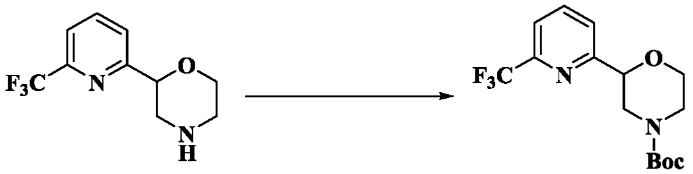

2-(6-(Trifluoromethyl)pyridin-2-yl) morpholine (0.33g, 1.23mmol, hydrochloride) was dissolved in DCM (10mL) at room temperature, then N,N-diethylethylamine **(621.46 mg, 6.14 mmol, 856.60 μL) and tert**-butoxycarbonyl tert-butyl carbonate (402.53 mg, 1.84 mmol) were added. The reaction was stirred at room temperature overnight. TLC indicated the end of the reaction, and the reaction solution was washed with saturated sodium bicarbonate solution and saturated NaCl aqueous solution. The organic phase was dried over anhydrous sodium sulfate, then filtered and concentrated under reduced pressure. The residues were purified by flash silica gel chromatography (elution with petroleum ether: ethyl acetate = 90:10 to 70: 30) to obtain the target product, which was light yellow oil (0.30 g), yield: 73.5%.
MS: 277.08[M-55].

Step 6: 2-(6-(Trifluoromethyl)pyridin-2-yl) morpholine

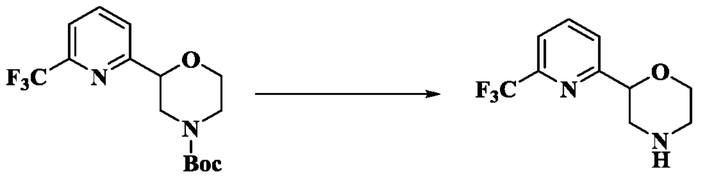

Boc protective group was removed according to Exmaple 10 to obtain the target product (0.04g).

**Example 21**

**2-(3-Fluoro-5-methylpyridin-2-yl) morpholine**

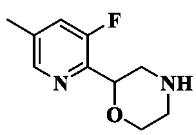

2-(3-Fluoro-5-methylpyridin-2-yl)morpholine was obtained according to the steps 1-5 of Example 19.
MS: 197.10 [M+1].

**Example 22**

**2-(3-Fluoro-6-methylpyridin-2-yl)morpholine**

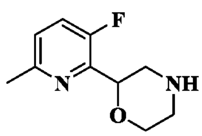

Step 1: 3-Fluoro-6-methyl-2-vinylpyridine

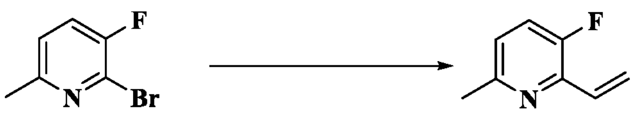

2-Bromo-3-fluoro-6-methyl-pyridine (5.0g, 26.31mmol), cesium carbonate (17.15 g, 52.63 mmol), [1, 1-bis (diphenylphosphorus) ferrocene] palladium dichloride (1.93 g, 2.63 mmol) and potassium trifluoride (vinyl) borane (4.23 g, 31.58 mmol) were dissolved in water (12 mL) and 1, 4-dioxane (60 mL) at room temperature, and then replaced with nitrogen. The reaction was heated to 90°C for 14 hours, then cooled to room temperature. LCMS indicated the end of the reaction, and the reaction solution was diluted with ethyl acetate, then washed with saturated NaCl aqueous solution. The organic phase was dried over anhydrous sodium sulfate, then filtered and concentrated under reduced pressure. The residues were purified by flash silica gel chromatography (elution: petroleum ether: ethyl acetate = 90:10 to 70:30) to obtain the target product as a colorless oil (1.70g), yield: 47.1%.
MS: 138.06[M+1].

Step 2:3-Fluoro-6-methyl-2-(oxiran-2-yl)pyridine

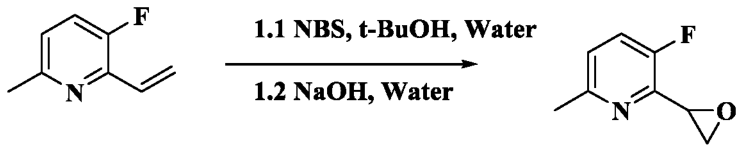


3-Fluoro-6-methyl-2-vinylpyridine (1.70g, 12.39mmol) was dissolved in water (40 mL) and tert-butanol (20mL) at room temperature, then N-bromosuccinimide (2.32g, 13.01mmol) was added. The reaction was heated to 40°C for 1 hour, then cooled in ice bath. A aqueous solution of sodium hydroxide (1.49g, 37.18mmol) was added dropwise, and the reaction solution was stirred for another hour. TLC indicated the end of the reaction. The reaction solution was diluted with water, then extracted with ethyl acetate. The organic phase was combined, dried over anhydrous sodium sulfate, then filtered and concentrated under reduced pressure. The residues were purified by flash silica gel chromatography (elution: petroleum ether: ethyl acetate = 90:10 to 70:30) to obtain the target product as a colorless oil (1.20g), yield: 63.2%.
MS: 154.06[M+1].

Step 3:2-(3-Fluoro-6-methylpyridin-2-yl) morpholine

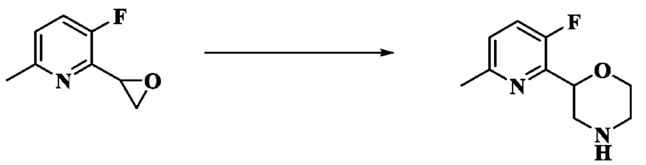

2-(3-Fluoro-6-methylpyridin-2-yl)morpholine was obtained according to step 2, step 3, step 4, step 5, step 6 of Example20 with 3-fluoro-6-methyl-2-(oxiran-2-yl) pyridine as raw material.
MS: 197.10[M+1].

## Example 23

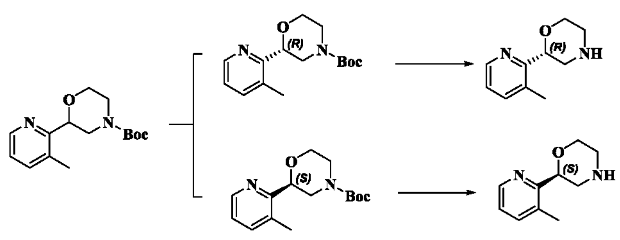

23-P1/23-P2 23-1/23-2

Tert-butyl 2 -(3-methylpyridin-2-yl)morpholin-4-carboxylate was obtained according to Example 20 with 3-methylpicoline aldehyde as raw material. Tert-butyl 2-(3-methylpyridin-2-yl)morpholin-4-carboxylate was subjected to chiral resolution **under the following conditions: (column: CHIRALPAK IC 4.6*250mm,** 5μm; Detection wavelength (nm):214; Temperature (°C): 35; Flow rate (mL/min): 1; Mobile phase: Hexane: EtOH = 70:30; Running time (min): 10; Linear mode: isocratic). 23-P1 (RT(retention time)= 2.560 min); 23-P2 (RT(retention time)= 3.680 min);

Boc protection group was removed according to Example 10. Compound 23-1 was obtained from compound 23-P1, and compound 23-2 was obtained from compound 23-P2.

Compound 23-1 MS: 179.1[M+1].

Compound 23-2 MS: 179.1[M+1].

**Example 24**

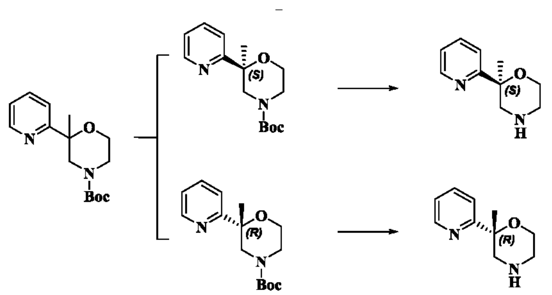

24-P1/24-P2 24-1/24-2

Tert-butyl-2-(5-fluoropyridin-2-yl)morpholin-4-carboxylate-5,5-d2 was obtained according to Example 20 with 1-(pyridin-2-yl)ethane-1-one as raw material. The compounds were resolved **by chiral column (CHIRALPAK IC 4.6*250mm,** 5μm; Detection wavelength (nm):214; Temperature (°C): 35; Flow rate (mL/min): 1; Mobile phase: Hexane: IPA = 70:30; Running time (min):10; Linear mode: isocratic). 24-P1 (RT(retention time)= 4.907 min); 24-P2 (RT(retention time)= 6.520 min);

Boc protection group was removed according to Example 10. Compound 24-1 was obtained from compound 24-P1, and compound 24-2 was obtained from compound 24-P2.

Compound 24-1 MS: 179.1[M+1].

Compound 24-2 MS: 179.1[M+1].

**Example 25**

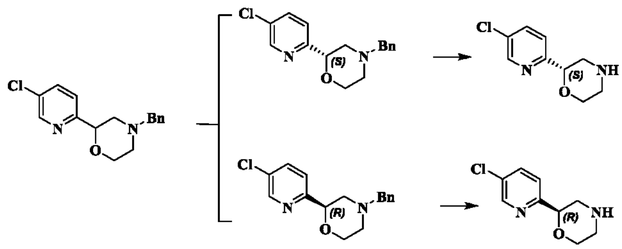

25-P1/25-P2 25-1/25-2

4-Benzyl-2-(5-chloropyridin-2-yl)morpholine was obtained according to Example 4 with 1-(5-chloropyridin-2-yl)ethane-1-one as raw material. The compound 4-benzyl-2-(5-chloropyridin-2-yl) morpholine was resolved by chiral column **(CHIRALCEL OJ 4.6*150mm, 5μm; Detection wavelength (nm):** 214; Temperature (°C): 35; Flow rate (mL/min): 1; Mobile phase: Hexane: EtOH = 70:30; Running time (min): 10; Linear mode: isocratic). Compound 25-P1 (RT(retention time)= 2.543 min) was obtained, and Compound 25-P2 (RT(retention time)= 2.843 min) was obtained.

According to step 6 of Example 4, compound 25-P1 was subjected to debenzylation to obtain compound 25-P1, and compound 25-P2 was subjected to debenzylation to obtain compound 25-2.

Compound 25-1 MS: 199.1[M+1].
Compound 25-2 MS: 199.1[M+1].

**Example 26**

**2-(Pyridin-2-yl)-1,4-oxazepane**

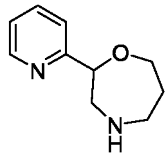

2-(Pyridin-2-yl)-1,4-oxazepane was obtained according to Example 4 with 2-bromo-1-(pyridin-2-yl)ethane-1-one as raw material.
MS: 179.1[M+1].

**Example 27**

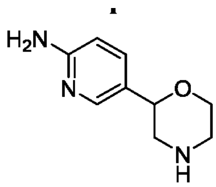

Step 1: Preparation of 4-benzyl-2-(6-chloropyridin-3-yl) morpholine

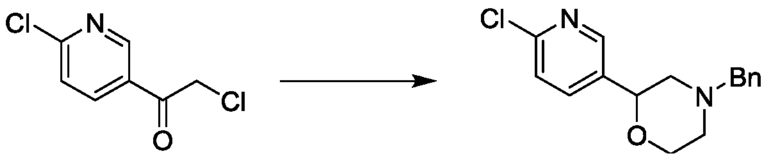

4-Benzyl-2-(6-chloropyridin-3-yl) morpholine was obtained according to steps 3-5 of Example 4 with 2-chloro-1-(6-chloropyridin-3-yl)ethane-1-one as raw material.
MS: 289.1[M+1].

Step 2: Preparation of N-(5-(4-Benzylmorpholin-2-yl) pyridin-2-yl)-1,1-diphenylimine

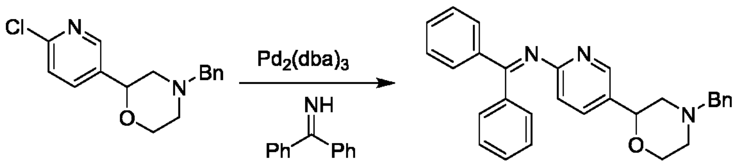


4-Benzyl-2-(6-chloropyridin-3-yl)morpholine (0.3g, 1.04mmol), dibenzylimine (225.94mg, 1.25mmol), 1,1'-binaphthyl-2,2'-diphenyl phosphine (BINAP) (103mg, 166μmol) and cesium carbonate (676.98 mg, 2.08 mmol) were

dissolved in 1,4-dioxane (25 mL) under nitrogen atmosphere, and tris(dibenzylideneacetone)dipalladium (76.11mg, 83.11μmol) was added. The mixture was stirred at 110 °C for 5 hours. The mixture was quenched with saturated NaCl aqueous solution (50mL). The aqueous phase was extracted with ethyl acetate (30mL×2). The organic phase was combined, washed with saturated NaCl aqueous solution (50mL×2), dried over anhydrous sodium sulfate, and concentrated under reduced pressure. The residues were purified by flash silica gel chromatography (elution: petroleum ether: ethyl acetate = 100:0 to 60:40) to obtain the target product N-(5-(4-benzylmorpholin-2-yl) pyridine-2-yl)-1,1-diphenylimine (0.38g, 876.50μmol, yield: 84.37%).

MS: 434.2[M+1].

Step 3: Preparation of 5-(4-benzylmorpholin-2-yl) pyridine-2-amine

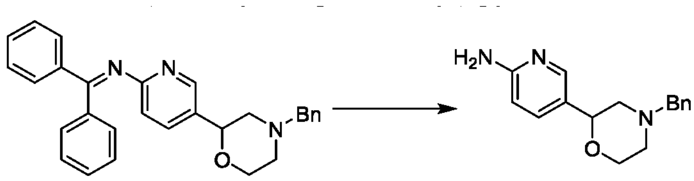

N-(5-(4-Benzylmorpholin-2-yl)pyridin-2-yl)-1, 1-dibenzyleneimine (0.3g, 691.97μmol) was dissolved in tetrahydrofuran (1mL) and methanol (5mL), and a aqueous solution of hydrochloric acid (2 M, 6 mL) was added under nitrogen atmosphere. The mixture was stirred at 23 °C for 12 hours. The mixture was quenched with saturated sodium bicarbonate aqueous solution (50mL). The aqueous phase was extracted with ethyl acetate (50mL×3). The organic phase was combined, washed with saturated NaCl aqueous solution (50mL×2), dried over anhydrous sodium sulfate and concentrated under reduced pressure. The residues were purified by flash silica gel chromatography (elution: dichloromethane: methanol = 100:0 to 95:5) to obtain the target product 5-(4-benzylmorpholin-2-yl)pyridin-2-amine (0.1g, 371.28μmol, yield: 53.65%).

MS: 270.2[M+1].

Step 4: Preparation of 5-morpholin-2-pyridin-2-amine

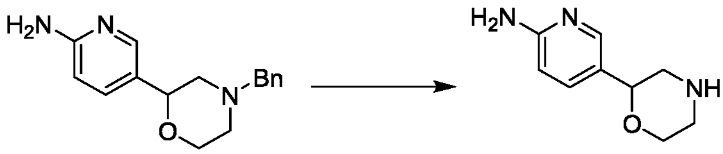

5-(4-Methylmorpholin-2-yl) pyridin-2-amine (24 mg, 89.11μmol) was dissolved in EtOH (10 mL), and 10% palladium carbon (6.00 mg, 50%w/w water) was added under nitrogen atmosphere. The mixture was stirred at 23°C under a hydrogen atmosphere (1atm) for 1 hour. The catalyst was removed by filtration. The filtration was concentrated to obtain the target product 5-morpholin-2-pyridin-2-amine (15.9 mg, 81.62μmol, yield: 91.60%).

$^1$H NMR (400 MHz, CDC13) δ 8.03 (d, J = 2.3 Hz, 1H), 7.46 (dd, J = 8.4, 2.3 Hz, 1H), 6.48 (d, J = 8.4 Hz, 1H), 4.53 - 4.40 (br, 2H), 4.40 - 4.31 (m, 1H), 4.05 - 3.91 (m, 1H), 3.83 - 3.67 (m, 1H), 3.05 - 2.92 (m, 2H), 2.92 - 2.73 (m, 2H).

MS: 180.1[M+1].

**Example 28**

**2-(Phenyl-4-d) morpholine**

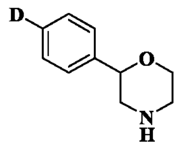

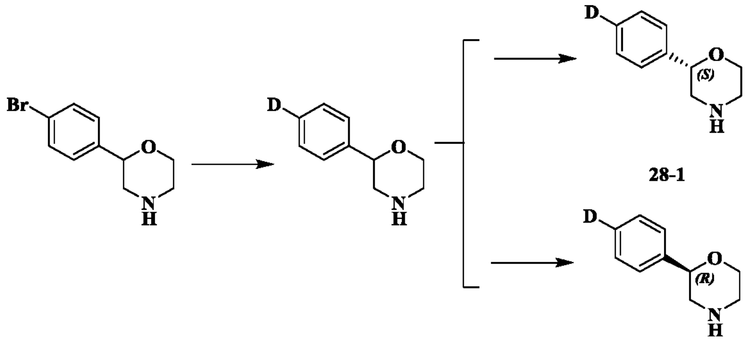

28-1

28-2

2-(4-Bromophenyl)morpholine (220mg, 908.67μmol) and cesium carbonate (740.16mg, 2.27mmol) were dissolved in deuterated methanol (5 mL) under nitrogen atmosphere, and 1,1'-Bis(diphenylphosphino)ferrocene]dichloropalladium (66.49mg, 90.87μmol) was added. The mixture was stirred at 80°C for 16 hours. The reaction solution was concentrated under reduced pressure. The crude product was separated by high pressure liquid preparative chromatography to obtain 2-(phenyl-4-d)morpholine (75mg, 50% yield).

$^1$H NMR (400 MHz, DMSO) δ 7.38 - 7.25 (m, 4H), 4.42 - 4.30 (m, 1H), 3.93 - 3.79 (m, 1H), 3.64 - 3.53 (m, 1H), 2.95 - 2.83 (m, 1H), 2.79 - 2.70 (m, 2H), 2.51 - 2.50 (m, 1H).

MS: 165.1[M+1].

The compound was resolved by chiral column (CHIRALPAK AD-H 4.6*250mm, 5μm; Detection wavelength (nm) : 214; Temperature (°C) : 35; Flow rate (mL/min): 1; Mobile phase: Hexane: EtOH:MeOH:DEA =70:15:15:0.1%; Running time (min):12; Linear mode: isocratic) to obtain compound 1-1 (30 mg, RT(retention time)= 5.630min), and compound 1-2 (30 mg, RT(retention time)= 6.743 min).

Compound 28-1: $^1$H NMR (400 MHz, DMSO) δ 7.38 - 7.25 (m, 4H), 4.42 - 4.30 (m, 1H), 3.93 - 3.79 (m, 1H), 3.64 - 3.53 (m, 1H), 2.95 - 2.83 (m, 1H), 2.79 - 2.70 (m, 2H), 2.51 - 2.50 (m, 1H).

MS: 165.1 [M+1].

Compound 28-2: $^1$H NMR (400 MHz, DMSO) δ 7.38 - 7.25 (m, 4H), 4.42 - 4.30 (m, 1H), 3.93 - 3.79 (m, 1H), 3.64 - 3.53 (m, 1H), 2.95 - 2.83 (m, 1H), 2.79 - 2.70 (m, 2H), 2.51 - 2.50 (m, 1H).

MS: 165.1[M+1].

**Example 29**

**2-Phenylmorpholin-5,5-d2**

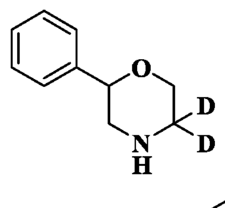

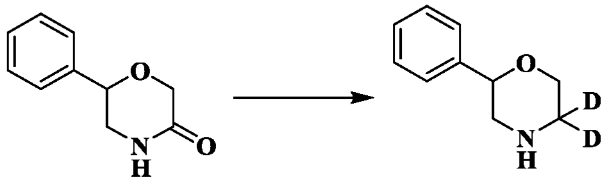

6-Phenyl-morpholin-3-one (500 mg, 2.82mmol) was dissolved in THF (10mL) under ice bath, then a solution of deuteroborane in tetrahydrofuran (0.75M, 15mL, 11.29mmol) was added, and then replaced with nitrogen. The reaction

was heated to 70°C for 2 hours, and cooled to room temperature. LCMS indicated the end of the reaction. The reaction solution was quenched with methanol, and then heated to 70°C for 2 hours. The reaction was cooled to room temperature, and concentrated under reduced pressure. The residues were dissolved with ethyl acetate, then washed with saturated ammonium chloride solution and saturated NaCl aqueous solution successively. The organic phase was dried over anhydrous sodium sulfate, then filtered and concentrated under reduced pressure. The residues were purified by flash silica gel chromatography (petroleum ether: ethyl acetate = 85:15 to 50) to obtain the target product 2-phenylmorpholin-5,5-d2 (396mg, yield: 84.9%).

MS: 166.1[M+1].

**Example 30**

**2-(Phenyl-4-d) morpholin-5, 5-d2**

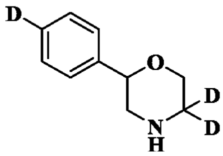

2-(Phenyl-4-d)morpholin-5,5-d2 was obtained according to Example 29 and Example 28 with 6-(4-chlorophenyl)-3-morpholinone as raw material.

MS: 167.1[M+1].

**Example 31**

**2- (Phenyl-d5)morpholin-5,5-d2**

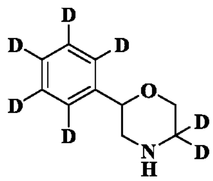

2-(Phenyl-d5)morpholin-5,5-d2 was obtained according to Example 29 with 6-(phenyl-d5)morpholin-3-one (prepared by the known method "Bioorg.Med.Chem.Lett.26(2016)1329-1332") as raw material.

**Example 32**

**2-(p-Benzyl)morpholin-5,5-d2**

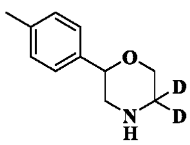

2-(p-Benzyl)morpholin-5,5-d2 was obtained according to Example 29 with 6-(p-benzyl)morpholin-3-one as raw material.

MS: 180.1[M+1].

**Example 33**

**2- (4-Fluorophenyl)morpholin-5,5-d2**

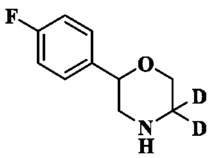

**Method 1:**

2-(4-Fluorophenyl)morpholin-5,5-d2 was obtained according to Example 29 with 6-(4-fluorophenyl)morpholin-3-one (prepared by the known method "Bioorg.Med.Chem.Lett.26(2016)1329-1332") as raw material.
MS: 184.1[M+1].

**Method 2:**

Step 1: 2- (4-Fluorophenyl)oxirane

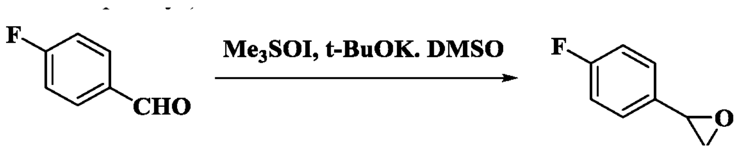


Trimethylsulfoxide (106.39g, 483.43mmol) was dissolved in dimethyl sulfoxide (200 mL) in an ice bath, then potassium tert-butanol (49.18g, 438.31mmol) was added and replaced with nitrogen. The reaction was stirred at room temperature for one hour. Thena solution of 4-fluorobenzaldehyde (40 g, 322.29 mmol) in dimethyl sulfoxide (200 mL) was added and stirred at room temperature for one hour. TLC indicated the end of the reaction. The reaction solution was quenched with saturated NaCl aqueous solution, and extracted with ethyl acetate. The organic phase was combined, dried over anhydrous sodium sulfate, then filtered and concentrated under reduced pressure. The crude product was purified by a flash silica gel column (elution: PE: EA = 95:5 to 90: 10) to obtain the target product, which was a colorless oil (19.5 g, yield: 43.8%).

Step 2: 2- (Benzylamino)-1-(4-fluorophenyl)ethane-1-ol

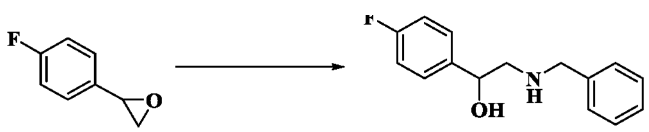

2-(4-Fluorophenyl)oxirane (14.5g, 104.9 mmol) was stirred at room temperature, then benzylamine (33.74g, 314.90mmol) was added, and replaced with nitrogen. The reaction was heated to 80°C for 6 hours, then cooled to room temperature, and stirred at room temperature for 7 hours. There has white solid precipitation. Then 120ml of water was added, and the reaction was stirred at room temperature for 1 hour. The reaction was filtered. The solid was washed twice with water and twice with petroleum ether successively, and then dried. The solid was directly used in the next step (15.8g, yield: 61.4%).
MS: 246. 1 [M+1].

Step 3: N-Benzyl-2-chloro-N-(2-(4-fluorophenyl)-2-hydroxyethyl) acetamide

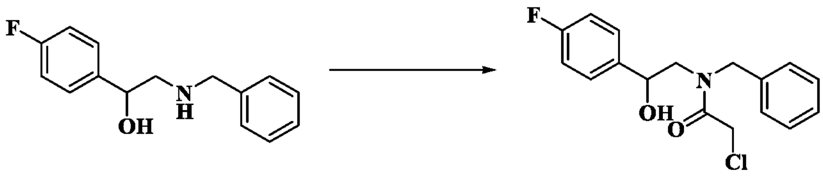

2-(Benzylamino)-1-(4-fluorophenyl)ethane-1-ol (12.5g, 50.96mmol) was dissolved in dichloromethane (100 mL) in an ice bath, then N,N- diethylacetamide (15.47g, 152.88mmol, 21.32mL) and chloroacetyl chloride (8.06g, 71.34mmol) was added successively. The reaction was stirred at room temperature for 2 hours. LCMS indicated the end of the reaction. The reaction solution was diluted with dichloromethane and then washed with saturated NaCl aqueous solution. The organic phase was dried over anhydrous sodium sulfate, then filtered and concentrated under reduced pressure to obtain a residue which was directly used in the next step (16.4 g, yield: 100%).

MS: 322.1[M+1].

Step 4: 4-Benzyl-6- (4-fluorophenyl) morpholin-3-one

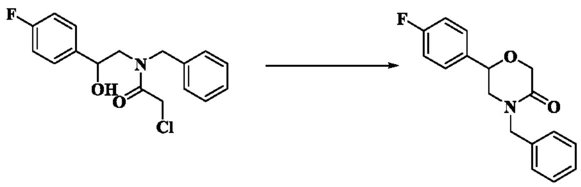

N-Benzyl-2-chloro-N-(2-(4-fluorophenyl)-2-hydroxyethyl)acetamide (16.4g, 50.97mmol) was dissolved in tetrahydrofuran (150 mL) in a ice bath, then potassium tert-butanol (11.44g, 101.94mmol) was added. The reaction was stirred at room temperature for 2 hours. LCMS indicated the end of the reaction. The reaction solution was quenched with saturated sodium chloride solution, and extracted with ethyl acetate. The organic phase was combined, dried over anhydrous sodium sulfate solution, then filtered and concentrated under reduced pressure. The crude product was purified by flash silica gel chromatography (elution: petroleum ether: ethyl acetate=80:20 to 60:40) to obtain the target product which was a yellow solid (12.4 g, yield: 85.3%).

MS: 286.1 [M+1].

Step 5: 4-Benzyl-2-(4-fluorophenyl)morpholin-5,5-d2

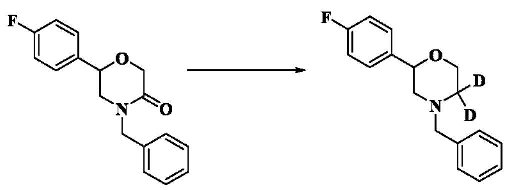

Deuterated lithium aluminum tetrahydride (1.15g, 27.34mmol) was dissolved in tetrahydrofuran (50 mL) in a ice salt bath, and then replaced with nitrogen. Then the mixture solution of 4-benzyl-6 -(4-fluorophenyl) morpholin-3-one (6.0g, 21.03mmol) and aluminum trichloride (3.65g, 27.34mmol) in tetrahydrofuran (50 mL) was added dropwise. After addtion, the reaction solution was reacted under ice bath for 2 hours. LCMS indicated the end of the reaction. The reaction solution was quenched with water (1.15mL), and dilute hydrochloric acid (1N, 10mL) was added to make the reaction solution clear. Then the solution was adjusted to pH 8-9 with 10% sodium hydroxide aqueous solution, and filtered. The filter residue was washed with ethyl acetate, and the filtrate was stratified. The organic phase was washed

with saturated NaCl aqueous solution, dried over anhydrous sodium sulfate, then filtered and concentrated under reduced pressure. The crude product was purified by flash column chromatography (PE: EA = 10:1 to 4:1) to obtaine the target product as colorless oil (5.5 g, yield: 95.7%).

MS: 274.1[M+1].

Step 6: Tert-butyl 2- (4-fluorophenyl)morpholin-4-carboxylate-5,5-d2

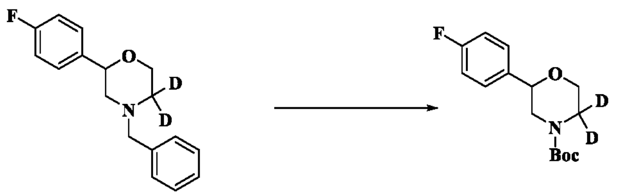

Tert-butoxycarbonyl tert-butyl carbonate (7.90g, 36.22mmol), 4-benzyl-2-(4-fluorophenyl)morpholine-5,5-d2 (5.5g, 20.12mmol), palladium/carbon (1.05g, 8.65mmol) was dissolved in ethanol (80 mL) at room temperature, and then replaced with hydrogen. The reaction was stirred at room temperature for 14 hours. LCMS indicated the end of the reaction. The reaction was filtered and concentrated under reduced pressure. The residues were purified by Flash column chromatography (elution: PE: EA=96:4 to 87:13) to obtain the target product as a colorless oil (4.8 g, yield: 84.2%).

MS: 228. 1[M-55].

Step 7: (*R*)-2-(4-fluorophenyl)morpholin-5,5-d2 and (*R*)-2-(4-fluorophenyl)morpholin-5,5-d2

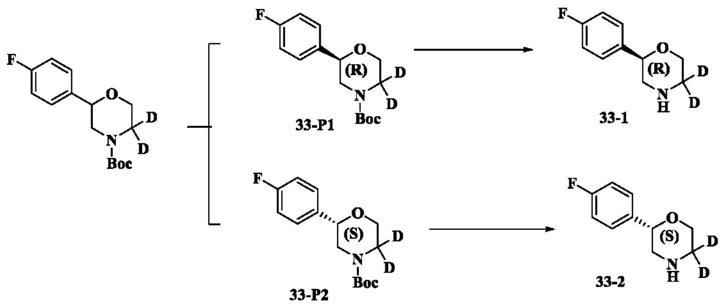


Compound tert-butyl 2-(4-fluorophenyl) morpholin-4-carboxylate-5,5-d2 was resolved by chiral column (chromatography column: CHIRALCEL OJ 4.6 * 150mm, 5 μM; Detection wavelength (nm): 214; Temperature (°C): 35; Flow rate (mL/min): 1; Mobile phase: Hexane: EtOH=90:10; Running time (min): 10; Linear mode: isocratic) to obtain compound 19-P1 (2.0 g, RT (retention time)=3.423 min) and compound 19-P2 (2.1 g, RT (retention time)=3.860 min);

Compound 6-P1 was dissolved in 1,4-dioxane, and hydrochloric acid/dioxane (4 M) was added. The reaction was stirred at room temperature for 1 hour. Solvent was concentrated under reduced pressure. Saturated sodium bicarbonate aqueous solution was added, and the reaction solution was extracted with dichloromethane. The organic phase was dried over oil pump to obtain compound 33-1. Boc protective group was removed by the same method for compound 33-P2 to obtain compound 33-2.

Compound 33-1: $^1$H NMR (400 MHz, DMSO) δ 7.45 - 7.30 (m, 2H), 7.24 - 7.02 (m, 2H), 4.43 - 4.27 (m, 1H), 3.91 - 3.80 (m, 1H), 3.62 - 3.49 (m, 1H), 2.94 - 2.84 (m, 1H), 2.53 - 2.39 (m, 1H).

MS: 184.1[M+1].

Compound 33-2: MS: 184.1[M+1].

**Example 34**

**2-Phenyl-1,4-oxazepane-5,5-d2**

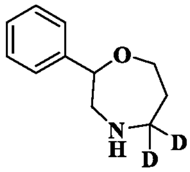

2-Phenyl-1,4-oxazepane-5,5-d2 was obtained according to Example 29 with 2-phenyl-1,4-oxazepane-5-one (prepared by the known method "ACS Combined Science, 2016, vol. 18, # 9, p. 569-574") as the raw material.

MS: 180.1[M+1].

**Example 35**

**2- (Phenyl-4-d)-1,4-oxazepane**

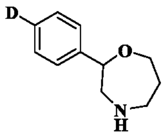

2-(Phenyl-4-d)-1,4-oxazepane was obtained according to Example 29 and Example 1 with 2-(4-bromophenyl)-1,4-oxazepane-5-one (prepared by the known method "ACS Combined Science, 2016, vol. 18, # 9, p. 569-574") as the raw material.

MS: 179.1 [M+1].

**Example 36**

**N. N-dimethyl-4- (morpholin-2-yl-5,5-d2)aniline**

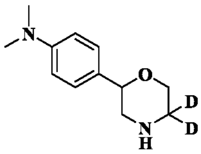

N,N-Dimethyl-4- (morpholin-2-yl-5,5-d2) aniline was obtained according to Example 29 with 6-(4-(dimethylamino)phenyl)morpholin-3-one (prepared by the known method "Bioorg. Med. Chem. Lett. 26 (2016) 1329-1332") as the raw material.

MS: 209.2 [M+1].

**Example 37**

**2-(4-(Methoxy-d3)phenyl)morpholine**

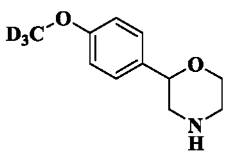

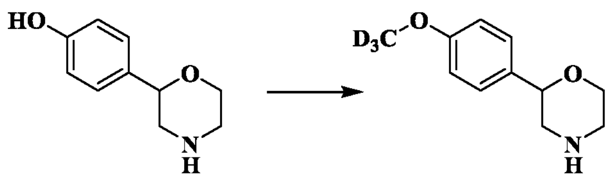

4-(Morpholin-2-yl)phenol (0.3g, 1.67mmol) was dissolved in anhydrous tetrahydrofuran (15mL), and sodium hydrogen (140.60mg, 3.52mmol, 60%w/w) was added in an ice bath under nitrogen atmosphere. The mixture was stirred under ice bath for 10 minutes, then deuteriodomethane (218.38mg, 1.51mmol) was added. The reaction solution was stirred overnight at room temperature. The mixture was cooled to 5°C, quenched with saturated NaCl aqueous solution (5mL). The organic phase was separated, and dried over anhydrous sodium sulfate, and concentrated under reduced pressureconcentrated under reduced pressure to obtain crude product. The crude product was separated by high-pressure liquid preparative chromatography to obtain 2- (4-(methoxy-d3)phenyl) morpholine (81mg, yield: 24.66%).
MS: 197.1[M+1].

**Example 38**

**2-(Phenyl-2-d)morpholine**

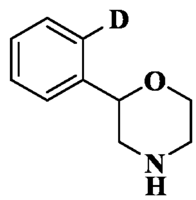

2-(Phenyl-2-d)morpholine was obtained according to Example 28 with 2-(2-bromophenyl) morpholine as the raw material.
MS: 165.1 [M+1].

**Example 39**

**2-(Phenyl-3-d)morpholine**

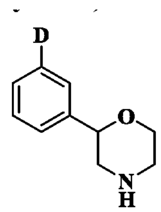

2-(Phenyl-3-d)morpholine was obtained according to Example 28 with 2-(3-bromophenyl)morpholine as the raw material.
MS: 165.1 [M+1].

**Example 40**

**2-(Phenyl-2,4-d2)morpholine**

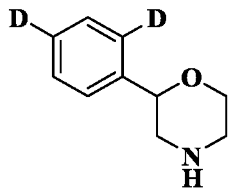

2-(Phenyl-2,4-d2)morpholine was obtained according to Example 29 and Example 28 with 6- (2,4-dibromophenyl)morpholin-3-one (prepared by the known method "Bioorg. Med. Chem. Lett. 26 (2016) 1329-1332") as the raw material.

MS: 166.1 [M+1].

**Example 41**

**2- (Phenyl-2,4,6-d3)morpholine**

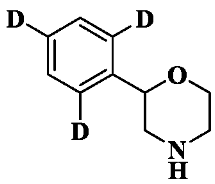

2-(Phenyl-2,4,6-d3)morpholine was obtained according to Example 29 and Example 28 with 6-(2,4,6-trichlorophenyl)morpholin-3-one (prepared by the known method "Bioorg. Med. Chem. Lett. 26 (2016) 1329-1332") as the raw material.

MS: 167.1 [M+1].

**Example 42**

**2-(Phenyl-4-d)morpholin-2-d**

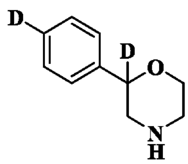

2-(Phenyl-4-d)morpholin-2-d was obtained according to Example 29 and Example 28 with 6-(4-bromophenyl)morpholin-3-one-6-d (prepared by the known method "Bioorg. Med. Chem. Lett. 26 (2016) 1329-1332", wherein sodium borodeuteride was used instead of sodium borohydride) as the raw material.

MS: 166.1 [M+1].

**Example 43**

**2- (Phenyl-2,4,6-d3) morpholin-5,5-d2**

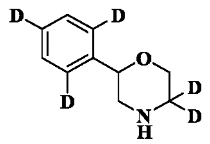

2-(Phenyl-2,4,6-d3)morpholin-5,5-d2 was obtained according to Example 29 and Example 28 with 6-(2,4,6-trichlorophenyl)morpholin-3-one (prepared by the known method "Bioorg. Med. Chem. Lett. 26 (2016) 1329-1332") as the raw material.

MS: 169.1 [M+1].

**Example 44**

**2- (M-methylphenyl)morpholine-5,5-d2**

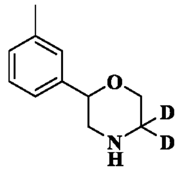

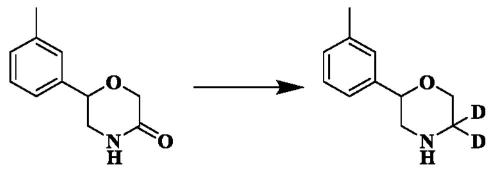

2-(m-Methylphenyl)morpholin-5,5-d2 was obtained according to Exmaple 29 with 6-(m-methylphenyl)morpholin-3-one as the raw material.

MS: 180.1 [M+1].

**Example** 45

**2- (3-Chlorophenyl)morpholin-5,5-d2**

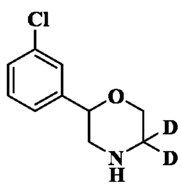

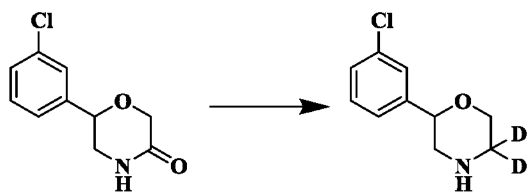

2-(3-Chlorophenyl)morpholin-5,5-d2 was obtained according to Example 29 with 6-(3-chlorophenyl)-morpholin-3-one as the raw material.

MS: 200.1 [M+1].

The single chiral compounds of Examples 29-45 were obtained according to the resolution method of Example

28:

| Examples | Single chiral compounds | MSm/z (ESI) |
|---|---|---|
| 29 | 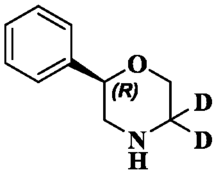 29-1 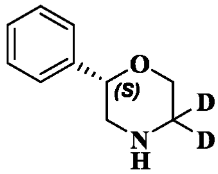 29-2 | 166.1 |
| 30 | 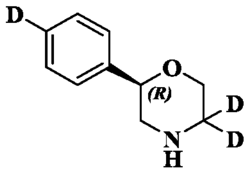 30-1 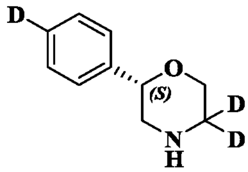 30-1 | 167.1 |
| 31 | 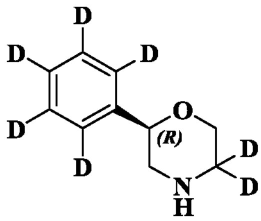 31-1 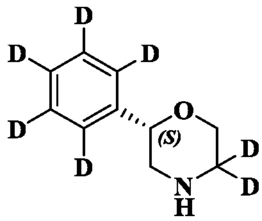 31-2 | 171.1 |
| 32 | 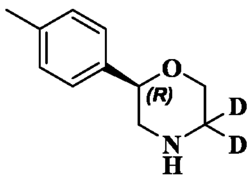 32-1 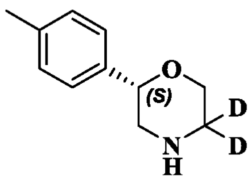 32-2 | 180.1 |
| 33 | 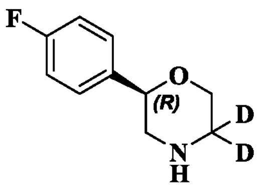 33-1 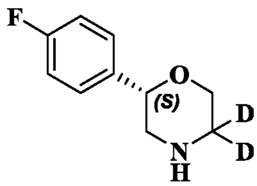 33-2 | 184.1 |
| 34 | 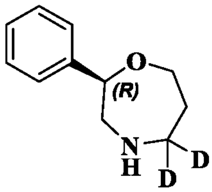 34-1 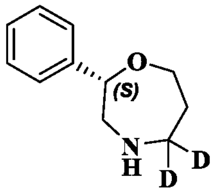 34-2 | 180.1 |

(continued)

| Examples | Single chiral compounds | MSm/z (ESI) |
|---|---|---|
| 35 | 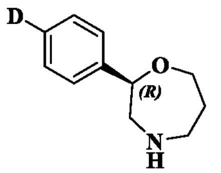 35-1 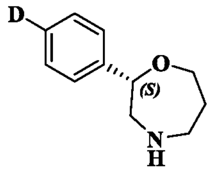 35-2 | 179.1 |
| 36 | 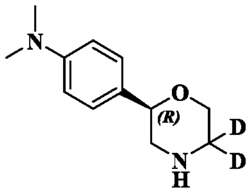 36-1 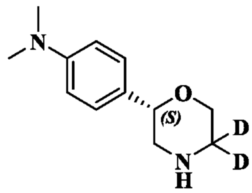 36-2 | 209.2 |
| 37 | 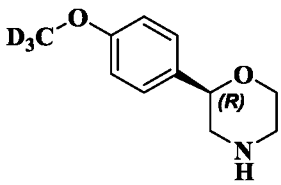 37-1 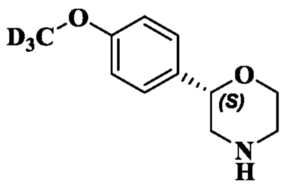 37-2 | 197.1 |
| 38 | 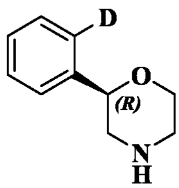 38-1 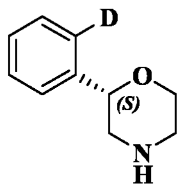 38-2 | 165.1 |
| 39 | 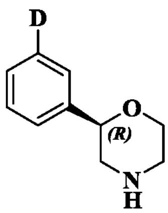 39-1 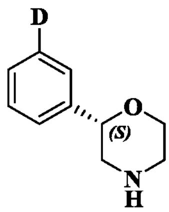 39-2 | 165.1 |
| 40 | 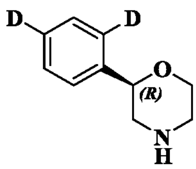 40-1 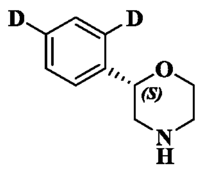 40-2 | 166.1 |

(continued)

| Examples | Single chiral compounds | MSm/z (ESI) |
|---|---|---|
| 41 | 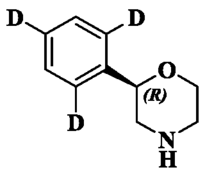 41-1 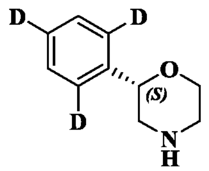 41-2 | 167.1 |
| 42 | 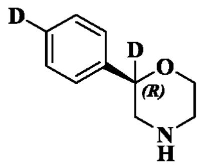 42-1 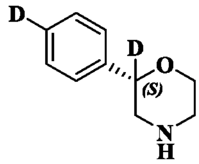 42-2 | 166.1 |
| 43 | 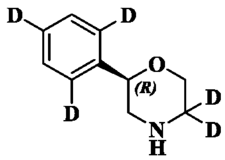 43-1 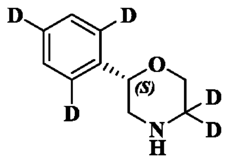 43-2 | 169.1 |
| 44 | 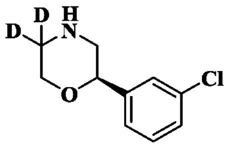 44-1 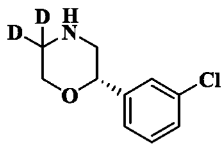 44-2 | 180.1 |
| 45 | 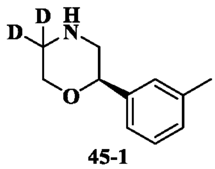 45-1 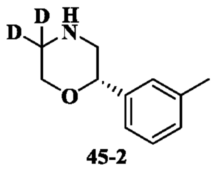 45-2 | 200.1 |

**Example 46**

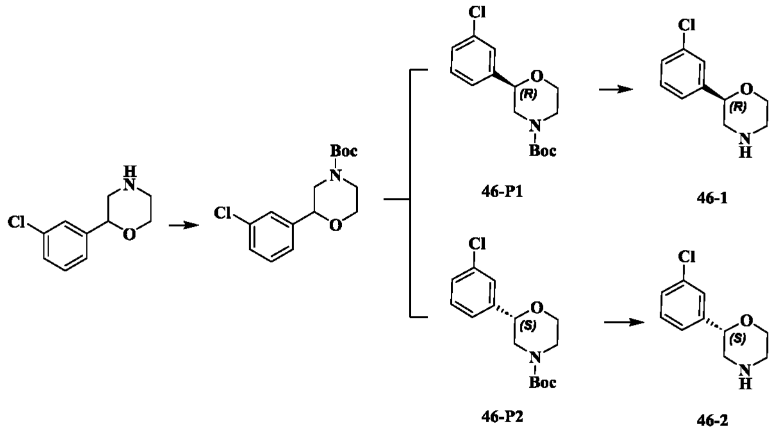

46-P1 46-1

46-P2 46-2

2-(3-Chlorophenyl)morpholine hydrochloride (0.6g, 2.56mmol) and triethylamine (777.98 mg, 7.69 mmol, 1.07 mL) were dissolved in dichloromethane (20mL), and di-tert-butyl dicarbonate (727.11 mg, 3.33 mmol) was added under nitrogen atmosphere. The mixture was stirred at 18°C for 3 hours, and then quenched with saturated NaCl aqueous solution (50mL). The reaction solution was extracted with methylene chloride (50mL). The organic phase was combined. The organic phase was washed with saturated NaCl aqueous solution (50mL×2), dried over anhydrous sodium sulfate and concentrated under reduced pressure. The residues were purified by flash silica gel chromatography (elution: petroleum ether: ethyl acetate = 100:0 to 80:20) to obtain the target product tert-butyl 2-(3-chlorophenyl) morpholin-4-carboxylate (0.4g, 1.34mmol, yield: 52.42%).

Tert-butyl 2-(3-chlorophenyl)morpholin-4-carboxylate was resolved by chiral column (chromatography column: CHIRALCEL OJ 4.6 * 150mm, 5) μM; Detection wavelength (nm): 214; Temperature (°C): 35; Flow rate (mL/min): 1; Mobile phase: Hexane: EtOH=70:30; Running time (min): 10; Linear mode (isocratic) to obtain compound 46-P1 (RT (retention time)=2.543 min) and compound 46-P2 (RT (retention time)=2.843 min);

Compound 46-1 was obtained by removing the Boc protective group from Compound 46-P1 according to the seventh step of Method 2 in Example33, and compound 46-2 was obtained by removing the Boc protective group from compound 46-P2 according to the seventh step of Method 2 in Example33.

Compound 46-1 MS: 198.1 [M+1].

Compound 46-2 MS: 198.1 [M+1].

**Example 47**

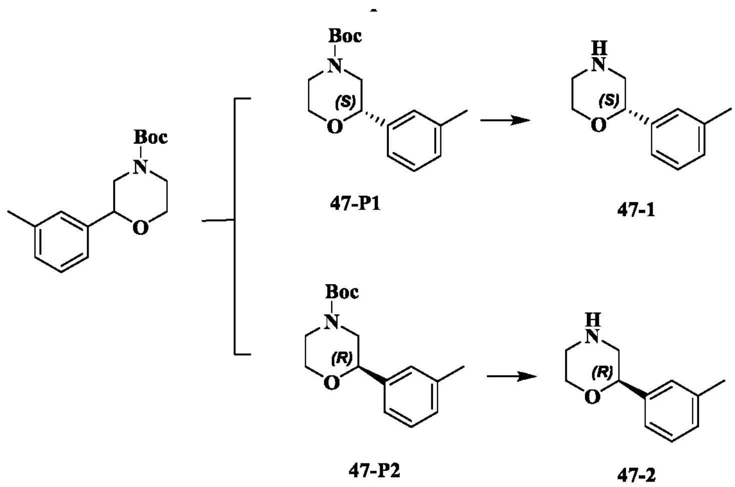

47-P1 47-1

47-P2 47-2

Tert-butyl 2-(m-benzyl)morpholine-4-carboxylate was obtained according to Method 2 of Example 33 with m-methyl benzaldehyde as the raw material.

Tert-butyl 2-(m-benzyl)morpholine-4-carboxylate was resolved by chiral column (chromatography column: CHIRALCEL OJ 4.6*150mm, 5) μM; Detection wavelength (nm): 214; Temperature (°C): 35; Flow rate (mL/min): 1; Mobile phase: Hexane: EtOH=70:30; Running time (min): 10; Linear mode: isocratic), to obtain the compound 47-P1 (RT (retention time)=2.807 min) and compound 47-P2 (RT (retention time)=3.300 min);

Compound 47-1 was obtained by removing the Boc protective group from Compound 47-P1 according to the seventh step of Method 2 in Example33, and compound 47-2 was obtained by removing the Boc protective group from compound 47-P2 according to the seventh step of Method 2 in Example33.

Compound 47-1 MS: 178.1 [M+1].

Compound 47-2 MS: 178.1 [M+1].

### Example 48

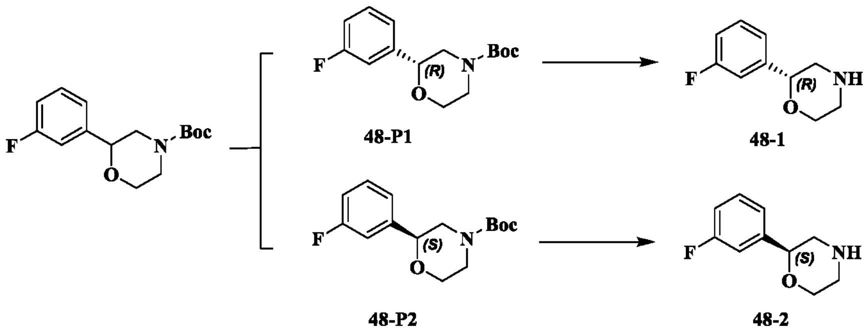

Tert-butyl 2-(3-fluorophenyl)morpholine-4-carboxylate was obtained according to Method 2 of Example 33 with 3-fluorobenzaldehyde as the raw material.

Tert-butyl 2-(3-fluorophenyl)morpholine-4-carboxylate was resolved by chiral column (chromatography column: CHIRALCEL OJ 4.6 * 150mm, 5) μM; Detection wavelength (nm): 214; Temperature (°C): 35; Flow rate (mL/min): 1; Mobile phase: Hexane: EtOH=90:10; Running time (min): 10; Linear mode (isocratic), to obtain compounds 48-P1 (RT (retention time)=3.077 min) and 48-P2 (RT (retention time)=3.467 min);

Compound 48-1 was obtained by removing the Boc protective group from Compound 48-P1 according to the seventh step of Method 2 in Example33, and compound 48-2 was obtained by removing the Boc protective group from compound 48-P2 according to the seventh step of Method 2 in Example33.

Compound 48-1 MS: 182.1 [M+1].

Compound 48-2 MS: 182.1 [M+1].

### Biological Assay and Evaluation

The present invention is further illustrated below in combination with the following test examples, which are not intended to limit the scope of the present invention.

### Test Example1. Determination of the effect of the present compound on cAMP content in cells which stably expressTAAR1 receptor

#### 1.1 Experimental purpose

Determination of the activation effect of compounds on TAAR1 receptors.

#### 1.2. Experimental reagents and instruments

1.2.1 Instruments:

384 well - test plate (Perkin Elmer; 6007680)

96-well conical btm PP Pit nature RNASE/Dnase-free plate (ThermoFisher; 249944)

EnVision (Perkin Elmer)

1.2.2 Reagents:

Fetal Bovine Serum (Gibco; 10999141)
Ham's F-12K (Kaighn's) Medium (Hyclone; SH30526.01)
Penicillin-Streptomycin, Liquid (Gibco; 15140122)
Hygromycin B
BSA stabilizer (Perkin Elmer; CR84-100)
cAMP kit (Cisbio; 62AM4PEC)
IBMX (Sigma; I5879)
HEPES (Gibco; 15630080)
HBSS (Gibco; 14025076)
TrypLE (ThermoFisher; 12604021)
DMEM

**1.3 Experimental methods**

1) Preparation of experimental buffer: 1* HBSS + 20mM HEPES + 0.1% BSA + 500 μM IBMX.
2) Complete medium:

Ham's F12K + 10% Fetal Bovine Serum +1* Penicillin-Streptomycin + 200 μg/mL Hygromycin B for CHO
DMEM + 10% Fetal Bovine Serum +1* Penicillin-Streptomycin + 200 μg/mL Hygromycin B for HEK293

3) TAAR1 cell line was cultured in complete medium at 37 °C and 5% $CO_2$; After TrypLE digestion, the cells were resuspended in the experimental buffer and seeded into a 384 cell culture plate with a density of 8000 cells/15ul/well.
4) The compound was diluted with experimental buffer and added 5μL to each well to culture at 37 °C for 30 minutes.
5) cAMP-d2 and Anti cAMP Eu3+ were freeze thaw, and diluted 20 times with lysis buffer; 10μL cAMP-d2 was added to the experimental well, and then 10μL Anti cAMP Eu3+ was added into the experimental well; the reaction plate was centrifuged at 200g for 30 seconds at room temperature , and stand at 25 °C for 1 hour; data was collected by Envision.

**1.4. Experimental data processing methods**

1)

$$Z' \text{ factor} = 1-3*(SDMax+SDMin)/(MeanMax-MeanMin);$$

2)

$$CVMax = (SDMax/MeanMax)*100\%;$$

3)

$$CVMin = (SDMin/MeanMin)*100\%;$$

4)

$$S/B = Singal/Background;$$

5) EC50 of compounds was calculated using GraphPad nonlinear fitting formula:

$$Y=Bottom + (Top-Bottom)/(1+10^{\wedge}((LogEC_{50}-X)*HillSlope))$$

X: Log value of the compound concentration; Y: Activation %

**1.5 Experimental results**

**Table1 EC50 values of the effects of compounds on cAMP content in cells which stably express TAAR1 receptor**

| Number | **TAAR1 cAMP Agonist $EC_{50}$(nM)** |
|---|---|
| Compound A | 7.1 |
| Example 6-2 | 3.4 |
| Example 7-2 | 5.9 |
| Example 10-2 | 5.4 |
| Example 16-1 | 21.3 |
| Example 17-2 | 8.3 |
| Example 19-2 | 13.6 |
| Example 25-2 | 206.9 |
| Example 28-2 | 3.0 |
| Example 33-1 | 7.9 |
| Example 46-1 | 17.7 |
| Example 47-2 | 11.5 |
| Example 48-1 | 2.1 |

**1.6. Experimental conclusion**

The example compounds of the present invention shows good activation effect in the experiment of the effect on cAMP in cells which stably expressTAAR1 receptor.

**Test Example 2. Pharmacodynamic evaluation of the present compounds in mice model of high spontaneous activity induced by phencyclidine (PCP)**

**2.1. Experimental purpose**

The purpose of the experiment is to evaluate the efficacy of compounds in a high spontaneous activity model of C57BL/6 mice induced by phencyclidine (PCP) using the spontaneous activity method.

**2.2. experimental animal**

C57BL/6 mice, provided by Shanghai Lingchang Biotechnology Co., Ltd., male, 6-8 weeks, 20-24 g, 36 mice.

**2.3 Experimental instruments**

**Table 2. Main experiment instruments**

| Name | Supplier/Manufacturer | Model |
|---|---|---|
| Weighing balance | Shanghai Sainyu Hengping Scientific Instrument Co, LTD | MP5002 model-SHP021018060695 |
| Electronic balance | Sartorius | SECURA225D-1CN |
| Test box | Shanghai Lanyuao Industry and Trade Co., Ltd | Internal length, width, and height (27.4cm*27.4cm*35cm) |
| Anymaze software | Stoelting | 60003 |
| Pipette | Eppendorf | 5000ul-4057098 |

**Table 3. Main experiment reagents**

| Name | Supplier/Manufa cturer | Batch number |
|---|---|---|
| Hydroxypropyl-β-cyclodextri n (HP-β-CD) | Shanghai Bide Pharmaceutical Technology Co., Ltd | BKX281 |
| Saline | Zhejiang Dubang Pharmaceutical Co., Ltd | 2107260107 |
| Phencyclidine hydrochloride | Shanghai Yuansibiaowu Technology Co., Ltd | 001002027FY082201 90510 |

**2.4 Experimental methods**

2.4.1 Grouping

The day before the experimental test, the animals were placed in the laboratory and acclimated for 8 hours.

On the day of adaptation, the animals were randomly grouped according to their weight.

2.4.2 Experimental administration

The animals were weithed and treated with solvent control (20% HP-β-CD), compounds of the present invention (oral administration) respectively. 30 minutes later, modeling (intraperitoneal administration) was performed, and the animals were given physiological saline or phencyclidine, with dosage volume of 10 mL/kg.

2.4.3 Testing

After the administration, the animals were placed in observation box and the spontaneous activity was detected for 30 minutes. The moving distance (meters) of the animals in the observation box was counted every 5 minutes.

After 30 minutes, the animals were taken out for modeling and then placed in the observation box to test their spontaneous activity for 60 minutes. The moving distance (meters) of the animals in the observation box was counted every 5 minutes.

After the test, the animals were taken out and the observation box was cleaned with 75% alcohol.

The test results were exported from the computer.

2.4.4 Data collection and analysis

Excel software was used to collect data. A Two-Way ANOVA or One-Way ANOVA was conducted used Prism (Graph pad software, Inc.) software. $p<0.05$ was considered significant difference.

**2.5 Experimental results**

The effects of the compounds on the moving distance in the high spontaneous activity model of C57BL/6 mice

induced by phencyclidine (PCP) are shown in Table 4. Compared to the solvent control group (20% HP- β-CD+Saline), PCP can significantly induce high spontaneous activity within 60 minutes after modeling. The compounds of the present invention can significantly reduce the total spontaneous activity distance within 60 minutes at the dose of 1-10 mg/kg compared to the molding group (20%HP-β-CD+PCP 5 mg/kg). No other obvious side effects were found during the experiment.

**Table 4. Effects of compounds on moving distance at different times in the high spontaneous activity model of mice induced by PCP**

| Groups | Number of animals (N) | Total distance (m, Mean±SEM) |
|---|---|---|
| | | 60 minutes |
| 20%HP-β-CD±Saline | 12 | 14.71 ± 3.67 |
| 20%HP-P-CD+PCP 5 mg/kg | 12 | 197.01 ± 13.42### |
| Compound A 6 mg/kg+ PCP 5 mg/kg | 12 | 113.09 ± 12.69*** |
| 20%HP-β-CD+Saline | 12 | 29.28 |
| 20%HP-P-CD+PCP 5 mg/kg | 12 | 275.8 |
| Compound 10-2 3 mg/kg+PCP 5 mg/kg | 12 | 152.6*** |
| Example17-2 1 mg/kg+PCP 5 mg/kg | 12 | 140.1*** |
| Example 17-2 3 mg/kg+PCP 5 mg/kg | 12 | 114.1*** |
| Example 33-1 1 mg/kg+PCP 5 mg/kg | 12 | 136.4*** |
| 实施例 33-1 3 mg/kg+PCP 5 mg/kg | 12 | 126.3*** |
| Example 33-1 10 mg/kg+PCP 5 mg/kg | 12 | 83.23*** |
| 20%HP-β-CD+Saline | 12 | 14.71 ± 3.67 |
| 20%HP-β-CD+PCP 5 mg/kg | 12 | 197.01 ± 13.42### |
| Example28-2 3 mg/kg+PCP 5 mg/kg | 12 | 113.09 ± 12.69*** |
| Note: The total distance between groups was analyzed using Dunnett's test for multiple comparisons in One-way ANOVA, ###p<0.001, p Value (compared with 20%HP-β-CD + Saline group); ***p<0.001 p Value (compared with 20%HP-β-CD + PCP 5 mg/kg group). | | |

**2.6. Experimental conclusion**

In this experiment, we evaluated the efficacy of the compounds of the present invention in the mouse model of high spontaneous activity induced by phencyclidine. Phencyclidine (5 mg/kg) can significantly induce high spontaneous activity within 60 minutes after modeling. The compounds of the present invention can significantly inhibit the high spontaneous activity induced by phencyclidine, and significantly reduce the total distance of spontaneous activity within 60 minutes.

**Test Example 3. Pharmacokinetic Assay of Balb/c Mice**

**3.1. Research purpose**

Balb/c Mice were used as test animals. The pharmacokinetic behavior of the compounds of the present invention was studied in the plasma and brain of mice administered orally at the dose of 5mg/kg.

**3.2 Test Protocol**

3.2.1 Test drug

Compounds of the present invention, self-made.

3.2.2 Experimental animals

Balb/c Mice, male, Shanghai Jiesijie Experimental Animal Co., Ltd., animal production license number (SCXK (Shanghai) 2013-0006 N0.311620400001794).

3.2.3 Administration

After fasting overnight, 24 male Balb/c mice were administered p.o. at a dose of 5 mg/kg and an administration volume of 10 mL/kg, respectively.

3.2.4 Drug formulation

Drug formulation for oral administration: 0.5% CMC-Na (1% Tween 80).
5g of carboxymethyl cellulose sodium (HEC, CMC Na, viscosity: 800-1200Cps) was weighed and dissolved in 1000 mL of pure water. 10 g Tween 80 was added, and mixed evenly under stirring to form a clarified solution.
Compound 16-1 was weighed and added into a 4-mL glass bottle. 2.4 mL of the solution was added and sonicated for 10 minutes to obtain colorless clear solution with the concentration of 0.5 mg/mL.

3.2.5 Sample collection:

After $CO_2$ euthanasia of experimental animals, heart blood and brain tissue were collected at 0.25 h, 0.5 h, 1 h, 2 h, 4 h, 6 h, 8 h, and 24 h after administration. The blood was placed in EDTA-2K tubes and centrifuged at 4°C and 6000 rpm for 6 minutes to separate plasma. The brain tissue was washed with pre-cooled PBS, dried, weighed, and stored at -80°C. The animals were feed 4 hours after administration.

3.2.6 Sample process

1) 160 μL of acetonitrile was added to 40 μL of the plasma sample for precipitation, and then the mixture was centrifuged at 3500×g for 5-20 min.
2) 100μL of the supernatant was taken to analyze the concentration of the test compound by LC/MS/MS.

3.2.7 Liquid chromatography analysis

• Liquid chromatography condition: Shimadzu LC-20AD pump
• Mass spectrometry condition: AB Sciex API 4000 mass spectrometer
• Chromatographic column: phenomenex Gemiu 5 μm C18 50 × 4.6 mm
• Mobile phase: Eluent A was 0.1% formic acid in water, and Eluent B was acetonitrile
• Flow rate: 0.8 mL/min
• Elution time: 0-4.0 minutes, the eluent is as follows:

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

**3.3. Experimental results and analysis**

The main pharmacokinetic parameters were calculated using WinNonlin8.1, and the results of the pharmacokinetic experiments in mouse are shown in the table below.

**Table 5. Pharmacokinetic parameters of the compounds of the present invention after oral administration in mice**

| Number | | $t_{max}$(h) | $C_{max}$(ng/mL) | $AUC_{0-t}$(ng/mL×h) | $t_{1/2}$(h) |
|---|---|---|---|---|---|
| Example 6-2 | Brain | 0.25 | 3033 | 6479 | 0.8 |
| | Plasma | 0.25 | 2050 | 4299 | 0.8 |
| Example 7-2 | Brain | 0.5 | 2503 | 4410 | 0.9 |
| | Plasma | 0.5 | 1557 | 2845 | 0.8 |
| Example 10-2 | Brain | 0.5 | 3537 | 12522 | 2.5 |
| | Plasma | 0.25 | 1320 | 5432 | 2.4 |
| Example 16-1 | Brain | 0.25 | 1740 | 5633 | 2.9 |
| | Plasma | 0.25 | 890 | 2272 | 1.9 |
| Example 17-2 | Brain | 0.5 | 3157 | 7505 | |
| | Plasma | 0.25 | 1454 | 2630 | 1.3 |
| Example 19-2 | Brain | 2.0 | 2513 | 8642 | 1.1 |
| | Plasma | 2.0 | 705 | 2756 | 3.7 |
| Example 28-2 | Brain | 0.25 | 2113 | 3978 | 1.3 |
| | Plasma | 0.25 | 1340 | 2816 | 1.4 |
| Example 33-1 | Brain | 0.25 | 8363 | 15262 | 3.2 |
| | Plasma | 0.25 | 1970 | 5034 | 3.2 |

**4. Experimental conclusion**

The data in the table shows that in the pharmacokinetic evaluation experiment of mice, the compounds of the present invention show high exposure after oral administration.

**Test Example4. Determination of the effect of the present compounds on calcium current in the cells which stably express ADRA1A receptor**

**1. Experimental purpose**

Determination of the activation effect of the compounds on ADRA1A receptors.

**2. Experimental reagents and instruments**

2.1. Instruments

384 well - test plate (Corning; 3764)
384-well compound plate (Corning; 3657)
384-well flipr tetra pipet tips (Molecular Devices; 9000-0764)
FLIPR Tetra (Molecular Devices)

2.2. Reagents

Fetal Bovine Serum (AusGeneX; BS500-S)
Ham's F-12K (Kaighn's) Medium (Hyclone; H30526.01)
Penicillin-Streptomycin, Liquid (Gibco; 5140122)
Hygromycin B (Invivogen; ant-hg-5)
PBS (Solarbio; 1020-500)
1X TrypLE Express Enzyme, no phenol red (500ml) (ThermoFisher Scientific; 12604021)
FLIPR Calcium 6 Assay kit (Molecular Devices; 191)
HEPES (Gibco;630080)
HBSS (Gibco;025076)

**3. Experimental methods**

1) Flp In-CHO-ADRA1A Stable Pool cell lines were cultured in complete medium at 37°C, 5% $CO_2$ to 70%-90% fusion.
2) Complete culture medium: Ham's F12K + 10%FBS + 1* Penicillin - Streptomycin + 600μg/mL Hygromycin B; Cell inoculation medium: Ham's F12K + + 10% FBS; Experimental buffer: 1X HBSS + 20mM HEPES
3) After TrypLE digestion, the cells were re-suspended in the inoculation medium and inoculated in 384 well cell culture plate (Corning, 3764). 7000 cells were inoculated into each well and incubated overnight at 37°C and 5% $CO_2$.
4) 20X Component A was freeze-thaw to room temperature, and then diluted with experimental buffer to 2X working concentration including 5mM Probenecid, which was then placed at room temperature for use.
5) The cell culture plate was taken out, and let to stand at room temperature for 10 minutes. The plate was subject to inverted centrifugation at 200g, and RT centrifugation for 1 second, and then 20μL Assay buffer was quickly added to the cell culture plate. Then 20μL 2X Component A containing 5mM Probenecid was added to each experimental well, which was centrifuged by RT at 200g for 3-5 seconds, and incubated at 37 °C for 2 hours.
6) The working solution (5X) for the compound to be tested was prepared and placed and at room temperature for use.
7) The cell culture plate was taken out and let to stand at room temperature for 10 minutes.
8) 10μL diluted compound working solution in step 6) was added to each the experimental well using FLIPR Tetra and data were collected.

**4. Experimental data processing methods**

1)

$$\text{Z' factor} = 1\text{-}3*(\text{SDMax}+\text{SDMin})/(\text{MeanMax}-\text{MeanMin});$$

2)

$$\text{CVMax} = (\text{SDMax}/\text{MeanMax})*100\%;$$

3)

$$\text{CVMin} = (\text{SDMin}/\text{MeanMin})*100\%;$$

4)

$$\text{S/B} = \text{Singal/Background};$$

5) EC50 of the compounds was calculated by GraphPad nonlinear fitting formula:

$$Y=\text{Bottom} + (\text{Top}-\text{Bottom})/(1+10\text{^}((\text{LogEC}_{50}-X)*\text{HillSlope}))$$

X: Log value of the compound concentration; Y: Activation %

5. Experimental results

**Table 6. EC50 values of the compounds on calcium current in cells which stably express ADRA1A receptor**

| Number | **ADR-α1 Ca Flux $EC_{50}$(nM)** |
|---|---|
| Example 10-2 | 1170 |
| Example 17-2 | 3166 |
| Example 33-1 | 1415 |

**6. Experimental conclusion**

From the data in the table, it can be seen that the example compounds of the present invention exhibits weak partial activation activity in the experiment of effect on calcium current in the cells which stably express ADRA1A receptor.

**Claims**

1. A compound of formula (I), a stereoisomer thereof or a pharmaceutically acceptable salt thereof,

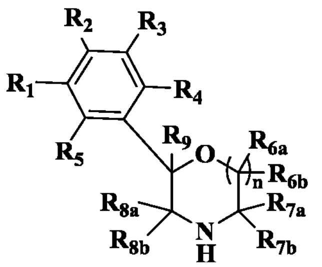

(I)

wherein,

$R_1$, $R_2$, $R_3$, $R_4$, $R_5$, and $R_9$ are each independently selected from the group consisting of hydrogen, deuterium, halogen, amino, hydroxyl, cyano, $C_{1-6}$ alkyl, $C_{1-6}$ alkoxy, $C_{1-6}$ deuterated alkyl, $C_{1-6}$ deuterated alkoxy, $C_{1-6}$ haloalkyl and $C_{3-12}$ cycloalkyl, which can be each optionally further substituted;
$R_{6a}$, $R_{6b}$, $R_{7a}$, $R_{7b}$, $R_{8a}$ and $R_{8b}$ are each independently selected from the group consisting of hydrogen and deuterium;
n is 0, 1, 2, or 3;
with the proviso that, at least one of $R_1$, $R_2$, $R_3$, $R_4$, $R_5$, $R_9$, $R_{6a}$, $R_{6b}$, $R_{7a}$, $R_{7b}$, $R_{8a}$ and $R_{8b}$ contains D.

2. The compound, the stereoisomer thereof or the pharmaceutically acceptable salt thereof according to claim 1, **characterized in that** the compound is further shown as formula (I-1) or (I-2),

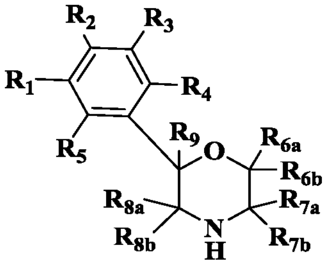

( I-1 )

or

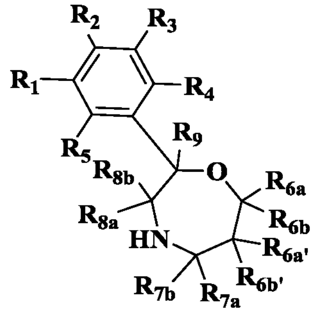

( I-2 )

wherein,

$R_1$, $R_2$, $R_3$, $R_4$, $R_5$ and $R_9$ are each independently selected from the group consisting of hydrogen, deuterium, halogen, amino, hydroxyl, cyano, $C_{1-6}$ alkyl, $C_{1-6}$ alkoxy, $C_{1-6}$ deuterated alkyl, $C_{1-6}$ deuterated alkoxy, $C_{1-6}$ haloalkyl and $C_{3-12}$ cycloalkyl, which are optionally further substituted by substituents selected from the group consisting of deuterium, halogen, $C_{1-6}$ alkyl, $C_{1-6}$ alkoxy, $C_{1-6}$ deuterated alkyl, $C_{1-6}$ deuterated alkoxy and $C_{1-6}$ haloalkyl; preferably, $R_1$, $R_2$, $R_3$, $R_4$, $R_5$ and $R_9$ are each independently selected from the group consisting of hydrogen, deuterium, fluorine, chlorine, methyl, deuterated methyl, deuterated methoxy and cyclopropyl; further preferably, $R_1$, $R_2$, $R_3$, $R_4$, $R_5$ and $R_9$ are each independently selected from the group consisting of hydrogen, deuterium, methyl, fluorine, $-OCD_3$ and $-N(CH_3)_2$;

$R_{6a}$, $R_{6b}$, $R_{6a'}$, $R_{6b'}$, $R_{7a}$, $R_{7b}$, $R_{8a}$ and $R_{8b}$ are each independently selected from the group consisting of hydrogen and deuterium;

With the proviso that, at least one of $R_1$, $R_2$, $R_3$, $R_4$, $R_5$, $R_9$, $R_{6a}$, $R_{6b}$, $R_{6a'}$, $R_{6b'}$, $R_{7a}$, $R_{7b}$, $R_{8a}$ and $R_{8b}$ contains D.

3. The compound, the stereoisomer thereof or the pharmaceutically acceptable salt thereof according to claim 2, **characterized in that** the compound is further shown as formula (I-3) or (I-4),

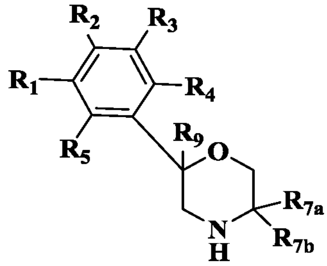

( I-3)

or

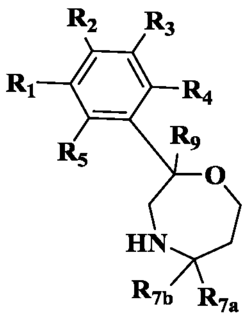

( I-4 ) .

4. The compound, the stereoisomer thereof or the pharmaceutically acceptable salt thereof according to claim 1, **characterized in that** the compound is further shown as formula (II),

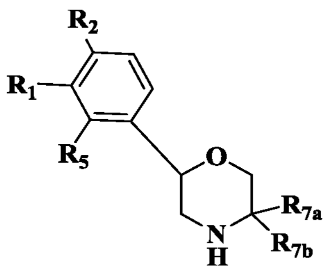

( II )

$R_1$, $R_2$ and $R_5$ are each independently selected from the group consisting of hydrogen, deuterium, halogen, amino, hydroxyl, cyano, $C_{1-3}$ alkyl, $C_{1-3}$ alkoxy, $C_{1-3}$ deuterated alkyl, $C_{1-3}$ deuterated alkoxy, $C_{1-3}$ haloalkyl and $C_{1-3}$ alkylamine;
$R_{7a}$ and $R_{7b}$ are each independently selected from the group consisting of hydrogen and deuterium;
at least one of $R_1$, $R_2$, $R_5$, $R_{7a}$ and $R_{7b}$ contains D.

**5.** The compound, the stereoisomer thereof or the pharmaceutically acceptable salt thereof according to claim 4, **characterized in that** the compound is further shown as formula (II-2),

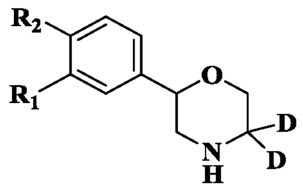

( II-2 )

$R_1$ and $R_2$ are each independently selected from the group consisting of hydrogen, deuterium, halogen, amino, hydroxyl, $C_{1-3}$ alkyl, $C_{1-3}$ alkoxy, $C_{1-3}$ deuterated alkyl, $C_{1-3}$ deuterated alkoxy, $C_{1-3}$ haloalkyl and $C_{1-3}$ alkylamine; preferably, $R_1$ and $R_2$ are each independently selected from the group consisting of hydrogen, deuterium, fluorine, chlorine, dimethylamino, methyl, methoxyl, deuterated methyl and deuterated methoxyl.

**6.** The compound, the stereoisomer thereof or the pharmaceutically acceptable salt thereof according to claim 5, **characterized in that** the compound is further shown as formula (II-2-a) or (II-2-b),

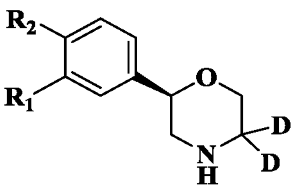

( II-2-a) or

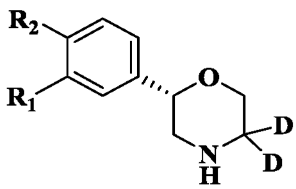

( II-2-b ) .

**7.** A compound of formula (I'), a stereoisomer thereof or a pharmaceutically acceptable salt thereof,

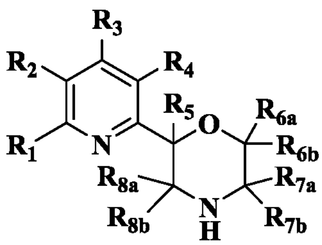

( I ')

wherein,

$R_1$, $R_2$, $R_3$ and $R_4$ are each independently selected from the group consisting of hydrogen, deuterium, halogen, amino, hydroxyl, cyano, $C_{1-6}$ alkyl, $C_{1-6}$ deuterated alkyl, $C_{1-6}$ haloalkyl and $C_{3-12}$ cycloalkyl;
$R_5$,$R_{6a}$,$R_{6b}$,$R_{7a}$,$R_{7b}$,$R_{8a}$ and $R_{8b}$ are each independently selected from the group consisting of hydrogen and deuterium;
with the proviso that,
$R_1$, $R_2$, $R_3$. $R_4$, $R_5$, $R_{6a}$, $R_{6b}$, $R_{7a}$, $R_{7b}$, $R_{8a}$ and $R_{8b}$ are not hydrogen at the same time, and when $R_1$, $R_2$, $R_3$ and $R_4$ are each independently selected from the group consisting of halogen, cyano, hydroxyl, amino, $C_{1-6}$

alkyl and $C_{1-6}$ haloalkyl, at least one of $R_5$, $R_{6a}$, $R_{6b}$, $R_{7a}$, $R_{7b}$, $R_{8a}$ and $R_{8b}$ is D.

**8.** A compound of formula (V), a stereoisomer thereof or a pharmaceutically acceptable salt thereof,

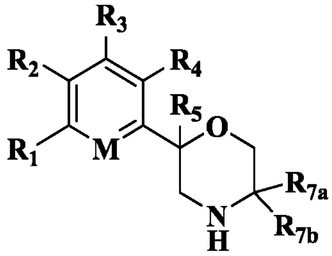

(V)

wherein,

M is selected from the group consisting of N and $CR_a$;
$R_a$, $R_1$, $R_2$, $R_3$ and $R_4$ are each independently selected from the group consisting of hydrogen, deuterium, halogen, amino, hydroxyl, cyano, $C_{1-6}$ alkyl, $C_{1-6}$ deuterated alkyl, $C_{1-6}$ haloalkyl and $C_{3-12}$ cycloalkyl;
$R_5$, $R_{7a}$ and $R_{7b}$ are each independently selected from the group consisting of hydrogen and deuterium, and at least one of them is deuterium .

**9.** The compound, the stereoisomer thereof or the pharmaceutically acceptable salt thereof according to claims 7 or 8, **characterized in that** $R_1$, $R_2$, $R_3$ and $R_4$ are each independently selected from the group consisting of hydrogen, deuterium, halogen, $C_{1-3}$ alkyl, $C_{1-3}$ deuterated alkyl and $C_{3-6}$ cycloalkyl; preferably selected from the group consisting of hydrogen, deuterium, fluorine, chlorine, methyl, deuterated methyl and cyclopropyl.

**10.** The compound, the stereoisomer thereof or the pharmaceutically acceptable salt thereof according to claim 8, **characterized in that** the compound is further shown as formula (VI),

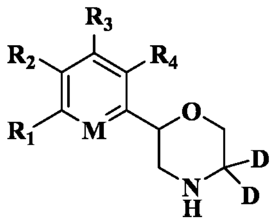

(VI) ,

M is selected from the group consisting of N and CH;
$R_1$, $R_2$, $R_3$ and $R_4$ are each independently selected from the group consisting of hydrogen, deuterium, halogen, $C_{1-3}$ alkyl, $C_{1-3}$ deuterated alkyl and $C_{3-6}$ cycloalkyl;
preferably, $R_1$ and $R_2$ are each independently selected from the group consisting of fluorine, $C_{1-3}$ alkyl, $C_{1-3}$ deuterated alkyl and $C_{3-6}$ cycloalkyl; $R_3$ and $R_4$ are each hydrogen.

**11.** The compound, the stereoisomer thereof or the pharmaceutically acceptable salt thereof according to claim 10, **characterized in that** the compound is further shown as formula (VI-1) or (VI-2),

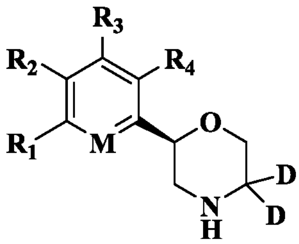

( VI-1 ) or

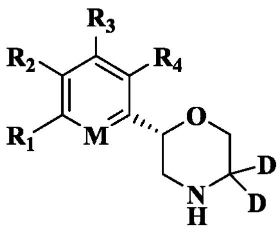

( VI-2 ) .

**12.** A compound, the stereoisomer thereof or the pharmaceutically acceptable salt thereof, which is

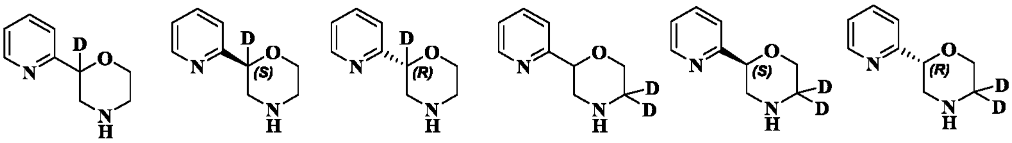

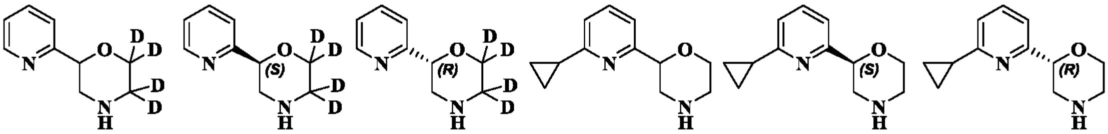

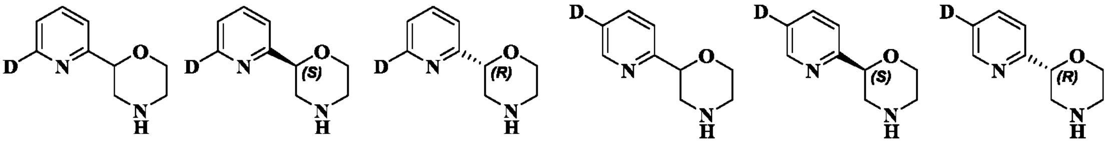

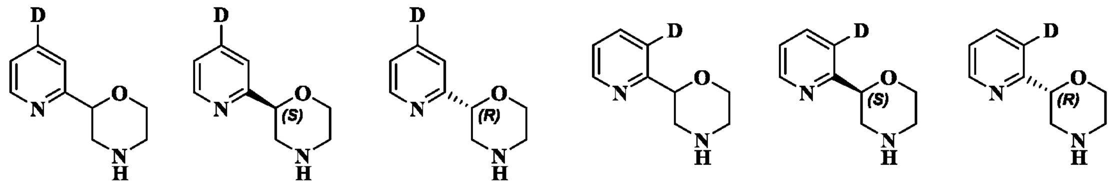

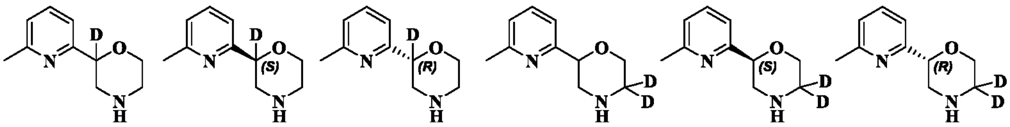

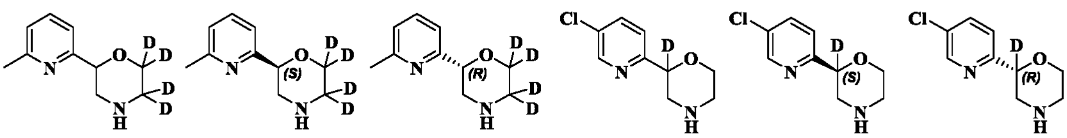

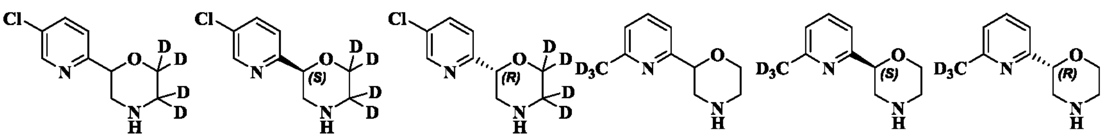

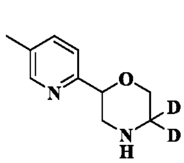
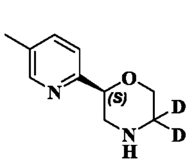
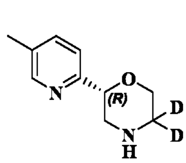
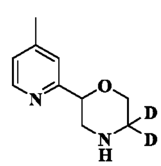
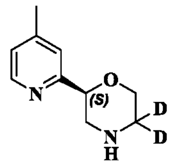
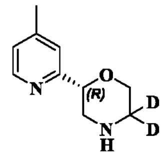
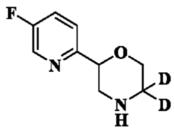
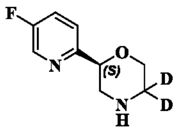
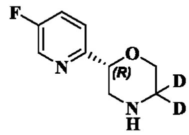
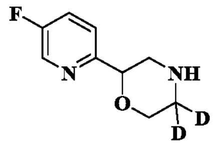
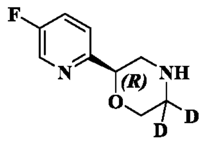
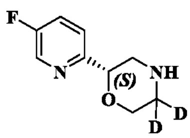
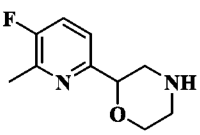
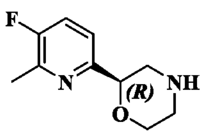
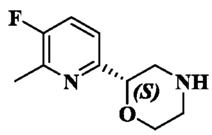
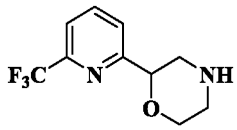
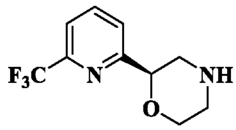
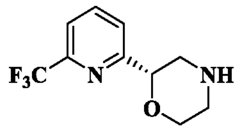
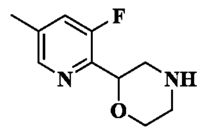
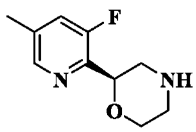
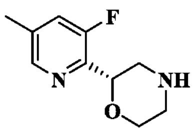
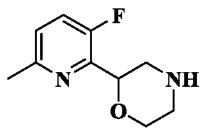
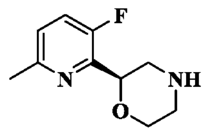
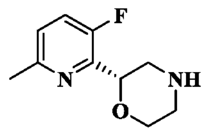
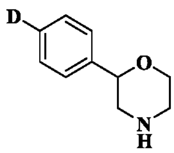
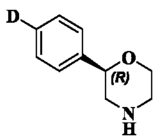
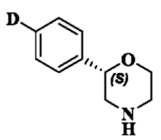
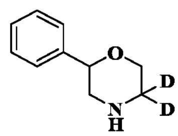
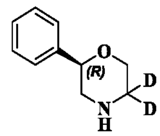
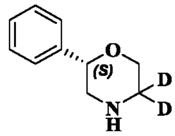
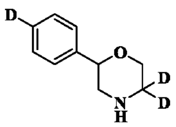
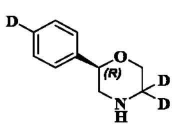
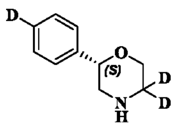
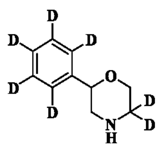
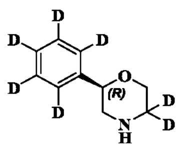
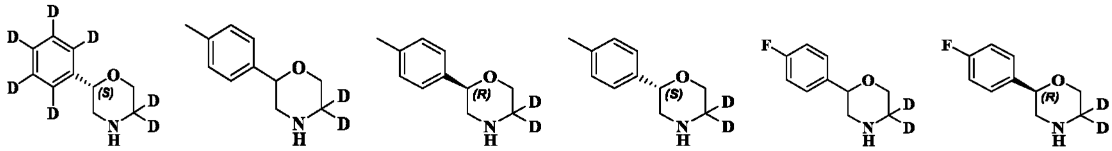
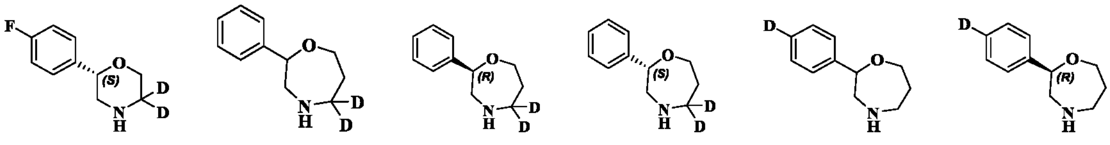

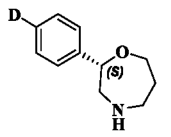 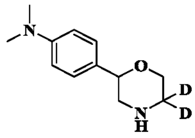 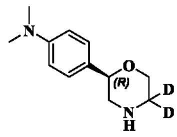 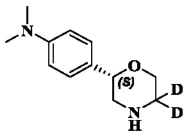 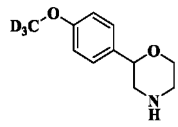

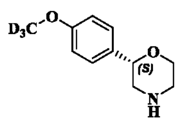 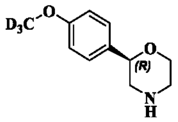 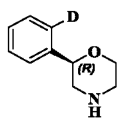 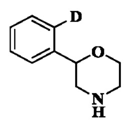 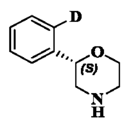 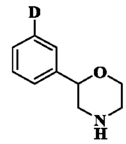 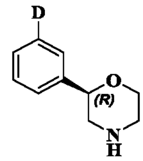

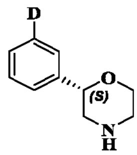 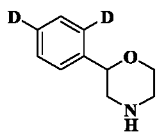 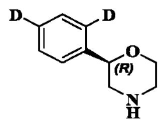 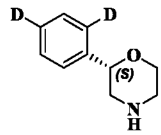 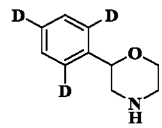 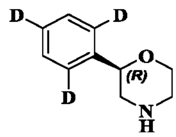

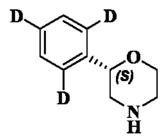 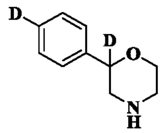 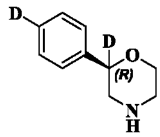 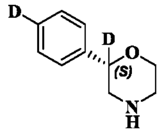 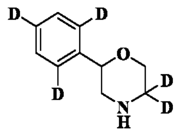 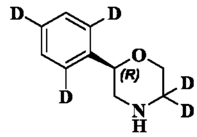

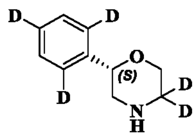 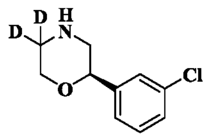 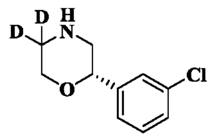 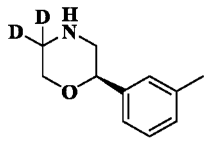 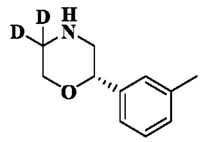

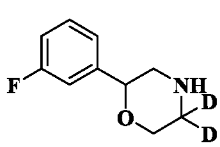 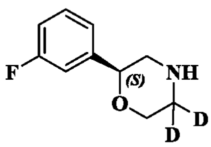 or 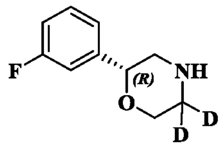 .

13. A pharmaceutical composition comprising a therapeutically effective dose of the compound, the stereoisomer thereof or the pharmaceutically acceptable salt thereof according to any one of claims 1 to 12, and one or more pharmaceutically acceptable carriers, diluents or excipients.

14. The pharmaceutical composition according to claim 13, **characterized in that** the weight pecentage of the compound, the stereoisomer thereof or the pharmaceutically acceptable salt thereof in terms of free base is 0.1%~95%, preferably 0.5% to 85%, more preferably 1% to 60%, further preferably 5% to 50%, and yet preferably 5-15%, 15-30%, 30-40% or 40-50%, and yet preferably 5-10%, 10-15%, 15-20%, 20-25%, 25-30%, 30-35%, 35-40%, 40-45% or 45-50%.

15. The pharmaceutical composition according to claims 13 or 14, **characterized in that** the pharmaceutical composition is selected from the group consisting of tablet, capsule, liquid and injection; optionally, the pharmaceutical composition comprises filler; optionally, the pharmaceutical composition comprises disintegrant; optionally, the pharmaceutical composition comprises binder; optionally, the pharmaceutical composition comprises surfactant; preferably, the pharmaceutical composition comprises one or more of filler, disintegrant, binder, surfactant, sweetening agent, glidant and lubricant.

16. The pharmaceutical composition according to any one of claims 13 to 15, **characterized in that** the pharmaceutical composition is flash-release preparation or sustained-release preparation.

17. The compound, the stereoisomer thereof or the pharmaceutically acceptable salt thereof according to any one of claims 1 to 12, or the pharmaceutical composition according to any one of claims 13 to 16, **characterized in that**

the unit dose of the compound in terms of free base is 1-1000mg, preferably 1-500mg, more preferably 1-300mg, further preferably 3-300mg, and yet preferably 5-200mg, or preferably 1mg, 2mg, 3mg, 4mg, 5mg, 10mg, 15mg, 20mg, 25mg, 30mg, 35mg, 40mg, 45mg, 50mg, 55mg, 60mg, 65mg, 70mg, 75mg, 80mg, 90mg, 95mg, 100mg, 200mg, 300mg, 400mg and 500mg.

**18.** Use of the compound, the stereoisomer thereof or the pharmaceutically acceptable salt thereof according to any one of claims 1 to 12, or the pharmaceutical composition according to claims 13 to 17 in the preparation of a TAAR1 agonist.

**19.** Use of the compound, the stereoisomer thereof or the pharmaceutically acceptable salt thereof according to any one of claims 1 to 12, or the pharmaceutical composition according to claims 13 to 17 in the preparation of medicaments for the treatment, prevention or management of neurological disorders; wherein the neurological disorder is selected from the group consisting of schizophrenia, social dysfunction and psychosis; preferably, the schizophrenia is selected from the group consisting of schizophrenia spectrum disorders, acute schizophrenia, chronic schizophrenia, NOS schizophrenia, paranoid schizophrenia, schizoid personality disorder and schizoid personality disorder; preferably, the psychosis is selected from the group consisting of delusional disorder, brief psychotic disorder, shared psychotic disorder, mental disorders caused by physical diseases, drug-induced psychosis, psychoaffective disorder, aggression, delirium, excitative psychosis, Tourette syndrome, organic or NOS psychosis, seizure, agitation, post-traumatic stress disorder, behavior disorder, neurodegenerative disease, Alzheimer's disease, Parkinson's disease, dyskinesia, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive disorder, vertigo, epilepsy, pain, neuropathic pain, sensitization accompanying neuropathic pain, inflammatory pain, fibromyalgia, migraine, cognitive impairment, movement disorder, restless leg syndrome, multiple sclerosis, sleep disorder, sleep apnea, narcolepsy, excessive daytime sleepiness, jet lag, drowsy side effect of medications, insomnia, substance abuse dependency, addiction, eating disorder, sexual dysfunction, hypertension, emesis, Lesche-Nyhane disease, hepatolenticular degeneration, autism, and premenstrual dysphoria.

**20.** A method for preparing the compound, the stereoisomer or the pharmaceutically acceptable salt thereof according to claim 10, **characterized in that** the method comprises the following steps:

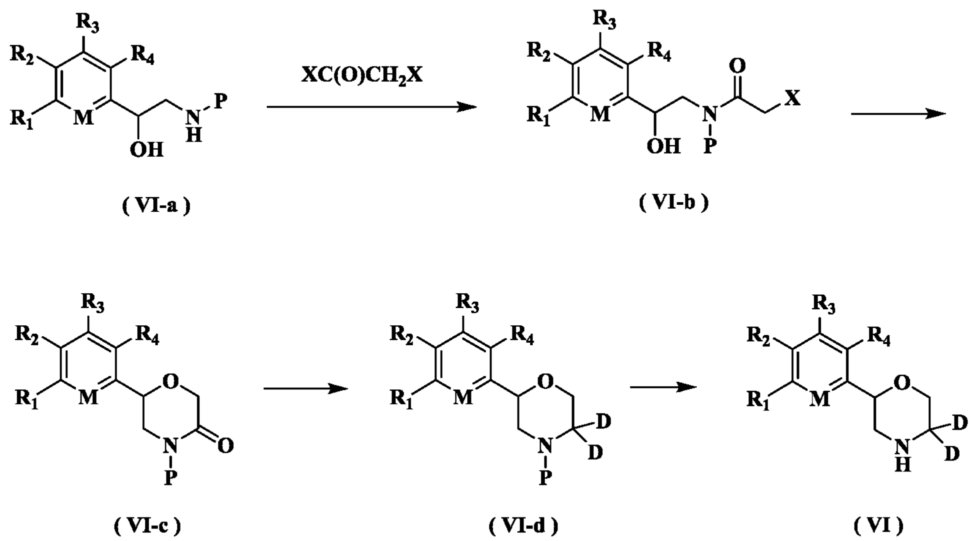


(VI-a) (VI-b) (VI-c) (VI-d) (VI)

a compound of formula (VI-a) is subject to acylation reaction to give a compound of formula (VI-b); the compound of formula (VI-b) is subject to cyclization to give a compound of formula (VI-c) under alkaline conditions; the compound of formula (VI-c) is subject to deutration to give a compound of formula (VI-d); the compound of formula (VI-d) is subject to deprotection to give a compound of formula (VI); optionally, a pharmaceutically acceptable salt can be further prepared;

wherein, X is halogen; P is amino protective group; $R_1$, $R_2$, $R_3$, and $R_4$ are as defined in Claim 10.

## INTERNATIONAL SEARCH REPORT

International application No.
**PCT/CN2022/129881**

**A. CLASSIFICATION OF SUBJECT MATTER**

C07D 265/30(2006.01)i; A61K 31/5377(2006.01)i; A61P 25/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, WPI, ISI web of knowledge, STN: 翰森生物医药, 吗啉, 吡啶, 氘代, 精神分裂症, HANSOH BIOMEDICAL, morpholine, pyridine, deuterium, schizophrenia, structural formula search.

**C. DOCUMENTS CONSIDERED TO BE RELEVANT**

| Category* | Citation of document, with indication, where appropriate, of the relevant passages | Relevant to claim No. |
|---|---|---|
| A | CN 104768950 A (HOFFMANN LA ROCHE) 08 July 2015 (2015-07-08) embodiments, and claims 1-16 | 1-20 |
| A | WO 2011069063 A2 (SUNOVION PHARMACEUTICALS, INC. et al.) 09 June 2011 (2011-06-09) embodiments, and claims 1-62 | 1-20 |
| A | CN 104603129 A (HOFFMANN LA ROCHE) 06 May 2015 (2015-05-06) embodiments, and claims 1-15 | 1-20 |
| A | CN 106255688 A (F. HOFFMANN-LA ROCHE LTD.) 21 December 2016 (2016-12-21) embodiments, and claims 1-15 | 1-20 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 January 2023** | **28 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/129881**

| Patent document cited in search report | Publication date (day/month/year) | Patent family member(s) | Publication date (day/month/year) |
|---|---|---|---|
| CN 104768950 A | 08 July 2015 | TW 201422612 A | 16 June 2014 |
| | | BR 112015009990 A2 | 11 July 2017 |
| | | AR 093372 A1 | 03 June 2015 |
| | | WO 2014072257 A1 | 15 May 2014 |
| | | JP 2016500706 A | 14 January 2016 |
| | | MX 2015005721 A | 08 September 2015 |
| | | RU 2015118290 A | 27 December 2016 |
| | | EP 2917211 A1 | 16 September 2015 |
| | | HK 1206727 A1 | 15 January 2016 |
| | | US 2016272626 A1 | 22 September 2016 |
| | | CA 2889627 A1 | 15 May 2014 |
| | | KR 20150065190 A | 12 June 2015 |
| WO 2011069063 A2 | 09 June 2011 | CA 2781716 A1 | 09 June 2011 |
| | | IL 243019 B | 30 August 2018 |
| | | JP 6028310 B1 | 16 November 2016 |
| | | RU 2012127770 A | 10 January 2014 |
| | | KR 20150090269 A | 05 August 2015 |
| | | NZ 600008 A | 31 October 2014 |
| | | HK 1212969 A1 | 24 June 2016 |
| | | JP 5710064 B1 | 30 April 2015 |
| | | IN 1373MUMNP2012 A | 11 October 2013 |
| | | CN 104193761 A | 10 December 2014 |
| | | KR 20120127581 A | 22 November 2012 |
| | | EP 2507245 A2 | 10 October 2012 |
| | | NZ 731621 A | 25 January 2019 |
| | | ES 2625330 T3 | 19 July 2017 |
| | | CN 102762575 A | 31 October 2012 |
| | | SG 181498 A1 | 30 July 2012 |
| | | ZA 201203533 B | 28 August 2013 |
| | | NZ 767139 A | 26 August 2022 |
| | | US 2013109677 A1 | 02 May 2013 |
| | | US 2022387382 A1 | 08 December 2022 |
| | | KR 20200013071 A | 05 February 2020 |
| | | RU 2018100098 A | 09 July 2019 |
| | | AU 2010325925 A1 | 07 June 2012 |
| | | US 2020323819 A1 | 15 October 2020 |
| | | JP 2017031198 A | 09 February 2017 |
| | | US 2016083399 A1 | 24 March 2016 |
| | | KR 20170100047 A | 01 September 2017 |
| | | IL 243015 B | 31 October 2018 |
| | | IL 220173 A0 | 31 July 2012 |
| | | CN 111560025 A | 21 August 2020 |
| | | RU 2641648 C1 | 19 January 2018 |
| | | KR 20210120133 A | 06 October 2021 |
| | | SG 10201510665 W A | 28 January 2016 |
| | | HK 1247911 A1 | 05 October 2018 |
| | | PT 2507245 T | 22 May 2017 |
| | | IL 243018 B | 31 May 2020 |
| | | JP 2020164541 A | 08 October 2020 |
| | | MX 2012006326 A | 09 October 2012 |

Form PCT/ISA/210 (patent family annex) (January 2015)

**INTERNATIONAL SEARCH REPORT**
**Information on patent family members**

International application No.
**PCT/CN2022/129881**

| Patent document cited in search report | Publication date (day/month/year) | Patent family member(s) | Publication date (day/month/year) |
|---|---|---|---|
| | | BR 122021013836 B1 | 24 May 2022 |
| | | BR 112012013431 A2 | 05 April 2016 |
| | | AU 2016200448 A1 | 18 February 2016 |
| | | AU 2017248551 A1 | 09 November 2017 |
| | | JP 2018104454 A | 05 July 2018 |
| | | IL 243021 B | 30 April 2018 |
| | | NZ 626068 A | 24 June 2016 |
| | | JP 2013512926 A | 18 April 2013 |
| | | EP 3252057 A2 | 06 December 2017 |
| | | SG 10201401661 R A | 30 July 2014 |
| | | NZ 711802 A | 30 June 2017 |
| CN 104603129 A | 06 May 2015 | BR 112015001771 A2 | 04 July 2017 |
| | | HK 1206714 A1 | 15 January 2016 |
| | | US 2015191458 A1 | 09 July 2015 |
| | | RU 2015111836 A | 10 November 2016 |
| | | EP 2895478 A1 | 22 July 2015 |
| | | ES 2605632 T3 | 15 March 2017 |
| | | WO 2014041106 A1 | 20 March 2014 |
| | | CA 2879176 A1 | 20 March 2014 |
| | | JP 2015526496 A | 10 September 2015 |
| | | KR 20150042848 A | 21 April 2015 |
| | | MX 2015001108 A | 08 April 2015 |
| CN 106255688 A | 21 December 2016 | MX 2016013412 A | 18 January 2017 |
| | | KR 20160147033 A | 21 December 2016 |
| | | EP 3137459 A1 | 08 March 2017 |
| | | WO 2015165835 A1 | 05 November 2015 |
| | | WO 2015165085 A1 | 05 November 2015 |
| | | RU 2016146560 A | 01 June 2018 |
| | | AR 100210 A1 | 21 September 2016 |
| | | US 2017044145 A1 | 16 February 2017 |
| | | CA 2943575 A1 | 05 November 2015 |
| | | JP 2017513912 A | 01 June 2017 |
| | | TW 201546067 A | 16 December 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011069063 A2
- WO 2015165835 A


### Non-patent literature cited in the description


- *Bioorg.Med.Chem.Lett.,* 2016, vol. 26, 1329-1332
- *ACS Combined Science,* 2016, vol. 18 (9), 569-574
- *Bioorg. Med. Chem. Lett.,* 2016, vol. 26, 1329-1332